(19) Europäisches Patentamt
European Patent Office
Office européen des brevets

(11) EP 4 342 881 A1

# (12) EUROPEAN PATENT APPLICATION

published in accordance with Art. 153(4) EPC

(43) Date of publication:
**27.03.2024 Bulletin 2024/13**

(21) Application number: **21940177.5**

(22) Date of filing: **20.05.2021**

(51) International Patent Classification (IPC):
***C07C 229/16*** *(2006.01)* ***C07C 229/24*** *(2006.01)*
***C07C 229/26*** *(2006.01)* ***A61K 48/00*** *(2006.01)*
***C12N 15/00*** *(2006.01)*

(52) Cooperative Patent Classification (CPC):
**A61K 9/127; A61K 45/00; A61K 47/18; A61K 48/00; C07C 229/16; C07C 229/24; C07C 229/26; C07C 233/36; C07C 237/08; C12N 15/00**

(86) International application number:
**PCT/CN2021/094937**

(87) International publication number:
**WO 2022/241723 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Beijing Institute Of Technology**
**Beijing 100081 (CN)**

(72) Inventors:
- **HUANG, Yuanyu**
  **Beijing 100081 (CN)**
- **HUANG, Jinyu**
  **Beijing 100081 (CN)**
- **HU, Bo**
  **Beijing 100081 (CN)**

(74) Representative: **DehnsGermany Partnerschaft von Patentanwälten**
**Theresienstraße 6-8**
**80333 München (DE)**

## (54) COMPOUND, LIPOSOME, AND USES THEREOF

(57) A compound, a liposome, a method for preparing a liposome, a drug carrier, a pharmaceutical composition and a use of the pharmaceutical composition in the treatment or prevention of hyperlipidemia and related diseases thereof.

EP 4 342 881 A1

**Description**

**PRIORITY**

None.

**TECHNICAL FIELD**

The present disclosure belongs to the field of biomedicine and in particular, relates to a compound, a liposome, a drug carrier and the use thereof, a method for preparing a liposome, a pharmaceutical composition and use.

**BACKGROUND**

Nucleic acid-based molecules, such as siRNA, mRNA, etc. are very potential drug molecules and they need to enter cells to function. However, nucleic acid-based molecules themselves are compounds with large molecular-weight, highly electronegativity and easily degradable, which cannot enter cells themselves. Appropriate delivery carriers are therefore required to carry them into cells, or tissues *in vivo*.

Liposomes have been proved to be an effective nucleic acid carrier, and Onpattro, an siRNA drug encapsulated by a liposome has been marketed. With the development of research on nucleic acid drugs, especially siRNA drugs and mRNA drugs, there is a continuous demand for research on nucleic acid drug carriers, including the research of the liposomes targeting different cell types and tissue types, liposomes that better protect nucleic acids from being degraded by enzymes or liposomes that better released in cells, and liposomes with lower toxicity, and liposomes that are readily biodegradable, etc.

Therefore, there is a need to develop a liposome that has high stability, low toxicity, and are readily biodegradable.

**SUMMARY**

The present disclosure aims to solve at least one of the technical problems in the related technology to a certain extent.

Therefore, in the first aspect of the present disclosure, the present disclosure provides a compound represented by formula (I) or a stereoisomer, tautomer, solvate, or pharmaceutically acceptable salt of the compound represented by formula (I),

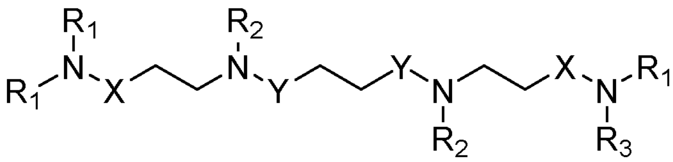

(I)

wherein X, Y, $R_1$, $R_2$, $R_3$ have the definitions as described in the present disclosure.

According to an embodiment of the present disclosure, X and Y are each independently selected from $-CH_2-$ or -CO-, respectively; X and Y are not $-CH_2$ - or -CO- at the same time, wherein each X is the same or different and each Y is the same or different; $R_2$ is selected from H, optionally substituted $C_1$-$C_{20}$ alkyl, optionally substituted $C_1$-$C_{20}$ alkenyl, or optionally substituted $C_1$-$C_{20}$ alkyne; $R_3$ is selected from H, optionally substituted $C_1$-$C_{20}$ alkyl, optionally substituted $C_1$-$C_{20}$ alkenyl, or optionally substituted $C_1$-$C_{20}$ alkyne, where the substituent groups are each independently selected from H, F, Cl, Br, CN and $NO_2$; $R_1$ is selected from

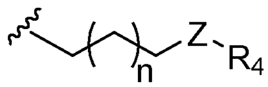 or 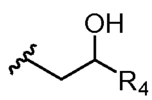,

where n is an integer selected from 0-10, and Z is selected from

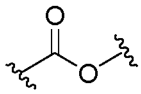 or 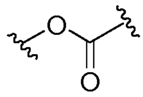 ;

$R_4$ is selected from optionally substituted $C_4$-$C_{20}$ alkyl, optionally substituted $C_4$-$C_{20}$ alkyne, or optionally substituted $C_4$-$C_{20}$ alkyne, where the substituent groups are each independently selected from H, F, Cl, Br, CN, and $NO_2$.

According to an embodiment of the present disclosure, $R_2$ is selected from H, $CH_3$-, or $R_1$; $R_3$ is selected from H, or $R_1$; $R_1$ is selected from

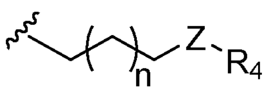 or 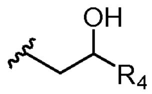

where n is an integer selected from 1-5, and Z is selected from

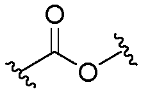 or 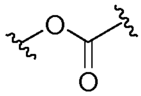 ;

$R_4$ is selected from $C_4$-$C_{20}$ alkyl, $C_4$-$C_{20}$ alkenyl or $C_4$-$C_{20}$ alkynyl, wherein the $C_4$-$C_{20}$ alkenyl contains 1-3 double bonds and the $C_4$-$C_{20}$ alkynyl contains 1-3 triple bonds.

According to an embodiment of the present disclosure, $R_4$ is selected from $C_5$-$C_{15}$ alkyl, $C_5$-$C_{15}$ alkenyl, wherein $C_5$-$C_{15}$ alkenyl contains 1-3 double bonds.

According to an embodiment of the present disclosure, $R_1$, $R_2$ and $R_3$ are the same; $R_4$ is selected from $C_{10}$-$C_{15}$ alkyl, $C_{10}$-$C_{15}$ alkenyl, wherein the $C_{10}$-$C_{15}$ alkenyl contains 1-3 double bonds.

According to an embodiment of the present disclosure, the compound has the structure of one of the following:

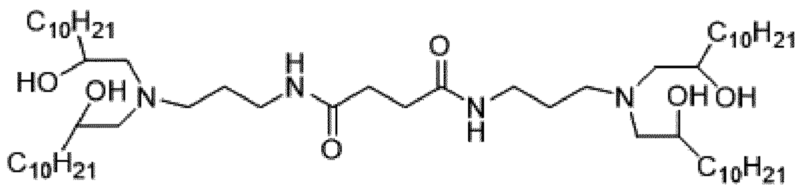

(A4)

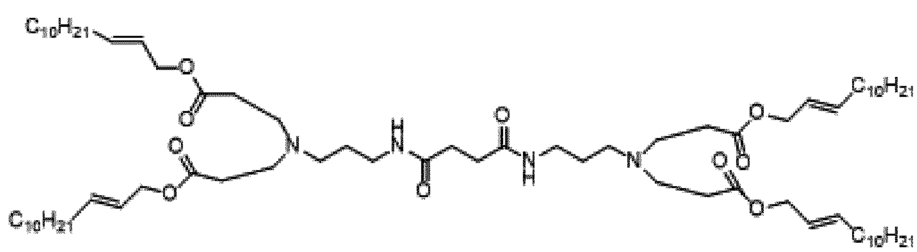

;

(A10)

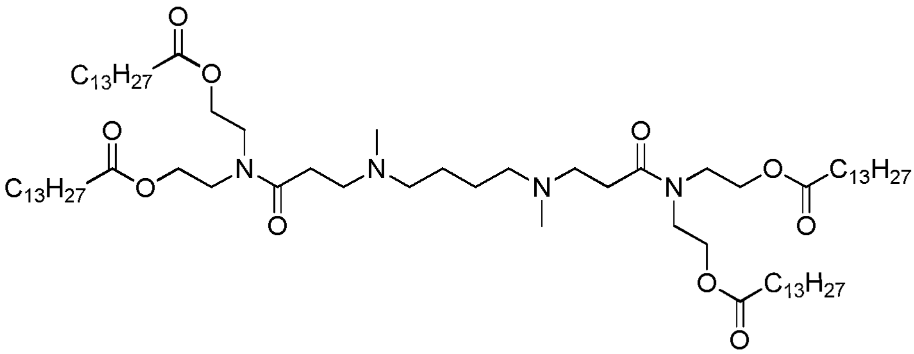

;

(B8)

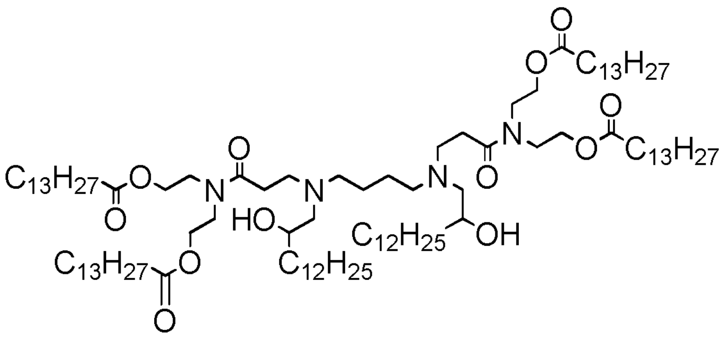

(C3).

According to an embodiment of the present disclosure, the skeleton of the compound includes a skeleton main chain structure of 4 amine groups and a plurality of branched alkyl or substituted alkyl structures. On the skeleton main chain, each amine group is linked by an alkyl or an acyl, wherein there are at least two acyl groups, which reduces the basicity of the compound, reduces the effect of the basicity of the alkylamino group on the chemical stability of the ester group contained in the branched chain, and reduces the positive charge of the compound, which is beneficial for reducing the positive charge carried by the liposome. At the same time, the amide bond enhances the metabolizability in vivo, avoiding the accumulation of the compound in vivo.

In a second aspect of the present disclosure, the present disclosure provides the use of the aforementioned compound in the preparation of a liposome. According to an embodiment of the present disclosure, the aforementioned compounds are amine-containing lipid compounds having ionizable properties and can be used for preparing liposomes.

According to an embodiment of the present disclosure, the aforementioned compounds may be used to prepare liposomes in the form of polymers. The aforementioned compounds can form covalent connections with other substances to prepare liposomes. Such compounds can chemically react with other substances to produce liposomes. According to embodiments of the present disclosure, the specific manner of preparing liposomes using the aforementioned compounds is not limited, and the use of the above compounds to prepare liposomes containing all or part of the structure of the aforementioned compounds is regarded as the purpose of the present disclosure.

In a third aspect of the disclosure, the present disclosure provides a liposome. The liposome according to the embodiments of the present disclosure is made of the compound of the first aspect of the present disclosure. The liposome can encapsulate the added substance to be loaded by the flow action of the lipid bilayer, with good encapsulation effect, so that the loaded substance can efficiently enter cells and escape from the endosome to the cytoplasm.

According to an embodiment of the present disclosure, the aforementioned compound may form the liposome by self-assembly with a hydrophobic lipid and/or an amphiphilic lipid.

According to an embodiment of the present disclosure, the liposome further includes a hydrophobic lipid, an amphiphilic lipid, a buffering agent, and/or an organic solvent, the amphiphilic lipid includes at least one selected from a neutral lipid and a PEG lipid, the hydrophobic lipid includes a sterol.

According to an embodiment of the present disclosure, the neutral lipid comprises at least one selected from: distearoyl phosphatidyl choline (DSPC), dipalmitoyl phosphatidyl choline (DPPC), phosphatidyl choline (POPC), dioleoyl phosphatidyl ethanolamine (DOPE), dilauroyl phosphatidyl choline (DLPC), diethyl pyrocarbonate (DEPC), dimyristoyl

phosphatidyl choline (DMPC), and egg phosphatidylcholine (EPC).

According to an embodiment of the present disclosure, the PEG-lipid comprises at least one selected from: dipalmitoyl phosphatidyl ethanolamine-PEG (DPPE-PEG), distearoyl phosphatidyl ethanolamine-PEG (DSPE-PEG), dimyristyl glycerol (DMG-PEG), and dimethacrylate (DMA-PEG).

According to an embodiment of the present disclosure, the sterol is cholesterol.

According to an embodiment of the present disclosure, the buffering agent is an acidic buffering agent.

According to an embodiment of the present disclosure, the buffering agent includes at least one selected from citric acid, sodium citrate, acetic acid, sodium acetate, disodium hydrogen phosphate, sodium dihydrogen phosphate, trihydroxymethylaminomethane-hydrochloric acid, potassium dihydrogen phosphate-sodium hydroxide, boric acid-borax, glycine-hydrochloric acid, phthalic acid-hydrochloric acid, potassium hydrogen phthalate, and sodium dihydrogen phosphate-citric acid.

According to an embodiment of the present disclosure, the organic solvent includes at least one selected from methanol, ethanol, isopropanol, benzene, toluene, xylene, pentane, hexane, octane, cyclohexane, cyclohexanone, toluene cyclohexanone, chlorobenzene, dichlorobenzene, dichloromethane, ethyl ether, propylene oxide, acetone, methyl butanone, methyl isobutyl ketone, acetonitrile, pyridine, phenol, styrene, perchloroethylene, trichloroethylene, vinyl ethylene glycol ether, and triethanolamine.

According to an embodiment of the present disclosure, the amount of the compound is 20-80% mol/mol based on the liposome.

According to an embodiment of the present disclosure, the molar ratio of the compound, neutral lipid, sterol, and PEG-lipid is (20-80): (5-50): (10-60): (0.2-20).

According to an embodiment of the present disclosure, the volume of the buffering agent is 50-90% v/v based on the liposome.

According to an embodiment of the present disclosure, the volume of the buffering agent is 75% v/v based on the liposomes.

According to an embodiment of the present disclosure, the liposome with the above ratio has high transfection efficiency, good encapsulation effect, and strong drug delivery ability.

In a fourth aspect of the present disclosure, the present disclosure provides a method for preparing the aforementioned liposome. According to an embodiment of the present disclosure, comprising mixing a predetermined amount of the compound of the first aspect of the present disclosure with an organic solvent, so as to obtain the liposome. According to an embodiment of the present disclosure, dissolving the compound in an organic solvent facilitates liposome formation.

According to an embodiment of the present disclosure, the method further includes: dissolving a predetermined amount of lipid in the aforementioned organic solution and then mixing with a buffering agent to obtain the liposome. According to an embodiment of the present disclosure, the amount of the compound is 20-80% mol/mol based on the liposome; the molar ratio of the compound, the neutral lipid, the sterol and the PEG-lipid is (20-80): (5-50): (10-60): (0.2-20); the volume of the buffering agent is 75% v/v based on the liposome.

In a fifth aspect of the present disclosure, the present disclosure provides a drug carrier comprising the compound of the first aspect of the present disclosure or the liposome of the third aspect of the disclosure. According to an embodiment of the present disclosure, the compound is an ionizable amine-containing lipid, and the drug carrier is an ionizable carrier that can be loaded with a negatively charged drug, and deliver the drug into a cell and efficiently escape from the endosome to the cytoplasm, facilitating the pharmacodynamic exertion of the loaded drug.

In a sixth aspect the present disclosure, the present disclosure provides the use of the compound of the first aspect of the present disclosure, the liposome of the third aspect of the present disclosure or the drug carrier of the fifth aspect of the present disclosure in the preparation of a drug.

In a seventh aspect of the present disclosure, the present disclosure provides a pharmaceutical composition comprising a carrier and a pharmaceutically active ingredient, wherein the carrier includes the drug carrier of the fifth aspect of the present disclosure. According to an embodiment of the present disclosure, the drug carrier is an ionizable carrier that can load a drug carrier is an ionizable carrier that can be loaded with a negatively charged pharmaceutical active ingredient, and deliver the pharmaceutical active ingredient into a cell and efficiently escape from the endosome to the cytoplasm, facilitating the pharmacodynamic exertion of the loaded pharmaceutical active ingredient.

According to an embodiment of the present disclosure, the pharmaceutically active ingredient includes at least one selected from a DNA molecule, an RNA molecule, a protein and a small molecular drug.

According to an embodiment of the present disclosure, the pharmaceutically active ingredient is negatively charged. According to an embodiment of the present disclosure, the drug carrier includes an ionizable amine-containing lipid that is positively charged at a specific pH and can be combined with a negatively charged pharmaceutically active ingredient. In addition, the pharmaceutically active ingredient can be encapsulated by the flow action of the lipid bilayer and delivered to the cell.

According to an embodiment of the present disclosure, the pharmaceutically active ingredient contains nucleic

acid. According to an embodiment of the present disclosure, the pharmaceutically active ingredient contains DNA, RNA, nucleic acid-protein complex, nucleic acid-lipid complex, nucleic acid-nuclide complex and the like, and the type of the nucleic acid is not particularly limited.

According to an embodiment of the present disclosure, the mass ratio of the carrier to the pharmaceutically active ingredient is (5-40): 1.

According to an embodiment of the present disclosure, the mass ratio of the carrier to the pharmaceutically active ingredient is (8-20): 1.

According to an embodiment of the present disclosure, the mass ratio of the carrier to the pharmaceutically active ingredient is 15: 1.

In an eighth aspect of the present disclosure, the present disclosure provides a transfection complex comprising the above compound or the above liposome.

According to an embodiment of the present disclosure, the transfection complex includes at least one bioactive agent.

According to an embodiment of the present disclosure, the bioactive agent includes at least one selected from: a DNA molecule, an RNA molecule, a protein and a drug.

Additional aspects and advantages of the present disclosure will be set forth in part in the description which follows and, in part, will be obvious from the description, or may be learned by practice of the present disclosure.

## BRIEF DESCRIPTION OF DRAWINGS

The foregoing and/or additional aspects and advantages of the present disclosure will become apparent and readily appreciated from the following description of the embodiments, taken in conjunction with the accompanying drawings of which:

FIG. 1 is a result of a gel retardation test according to an embodiment of the present disclosure;

FIG. 2 is a stability result of a formulation of siA4-13 at 4°C according to an example of the present disclosure;

FIG. 3 is the quantitative data of the distribution of Cy-siFLA4-13 in C57BL/6 mice according to an embodiment of the present disclosure;

FIG. 4 shows the change in ApoB mRNA expression and the change in CHO, TG, LDL-c, and HDL-c levels at different time points after animals received siA4-13 formulations of the present disclosure at different concentrations or at different time points, wherein, graph a is the change in ApoB mRNA expression and the change in CHO, TG, LDL-c, and HDL-c levels at different time points after animals received 1 mg/kg or 3 mg/kg siA4-13 formulations, and graph b is the change in ApoB mRNA expression and the change in CHO, TG, LDL-c, and HDL-c levels at 48 hours after animals received siA4-13 formulations at a doses of 0.01 mg/kg to 1 mg/kg;

FIG. 5 shows the changes in serum biochemical indicators according to an embodiment of the present disclosure;

FIG. 6 shows the hypolipidemic effect and ANGPTL3 mRNA expression-inhibiting ability of siANA4-13 in db/db mice according to an embodiment of the present disclosure;

FIG. 7 shows the lipid deposition in the liver of animals in different treatment groups according to an embodiment of the present disclosure;

FIG. 8 shows the hypolipidemic effect and change in blood lipid within 4 weeks after siApoCA4-13 treatment and drug withdrawal according to an embodiment of the present disclosure;

FIG. 9 shows the result of liposome-mediated luciferase mRNA expression on cells according to different formulations of an embodiment of the present disclosure;

FIG. 10 shows the expression result of liposome-mediated luciferase mRNA in mice according to an embodiment of the present disclosure;

FIG. 11 shows the chemical structure of lipid E according to an embodiment of the present disclosure;

FIG. 12 is a stability test result of lipid A4 and lipid E at 40 degrees Celsius according to an embodiment of the present disclosure; and

FIG. 13 is a comparison of the efficacy of Lipo and A4-13 in C57 mice according to an embodiment of the present disclosure.

## DESCRIPTION OF EMBODIMENTS

The embodiments of the present disclosure will be described in detail, examples of which are illustrated in the accompanying drawings. The embodiments described below with reference to the figures are exemplary and are intended to be illustrative of the present disclosure and are not to be construed as limiting the disclosure.

The terms "first" and "second" are used for descriptive purposes only and are not to be construed as indicating or implying relative importance or implicitly indicating the number of technical features indicated. Thus, a feature defined

as "first" or "second" may explicitly or implicitly comprise at least one feature. In the description of the present disclosure, the meaning of "plurality" is at least two, such as two, three, etc. unless specifically limited otherwise.

Certain embodiments of the disclosure will now be described in detail, examples of which are illustrated in the accompanying structural formula and chemical formula. The present disclosure is intended to cover all alternatives, modifications and equivalents, which are included within the scope of the present disclosure as defined by the claims. One skilled in the art will recognize that many methods and materials similar or equivalent to those described herein can be used in the practice of the present disclosure. The present disclosure is in no way limited to the methods and materials described herein. In the event that one or more of the incorporated literature, patents, and similar materials differs from or contradicts this application, including but not limited to defined terms, term applications, described techniques, etc. this application controls.

It should be further recognized that certain features of the present disclosure, which are, for clarity, described in the context of separate embodiments, may also be provided in combination in a single embodiment. Conversely, various features of the present disclosure which are, for brevity, described in the context of a single embodiment, may also be provided separately or in any suitable subcombination.

Unless otherwise defined, all technical and scientific terms used herein have the same meaning as commonly understood by one of ordinary skill in the art to which the present disclosure belongs, and unless otherwise defined, all patent publications cited in the present disclosure are hereby incorporated by reference in their entirety.

The present disclosure will apply the following definitions unless otherwise indicated. For purposes of the present disclosure, the chemical elements are defined in accordance with the Periodic Table of the Elements, CAS Edition, and Handbook of Chemicals, 75, thEd, 1994. In addition, general principles of organic chemistry are described in "Organic Chemistry", Thomas Sorrell, University Science Books, Sausalito: 1999, and "March's Advanced Organic Chemistry", by Michael B. Smith and Jerry March, John Wiley & Sons, New York: 2007, the entire disclosure of the present disclosure hereby incorporate references.

As used herein, "liposome" or "lipid nanoparticles (LNP)" refers to a drug-loaded system which uses a biocompatible lipid material as a carrier to dissolve or encapsulate a drug or other biologically active substance in a lipid core or adsorb, and attach to the surface of a nanoparticle.

The present disclosure also includes isotopic labeled compounds of the present disclosure, which are identical to those described in the present disclosure except for the fact that one or more atoms are replaced by atoms with atomic masses or mass numbers different from those commonly found in nature. Exemplary isotopes that can also be incorporated into compounds of the present disclosure include isotopes of hydrogen, carbon, nitrogen, oxygen, phosphorous, sulfur, fluorine and chlorine, such as $^{2}H$, $^{3}H$, $^{13}C$, $^{14}C$, $^{15}N$, $^{16}O$, $^{17}O$, $^{31}P$, $^{32}P$, $^{36}S$, $^{18}F$, and $^{37}Cl$.

Compounds of the present disclosure that contain the aforementioned isotopes and/or other isotopes of other atoms, as well as pharmaceutically acceptable salts of such compounds, are included within the scope of the present disclosure. Isotopically-labeled compounds of the present disclosure, for example, radioactive isotopes such as $^{3}H$ and $^{14}C$, can be incorporated into compounds of the present disclosure for use in drug and/or substrate tissue distribution assays. Due to its ease of preparation and detection, tritiated, i.e. 3H, and carbon-14, i.e. $^{14}C$, isotopes are particularly preferred. In addition, replacing with heavy isotopes such as deuterium, i.e. $^{2}H$, can provide therapeutic advantages derived from greater metabolic stability, such as increased *in vivo* half-life or reduced dose requirements. Therefore, it may be preferable in some cases.

Stereochemical definitions and conventions used herein generally in accordance with S.P.Parker, Ed., McGraw-Hill Dictionary of Chemical Terms(1984)McGraw-Hill Book Company, New York; and Eliel, E. and Wilen, S., "Stereochemistry of Organic Compounds", John Wiley & Sons, Inc., New York, 1994. The compounds of the present disclosure may contain asymmetric or chiral centers and, therefore, exist in different stereoisomeric forms. It is expected that all stereoisomeric forms of the compounds of the present disclosure, including but not limited to, diastereomers, enantiomers and atropisomer, as well as the mixtures thereof such as racemic mixtures, are included within the scope of the present disclosure. Many organic compounds exist in optically active forms, i.e. they have the ability to rotate the plane of plane-polarized light. When describing an optically active compound, the prefixes D and L or R and S are used to denote the absolute configuration of the molecule with respect to the chiral center(s) in the molecule. The prefixes d and l or (+) and (-) are symbols employed to designate the rotation of plane-polarized light by the compound, where (-) or l indicates that the compound is levorotatory. Compounds prefixed with (+) or d are dextrorotatory. For a given chemical structure, these stereoisomers are identical except that they are mirror images of each another. A specific stereoisomer may also be referred to as an enantiomer, and a mixture of such isomers is often called a mixture of enantiomers. A 50: 50 mixture of enantiomers is referred to as a racemic mixture or a racemate, which may occur where there is no stereoselection or stereospecificity in a chemical reaction or process.

Depending on the choice of the starting materials and processes, the compounds according to the present disclosure can be present in the form of one of the possible isomers or as mixtures thereof, for example as pure optical isomers, or as isomer mixtures, such as racemic and diastereomer mixtures, depending on the number of asymmetric carbon atoms. The optically active (R) - or (S) - isomers can be prepared using chiral synthons or chiral formulations or

resolved using conventional techniques. If the compound contains a double bond, the substituent may be in the E or Z configuration; if the compound contains a disubstituted cycloalkyl, the substituent group of the cycloalkyl may be in cis-or cis-or trans-configuration.

The compounds of the present disclosure may contain asymmetric or chiral centers and, therefore, exist in different stereoisomeric forms. It is contemplated that all stereoisomeric forms of the compounds of the present disclosure, including but not limited to, diastereomers, enantiomers and atropisomer and geometric (or conformational) isomers and mixtures thereof such as racemic mixtures, are within the scope of the present disclosure.

Unless otherwise stated, structures depicted herein are also meant to include all isomeric (e.g. enantiomeric, atropisomer, and geometric (or conformational)) forms of the structure; for example, the R and S configurations for each asymmetric center, (Z) and (E) double bond isomers, and (Z) and (E) conformational isomers. Therefore, the individual stereoisomers of the compounds of, as well as enantiomeric mixtures, diastereomeric mixtures, and geometric isomer (or conformational isomer) mixtures the present disclosure are all within the scope of the present disclosure.

The term "tautomer" or "tautomeric form" refers to structural isomers having different energies which are interconvertible via a low energy barrier. If tautomerism is possible (such as in solution), then a chemical equilibrium of tautomers can be achieved. For example, protontautomer (also known as prototropic tautomer) include interconversions by migration of a proton, such as keto-enol isomerization and imine-enamine isomerization. Valence tautomer include interconversions by reorganization of some of the bonding electrons. A specific example of keto-enol tautomerism is the interconversion of pentane-2, 4-dione and 4-hydroxypent-3-en-2-one tautomers. Another example of tautomerism is phenol-keto tautomerism. A specific example of phenol-ketone tautomerism is the interconversion of pyridin-4-ol and pyridin-4 (1H)-one tautomers. Unless otherwise indicated, all tautomeric forms of the compounds of the present disclosure are within the scope of the present disclosure.

The "nitrogen oxide" used in the present invention means that when a compound contains several amine functional groups, one or more nitrogen atoms can be oxidized to form an N-oxide. Particular examples of N-oxides are N-oxides of tertiary amines or N-oxides of nitrogen atoms of nitrogen containing heterocycles. The corresponding amines can be treated with oxidizing agents such as hydrogen peroxide or peracids (e.g. peroxycarboxylic acids) to form N-oxides (see Advanced Organic Chemistry, wiley Interscience, 4th edition, Jerry March, pages). In particular, N-oxides can be prepared by the method of L. W. Deady (Syn. Comm. 1977, 7, 509-514) in which an amine compound is reacted with m-chloroperbenzoic acid (MCPBA), for example in an inert solvent such as dichloromethane.

The "solvate" of the present disclosure refers to the association formed by one or more solvent molecules and the compound of the present disclosure. The solvents that form solvates include, but are not limited to, water, isopropanol, ethanol, methanol, dimethyl sulfoxide, ethyl acetate, acetic acid, and aminoethanol. The term "hydrate" refers to an association where the solvent molecule is water.

The "pharmaceutically acceptable salts" used in the present invention refer to organic and inorganic salts of the compounds of the present disclosure. Pharmaceutically acceptable salts are well known in the art, as described in the literature: S. M. Berge et al., describe pharmaceutically acceptable salts in detail in J. Pharmaceutical Sciences, 1977, 66: 1-19. Pharmaceutically acceptable salts formed from non-toxic acids include, but are not limited to, inorganic acid salts formed by reaction with amino groups such as hydrochloride, hydrobromide, phosphate, sulfate, perchlorate, and organic acid salts such as acetate, oxalate, maleate, tartrate, citrate, succinate, malonate, or by other methods described in the literature such as ion exchange. Other pharmaceutically acceptable salts include adipate, alginate, ascorbate, aspartate, benzenesulfonate, benzoate, bisulfate, borate, butyrate, camsylate, camphor sulfonate, cyclopentyl propionate, digluconate, dodecyl sulfate, ethanesulfonate, formate, fumarate, glucoheptonate, glycerophosphate, gluconate, hemisulfate, heptaneate, hexanoate, hydroiodate, 2-hydroxyethanesulfonate, lacturonate, lactate, laurate, lauryl sulfate, malate, malonate, methanesulfonate, 2-naphthesulfonate, nicotinate, nitrate, oleate, palmitate, pamoate, pectate, persulfate, 3-phenylpropionate, picrate, pivalate, propionate, stearate, thiocyanate, p-toluenesulfonate, undecanoate, valerate, etc. The salts obtained with appropriate alkalis include salts of alkali metal, alkaline earth metal, ammonium, and N+($C_{1-4}$ alkyl)4. The present disclosure also contemplates the quaternary ammonium salts formed from any compound containing an N group. Water soluble, oil soluble, or dispersed products can be obtained through quaternization. Alkali or alkaline earth metal salts include sodium, lithium, potassium, calcium, magnesium, and the like. Pharmaceutically acceptable salts further include suitable, nontoxic ammonium, quaternary ammonium, and amine cations formed as counterions, such as halide, hydroxide, carboxylate, sulfate, phosphate, nitrate, $C_{1-8}$ sulfonate and aromatic sulfonate.

Any asymmetric atom (e.g. carbon or the like) of the compounds of the present disclosure can be present in racemic or enantiomerically enriched form, for example, the (R)-, (S)-or (R, S)-configuration. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in the (R)-or (S)-configuration. Substituent groups on atoms with unsaturated double bonds may, if possible, be present in cis-(Z)-or trans-(E)-form.

Thus, as described herein, the compounds of the present disclosure may exist in the form of one of the possible isomers, rotamers, atropisomers, tautomers or mixtures thereof, for example, as substantially pure geometric (cis or

trans) isomers, diastereomers, optical isomers (enantiomers), racemates or mixtures thereof.

Any resulting mixtures of isomers can be separated on the basis of the physicochemical differences of the constituents, into the pure or substantially pure geometric or optical isomers, diastereomers, and racemates, for example, by chromatography and/or fractional crystallization.

Any resulting racemates of final products or intermediates can be resolved into the optical antipodes by methods familiar to those skilled in the art by known methods, e.g. by resolving the diastereomeric salts thereof. Racemic products can also be resolved by chiral chromatography, e.g. high-pressure liquid chromatography (HPLC) using a chiral adsorbent. In particular, enantiomers can also be prepared by asymmetric syntheses (e.g. Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); and Wilen, S.H. Tables of Resolving Agents and Optical Resolutions p. 268 (E.L. Eliel, Ed., Univ. of Notre Dame Press, Notre Dame, IN 1972).

As described herein, the compounds of the present disclosure may be optionally substituted by one or more substituent groups, such as the compounds of the above general formula, or as specific examples in the examples, subclasses, and species encompassed by the present disclosure It is to be understood that the term "optionally substituted" is used interchangeably with the term "substituted or unsubstituted". The terms "optionally", "optional" or "option" means the subsequently described events or conditions that can but may not necessarily occur, and the description includes the circumstances in which the event or condition occurred, as well as the circumstances in which the event or condition did not occur. In general, the term "optionally", whether preceded by the term "substituted" or not, refers to one or more hydrogen atoms in a given structure have been replaced by the specified substituent. Unless otherwise indicated, an optional substituent group may be substituted at each substitutable position of the group. When more than one position in a given formula can be substituted with one or more substituent groups selected from a specified group, the substituent groups may be the same or different at each position. Wherein the substituent groups may be, but are not limited to, F, Cl, Br, CN, $N_3$, OH, NH2, $NO_2$, oxo (= O), and the like.

The term "$N_3$" denotes an azide structure. This group may be linked to other groups, for example to a methyl group to form azido methane ($MeN_3$), or to a phenyl group to form azido benzene ($PhN_3$).

In addition, it should be noted that, unless explicitly stated otherwise, the expressions "each... independently" and "... each independently" and "... independently" used in the present disclosure are interchangeable and should be understood in a broad sense to mean that no particular option expressed between the same symbols in different groups influences each other, or that no particular option expressed between the same symbols in the same groups influences each other.

In each part of this specification, the substituent groups of the presently disclosed compounds are disclosed in terms of types or ranges of the groups. It is specifically intended that the present disclosure include each individual sub-combination of the individual members of these types and ranges of the groups. For example, the term "$C_{1-6}$ alkyl" specifically refers to the independently disclosed methyl, ethyl, $C_3$ alkyl, $C_4$ alkyl, $C_5$ alkyl, and $C_6$ alkyl groups.

In each part of the present disclosure, linking substituent groups are described. When the structure clearly requires a linking group, the Markush variables recited for that group are understood to be linking groups. For example, if the structure requires a linking group and the Markush group definition for that variable recites "alkyl" or "aryl", it is understood that the "alkyl" or "aryl" represents a linked alkylene group or arylene group, respectively.

As used in the present disclosure, the term "alkyl" or "alkyl group" means a saturated linear or branched monovalent hydrocarbon radicals containing 1-20 carbon atoms. Unless otherwise specified, the alkyl group contains 1-20 carbon atoms; in some embodiments, the alkyl group contains 1-10 carbon atoms; in other embodiments, the alkyl group contains 1-9 carbon atoms; in other embodiments, the alkyl group contains from 1-8 carbon atoms; in other embodiments, the alkyl group contains 1-6 carbon atoms; in other embodiments, the alkyl group contains 1-4 carbon atoms; in other embodiments, the alkyl group contains 1-3 carbon atoms; in other embodiments, the alkyl group contains 10-20 carbon atoms; in other embodiments, the alkyl group contains 10-18 carbon atoms; in other embodiments, the alkyl group contains 10-16 carbon atoms; in other embodiments, the alkyl groups contains 10-14 carbon atoms; in other embodiments, the alkyl group contains 10-13 carbon atoms.

Examples of alkyl groups include, but are not limited to, methyl (Me, $-CH_3$), ethyl (Et, $-CH_2CH_3$), n-propyl (n-Pr, $-CH_2CH_2CH_3$), isopropyl (i-Pr, $-CH(CH_3)_2$), n-butyl (n-Bu, $-CH_2CH_2CH_2CH_3$), isobutyl (i-Bu, $-CH_2CH(CH_3)_2$), sec-butyl (s-Bu, $-CH(CH_3)CH_2CH_3$), tert-butyl (t-Bu, $-C(CH_3)_3$), n-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), n-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)2CH(CH_3)_2$), 3,3-dimethyl-2-butyl ($-CH(CH_3)C(CH_3)_3$), n-heptyl, n-octyl, and the like, wherein the alkyl groups may be independently unsubstituted or substituted by one or more substituent groups described in the

present disclosure.

As used in the present disclosure, the term "alkyl group" and its prefix "alkyl" both include straight and branched saturated carbon chains.

The term "alkenyl" refers to a linear or branched monovalent hydrocarbon group of 2-20 carbon atoms, 2-18 carbon atoms, 2-16 carbon atoms, 2-12 carbon atoms, 2-8 carbon atoms, 2-6 carbon atoms, or 2-4 carbon atoms, wherein at least one position C-C is in the sp2 double bond unsaturated state, wherein the alkenyl group may be independently unsubstituted or substituted by one or more substituent groups described herein, including groups with "cis" "trans" or "Z" "E" positions, wherein specific examples include, but are not limited to, vinyl ($-CH=CH_2$), allyl ($-CH_2CH=CH_2$) and the like.

The term "alkynyl" refers to a linear or branched monovalent hydrocarbon group of 2-20 carbon atoms, 2-18 carbon atoms, 2-16 carbon atoms, 2-12 carbon atoms, 2-8 carbon atoms, 2-6 carbon atoms, or 2-4 carbon atoms, wherein at least one position C-C is in the sp triple bond unsaturated state, wherein the alkynyl group may be independently unsubstituted or substituted by one or more substituent groups described herein, specific examples include, but are not limited to, ethynyl ($-C=CH$), propargyl ($-CH_2C\equiv CH$), 1-propynyl ($-C\equiv C-CH_3$) and the like.

The term "comprising" or "including" is an open expression, i.e. to include the content specified in the present disclosure, but not to exclude other aspects.

As described in the present disclosure,, the term "pharmaceutically acceptable carrier" includes any solvents, dispersion media, coatings, surfactants, antioxidants, preservatives (e.g. antibacterial agents, antifungal agents), isotonic agents, salts, drug stabilizers, binders, excipients, dispersing agents, lubricants, sweetening agents, flavoring agents, coloring agents, or combinations thereof, these carriers are known to those skilled in the art (as described in Remington's Pharmaceutical Sciences, 18th Ed. Mack Printing Company, 1990, pp. 1289-1329). Except for cases where any conventional carrier is incompatible with the active ingredient, its use in treatment or pharmaceutical composition is covered.

**Compound**

In the first aspect of the present disclosure, the present disclosure provides a compound represented by formula (I) or a stereoisomer, tautomer, solvate pharmaceutically acceptable salt of the compound represented by formula (I),

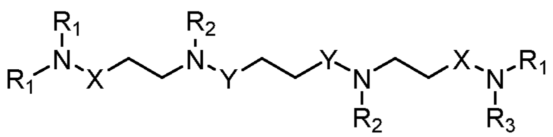

(I)

wherein X, Y, $R_1$, $R_2$, $R_3$ have the definitions as described in the present disclosure.

In some embodiments, each X and Y are independently selected from $-CH_2-$ or -CO-, respectively; X and Y are not $-CH_2$ - or -CO- at the same time, wherein each X is the same or different and each Y is the same or different; $R_2$ is selected from H, optionally substituted $C_1$-$C_{20}$ alkyl, optionally substituted $C_1$-$C_{20}$ alkenyl, or optionally substituted $C_1$-$C_{20}$ alkyne; $R_3$ is selected from H, optionally substituted $C_1$-$C_{20}$ alkyl, optionally substituted $C_1$-$C_{20}$ alkenyl, or optionally substituted $C_1$-$C_{20}$ alkyne, where the substituent groups are independently selected from H, F, Cl, Br, CN and $NO_2$; $R_1$ is selected from

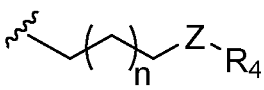 or 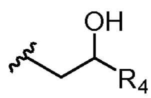,

wherein n is an integer selected from 0-10, and Z is selected from

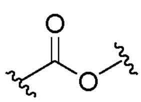 or 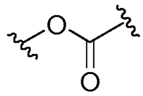 ;

$R_4$ is selected from optionally substituted $C_4$-$C_{20}$ alkyl, optionally substituted $C_4$-$C_{20}$ alkyne, or optionally substituted $C_4$-$C_{20}$ alkyne, wherein the substituent groups are independently selected from H, F, Cl, Br, CN, and $NO_2$.

In some embodiments, $R_2$ is selected from H, $CH_3$-, or $R_1$; $R_3$ is selected from H or $R_1$; $R_1$ is selected from

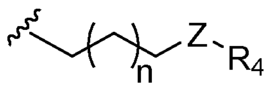 or 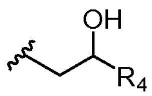 ,

wherein n is an integer selected from 1-5, and Z is selected from

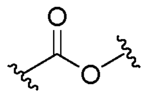 or 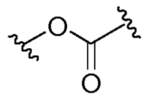 ;

$R_4$ is selected from $C_4$-$C_{20}$ alkyl, $C_4$-$C_{20}$ alkenyl or $C_4$-$C_{20}$ alkynyl, wherein the $C_4$-$C_{20}$ alkenyl contains 1-3 double bonds and the $C_4$-$C_{20}$ alkynyl contains 1-3 triple bonds.

In some embodiments, $R_4$ is selected from $C_5$-$C_{15}$ alkyl, and $C_5$-$C_{15}$ alkenyl, wherein the $C_5$-$C_{15}$ alkenyl contains 1-3 double bonds.

In some embodiments, $R_1$, $R_2$, and $R_3$ are the same; $R_4$ is selected from $C_{10}$-$C_{15}$ alkyl, $C_{10}$-$C_{15}$ alkenyl, wherein the $C_{10}$-$C_{15}$ alkenyl contains 1-3 double bonds.

Further, the compound has one of the following structures:

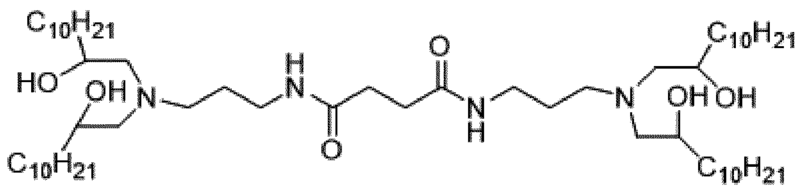 ;

(A4)

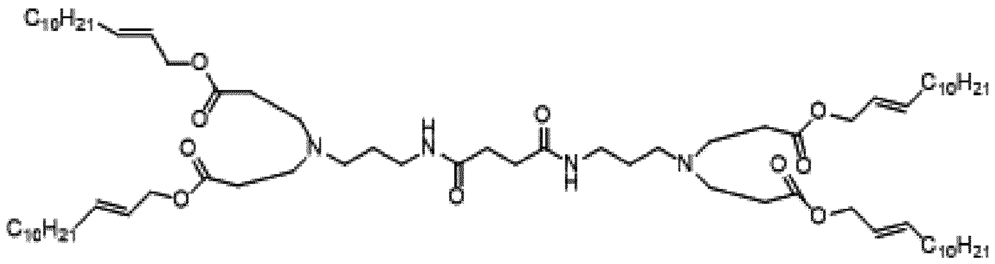 ;

(A10)

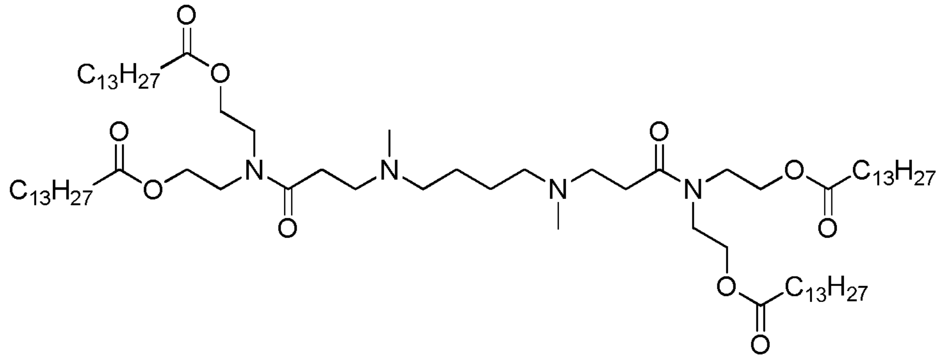

;

(B8)

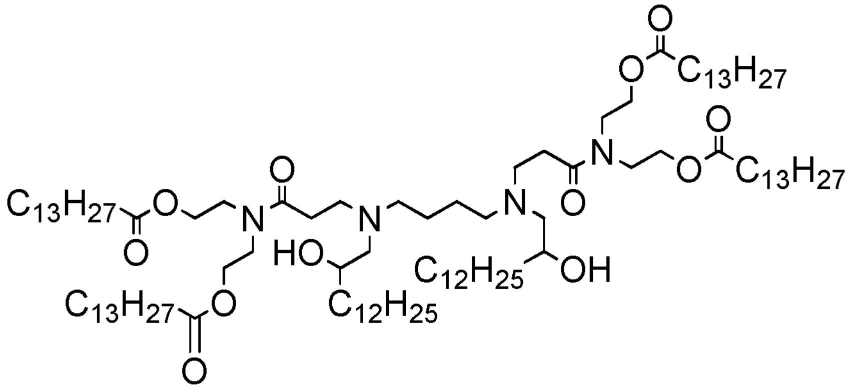

(C3).

The compounds described in the present disclosure are those having a hydrophilic head and a hydrophobic tail on a macromolecular chain. Wherein the hydrophilic head can be N1,N4-bis(3-aminopropyl)succinamide or 3,3'-(butane-1,4-diylbis(methylazanediyl)) dipropanamide or 3,3'-(butane-1,4-diylbis((2-hydroxypropyl) azanediyl) dipropanamide. The hydrophobic tail may be a straight carbon chain with double and/or ester bonds, and the total chain length can be 12, 14, 16, or 18 atoms. The compounds of the disclosure may be formed by one of the hydrophilic heads and one or more of the hydrophobic tails according to various ratios such as 1: 1, 1: 2, or 1: 3.

In some embodiments, the hydrophobic tail has at least one of the following chemical structures:

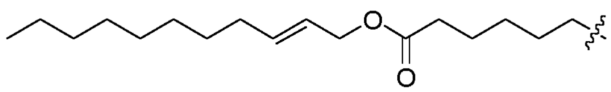

Formula 1

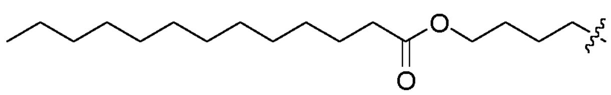

Formula

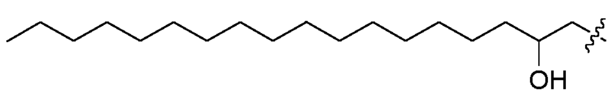

Formula 3

wherein the positions of the double bond and the ester bond in Formula 1 can be varied, according to convenient availability of the raw materials, the total chain length is set to be 16 atoms or 18 atoms; the position of the ester bond in Formula 2 can be varied, according to convenient availability of the raw materials, and the total chain length is set to 14, 16 or 18 atoms; the chain length in Formula 3 can be varied and can be 12, 14, 16, 18 (more preferably 12, 14 atoms).

In some embodiments, the salt refers to a pharmaceutically acceptable salt. The term "pharmaceutically acceptable" means that the substance or composition must be compatible chemically and/or toxicologically, with the other ingredients comprising a formulation, and/or the mammal being treated therewith.

The compounds of the present disclosure also include other salts of such compounds, which are not necessarily pharmaceutically acceptable salts, and which can be used for for the preparation and/or purification of the compounds of the present disclosure and/or for the separation of the intermediates of of enantiomers of the compounds of the present disclosure.

Pharmaceutically acceptable acid addition salts can be formed with inorganic acids and organic acids, for example, acetate, aspartate, benzoate, benzene sulfonate, bromide/hydrobromide, bicarbonate/carbonate, bisulphate/sulphate, camphorsulphonate, chloride/hydrochloride, teoclate, citrate, edisylate, fumarate, gluceptate, gluconate, glucuronate, hippurate, hydroiodide/iodide, isethionate, lactate, lactobionate, laurylsulphate, malate, maleate, malonate, mandelate, methanesulfonate, methyl sulfate, naphthoate, naphthalenesulfonate, nicotinate, nitrate, octadecanoate, oleate, oxalate, palmitate, pamoate, phosphate/hydrogen phosphate/dihydrogen phosphate, poly-galactonate, propionate, stearate, succinate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like.

Pharmaceutically alkali addition salts can be formed with inorganic and organic bases.

Inorganic bases from which salts can be derived include, for example, ammonium salts and metals from Groups I to XII of the Periodic Table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include primary, secondary, and tertiary amines, and substituted amines including naturally occurring substituted amines, cyclic amines, alkaline ion exchange resins, etc. Certain organic amines include, for example, isopropylamine, benzathine, cholinate, diethanolamine, diethylamine, lysine, meglumine, piperazine, and tromethamine.

The pharmaceutically acceptable salts of the present disclosure can be synthesized from a parent compound, a basic or acidic moiety, by conventional chemical methods. Generally, such salts can be prepared by reacting the free acid forms of these compounds with a stoichiometric amount of the appropriate base (such as Na, Ca, Mg, or K hydroxides, carbonates, bicarbonates or the like), or by reacting the free base forms of these compounds with a stoichiometric amount of the appropriate acids. Such reactions are typically carried out in water or an organic solvent or a mixture of both. Generally, nonaqueous media like ether, ethyl acetate, ethanol, isopropanol, or acetonitrile need to be used where appropriate. Lists of additional suitable salts can be found in, for example, "Remington's Pharmaceutical Sciences", 20th Edition, Mack Publishing Company, Easton, Pa. (1985); and "Handbook of Pharmaceutical Salts: Properties, Selection, and Use", Stahl and Wermuth (Wiley-VCH, Weinheim, Germany, 2002).

Furthermore, the compounds of the present disclosure, including the salts thereof, can also be obtained in the form of the hydrates thereof, or include other solvents used for the crystallization. The compounds of the present disclosure can form solvates with pharmaceutically acceptable solvents (including water) inherently or by design; thus, the present disclosure is intended to include both solvated and unsolvated forms.

Any formula given in the present disclosure is also intended to represent unlabeled forms as well as isotopically labeled forms of these compounds. The isotopically labeled compounds have structures depicted by the formulas given herein except that one or more atoms are replaced by an atoms having a selected atomic mass or mass number. Exemplary isotopes that can be incorporated into compounds of the present disclosure include isotopes of hydrogen, carbon, nitrogen, oxygen, phosphorous, sulfur, fluorine, and chlorine, such as $^{2}H$, $^{3}H$, $^{11}C$, $^{13}C$, $^{14}C$, $^{15}N$, $^{18}F$, $^{31}P$, $^{32}P$, $^{36}S$, $^{37}Cl$, or $^{125}I$.

In another aspect, the compounds of the present disclosure include compounds as defined herein labeled with various isotopes, for example, those compounds in which radioactive isotopes, such as $^{3}H$, $^{14}C$, and $^{18}F$ are present, or those compounds in which non-radioactive isotopes, such as $^{2}H$ and $^{13}C$ are present. Such isotopically labeled compounds are useful in metabolic studies (with $^{14}C$), reaction kinetic studies (with, for example, $^{2}H$ or $^{3}H$), detection or imaging techniques, such as positron emission tomography (PET) or single-photon emission computed tomography (SPECT) including drug or substrate tissue distribution assays, or in radiotherapy of patients. $^{18}F$-labeled compounds are particularly desirable for PET or SPECT studies. Isotopically-labeled compounds of formula (I) can be prepared by

conventional techniques familiar to those skilled in the art or as described in the examples and preparations herein using an appropriate isotopically-labeled reagent in place of the unlabeled reagent previously employed.

Further, substitution with heavier isotopes, particularly deuterium (i.e. $^2H$ or D) may afford certain therapeutic advantages resulting from greater metabolic stability, for example, resulting from increased *in vivo* half-life or reduced dosage requirements, or an improvement in therapeutic index. It should be understood that deuterium in this context is regarded as a substituent of a compound of the formula (I). The concentration of such heavier isotopes, particularly deuterium, may be defined by the isotopic enrichment factor. As used herein, the term "isotopic enrichment factor" refers to the ratio between the isotopic abundance and the natural abundance of a specified isotope. If a substituent in a compound of the present disclosure is designated as deuterium, for each designated deuterium atom, such compound has an isotopic enrichment factor of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation) at least 6600 (99% deuterium incorporation) or at least 6633.3 (99.5% deuterium incorporation). Pharmaceutically acceptable solvates in accordance with the present disclosure include those wherein the solvent of crystallization may be isotopically substituted, e.g. $D_2O$, acetone-d6, or DMSO-d6.

**Liposome and preparation method**

In another aspect of the present disclosure, the present disclosure provides a liposome. The liposome according to the embodiments of the present disclosure is made of the compound of the first aspect of the present disclosure. The liposome can encapsulate the added substance to be loaded by the flow action of the lipid bilayer, with good encapsulation effect, so that the loaded substance can efficiently enter cells and escape from the endosome to the cytoplasm.

The aforementioned compound may form the liposome by self-assembly with a hydrophobic lipid.

In some embodiments, the liposome further includes a hydrophobic lipid, an amphiphilic lipid, a buffering agent, and/or an organic solvent, wherein the amphiphilic lipid includes at least one selected from a neutral lipid and a PEG lipid, wherein the hydrophobic lipid includes a sterol.

In some embodiments, the neutral lipid comprises at least one selected from the group consisting of distearoyl phosphatidyl choline, dipalmitoyl phosphatidyl choline, phosphatidyl choline, dioleoyl phosphatidyl ethanolamine, dilauroyl phosphatidyl choline, diethyl pyrocarbonate, dimyristoyl phosphatidyl choline, and egg phosphatidylcholine.

In some embodiments, the PEG-lipid comprises at least one selected from the group consisting of dipalmitoyl phosphatidyl ethanolamine-PEG, distearoyl phosphatidyl ethanolamine-PEG, dimyristyl glycerol, and dimethacrylate.

In some embodiments, the sterol is cholesterol.

In some embodiments, the buffering agent is an acidic buffering agent.

In some embodiments, the buffering agent comprises at least one selected from citric acid, sodium citrate, acetic acid, sodium acetate, disodium hydrogen phosphate, sodium dihydrogen phosphate, trihydroxymethylaminomethane-hydrochloric acid, potassium dihydrogen phosphate-sodium hydroxide, boric acid-borax, glycine-hydrochloric acid, phthalic acid-hydrochloric acid, potassium hydrogen phthalate, and sodium dihydrogen phosphate-citric acid.

In some embodiments, the organic solvent comprises at least one selected from methanol, ethanol, isopropanol, benzene, toluene, xylene, pentane, hexane, octane, cyclohexane, cyclohexanone, toluene cyclohexanone, chlorobenzene, dichlorobenzene, dichloromethane, ethyl ether, propylene oxide, acetone, methyl butanone, methyl isobutyl ketone, acetonitrile, pyridine, phenol, styrene, perchloroethylene, trichloroethylene, vinyl ethylene glycol ether, and triethanolamine.

In some embodiments, based on the liposome, the amount of the compound is 20-80% mol/mol, further the amount of the compound is 20% mol/mol, 21% mol/mol, 22% mol/mol, 23% mol/mol, 24% mol/mol, 25% mol/mol, 26% mol/mol, 27% mol/mol, 28% mol/mol, 29% mol/mol, 30% mol/mol, 31% mol/mol, 32% mol/mol, 33% mol/mol, 34% mol/mol, 35% mol/mol, 36% mol/mol, 37% mol/mol, 38% mol/mol, 39% mol/mol, 40% mol/mol, 41% mol/mol, 42% mol/mol, 43% mol/mol, 44% mol/mol, 45% mol/mol, 46% mol/mol, 47% mol/mol, 48% mol/mol, 49% mol/mol, 50% mol/mol, 51% mol/mol, 52% mol/mol, 53% mol/mol, 54% mol/mol, 55% mol/mol, 56% mol/mol, 57% mol/mol, 58% mol/mol, 59% mol/mol, 60% mol/mol, 61% mol/mol, 62% mol/mol, 63% mol/mol, 64% mol/mol, 65% mol/mol, 66% mol/mol, 67% mol/mol, 68% mol/mol, 69% mol/mol, 70% mol/mol, 71% mol/mol, 72% mol/mol, 73% mol/mol, 74% mol/mol, 75% mol/mol, 76% mol/mol, 77% mol/mol, 78% mol/mol, 79% mol/mol, and 80% mol/mol.

In some embodiments, the molar ratio of the compound, neutral lipid, sterol, and PEG-lipid is (20-80): (5-50): (10-60): (0.2-20).

In some embodiments, the volume of the buffering agent is 50-90% v/v based on the liposome. Further, the volume of the buffering agent is 50% v/v, 51% v/v, 52% v/v, 53% v/v, 54% v/v, 55% v/v, 56% v/v, 57% v/v, 58% v/v, 59% v/v, 60% v/v, 61% v/v, 62% v/v, 63% v/v, 64% v/v, 65% v/v, 66% v/v, 67% v/v, 68% v/v, 69% v/v, 70% v/v, 71% v/v, 72% v/v, 73% v/v, 74%v/v, 75%v/v, 76%v/v, 77%v/v, 78%v/v, 79%v/v, 80%v/v, 81%v/v, 82%v/v, 83%v/v, 84%v/v,

85%v/v, 86%v/v, 87%v/v, 88%v/v, 89%v/v, and 90%v/v.

In some embodiments, the volume of the buffering agent is 75% v/v based on the liposome.

In some embodiments, the aforementioned compound may also form the liposome with a phospholipid derivative, including but not limited to, distearoyl phosphatidyl choline, distearoyl phosphatidyl ethanolamine, dioleoyl phosphatidyl ethanolamine.

In a particular embodiment, the liposome comprises the aforementioned compound, and further, the liposome further comprises a neutral lipid and a lipid capable of reducing aggregation of liposome particles.

In a particular embodiment, the liposome essentially consists of: (i) at least one lipid of the present disclosure; (ii) a neutral lipid selected from DSPC, DPPC, POPC, DOPE, DLPC, DEPC, DMPC, and EPC, and the like; (iii) a sterol such as cholesterol; and (iv) PEG-lipids, such as DPPE-PEG, DSPE-PEG, PEG-DMG or PEG-DMA, composed in a molar ratio of approximately 20-80% amine-containing lipid: 5-50% neutral lipid: 10-60% sterol: 0.2-20% PEG lipid.

In another aspect of the present disclosure, the present disclosure provides a method for preparing the aforementioned liposome. According to an embodiment of the present disclosure, the method comprises mixing a predetermined amount of a compound of the first aspect of the present disclosure with an organic solvent, so as to obtain the liposome.

In some embodiments, the aforementioned compound is present in the organic solution in a concentration of 10-30 mg/mL, further, the aforementioned compound is present in the organic solution in a concentration of 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, and 30 mg/mL.

In some embodiments, the method further comprises: dissolving a predetermined amount of hydrophobic lipid and amphipathic lipid in the aforementioned organic solution and then mixing with a buffering agent to obtain the liposome. According to an embodiment of the present disclosure, the amount of the compound is 20-80% mol/mol based on the liposome; the mass ratio of the compound, neutral lipid, sterol, and PEG-lipid is (20-80): (5-50): (10-60): (0.2-20); the volume of the buffering agent is 75% v/v based on the liposome.

In some embodiments, based on the liposome, the amount of the compound is 20% mol/mol, 21% mol/mol, 22% mol/mol, 23% mol/mol, 24% mol/mol, 25% mol/mol, 26% mol/mol, 27% mol/mol, 28% mol/mol, 29% mol/mol, 30% mol/mol, 31% mol/mol, 32% mol/mol, 33% mol/mol, 34% mol/mol, 35% mol/mol, 36% mol/mol, 37% mol/mol, 38% mol/mol, 39% mol/mol, 40% mol/mol, 41% mol/mol, 42% mol/mol, 43% mol/mol, 44% mol/mol, 45% mol/mol, 46% mol/mol, 47% mol/mol, 48% mol/mol, 49% mol/mol, 50% mol/mol, 51% mol/mol, 52% mol/mol, 53% mol/mol, 54% mol/mol, 55% mol/mol, 56% mol/mol, 57% mol/mol, 58% mol/mol, 59% mol/mol, 60% mol/mol, 61% mol/mol, 62% mol/mol, 63% mol/mol, 64% mol/mol, 65% mol/mol, 66% mol/mol, 67% mol/mol, 68% mol/mol, 69% mol/mol, 70% mol/mol, 71% mol/mol, 72% mol/mol, 73% mol/mol, 74% mol/mol, 75% mol/mol, 76% mol/mol, 77% mol/mol, 78% mol/mol, 79% mol/mol, and 80% mol/mol.

## Drug carrier, pharmaceutical composition, and transfection complex

In another aspect of the present disclosure, the present disclosure provides a drug carrier comprising the aforementioned compound or liposome. According to an embodiment of the present disclosure, the compound is an ionizable amine-containing lipid, and the drug carrier is an ionizable carrier that can be loaded with a negatively charged drug, and deliver the drug into a cell and efficiently escape from the endosome to the cytoplasm, facilitating the pharmacodynamic exertion of the loaded drug.

In another aspect of the present disclosure, the present disclosure provides a pharmaceutical composition comprising a carrier and a pharmaceutically active ingredient, wherein the carrier comprises a drug carrier of the fifth aspect of the present disclosure. According to an embodiment of the present disclosure, the drug carrier is an ionizable carrier and can be loaded with a negatively charged pharmaceutical active ingredient, and deliver the pharmaceutical active ingredient into a cell and efficiently escape from the endosome to the cytoplasm, facilitating the pharmacodynamic exertion of the loaded pharmaceutical active ingredient.

In some embodiments, the pharmaceutically active ingredient comprises at least one selected from: a DNA molecule, an RNA molecule, a protein and a hydrophobic drug. According to an embodiment of the present disclosure, the drug carrier contains a long carbon chain and may be loaded with a hydrophobic substance, for example, a hydrophobic small molecule drug.

Further, the drug carrier of the present disclosure may also be loaded with proteins, polypeptides, which are not particularly limited, and may be a peptide chain, a protein itself, a derivative of the above substances or a complex with other substances, e.g. Cas9 protein or a partial domain peptide fragment thereof, a nuclide-loaded protein, antibody and other substances.

Further, the drug carrier of the present disclosure may also be loaded with nucleic acid substances, e.g. an antisense nucleic acid and the like.

In some embodiments, the pharmaceutically active ingredients are negatively charged. According to an embodiment of the present disclosure, the drug carrier comprises an ionizable amine-containing lipid that is positively charged at a specific pH and can be combined with a negatively charged pharmaceutically active ingredient, and in addition, the pharmaceutically active ingredient can be encapsulated by the flow action of the lipid bilayer and presented to the cell.

In some embodiments, the pharmaceutically active ingredient contains nucleic acid. According to an embodiment of the present disclosure, the pharmaceutically active ingredient contains DNA, RNA, nucleic acid-protein complex, nucleic acid-lipid complex, nucleic acid-nuclide complex, and the like, and the nucleic acid type is not particularly limited. As a specific example, the nucleic acid can be siRNA, mRNA, tRNA, rRNA, cDNA, miRNA (micro RNA), ribozyme, antisense oligonucleotides, plasmid DNA, peptide nucleic acid, triplex-forming oligonucleotide (TFO), gene and the like. Among other things, the carriers of the present disclosure are particularly effective at transporting siRNA into cells. The nucleic acid to which the carrier of the present disclosure is applied may be a nucleic acid derived from a human, animal, plant, bacterium, virus, etc. or a nucleic acid prepared by chemical synthesis. Further, the nucleic acid may be any one of single-stranded, double-stranded, and triple-stranded, and the molecular weight thereof is not particularly limited. In addition, in the present disclosure, the nucleic acid may be chemically, enzymatically or peptide-modified. In the present disclosure, nucleic acid can be used alone or in appropriate combinations of two or more. In a preferred embodiment, the nucleic acid transfer carrier compositions of the disclosure preferably transfer small interfering nucleic acids (siRNA) or analogs thereof.

In some embodiments, the mass ratio of the carrier to the pharmaceutically active ingredient is (5-40): 1.

In some embodiments, the mass ratio of the carrier to the pharmaceutically active ingredient is (8-20): 1.

In some embodiments, the mass ratio of the carrier to the pharmaceutically active ingredient is 5: 1, 6: 1, 7: 1, 8: 1, 9: 1, 10: 1, 11: 1, 12: 1, 13: 1, 14: 1, 15: 1, 16: 1, 17: 1, 18: 1, 19: 1, 20: 1, 21: 1, 22: 1, 23: 1, 24: 1, 25: 1, 26: 1, 27: 1, 28: 1, 29: 1, 30: 1, 31: 1, 32: 1, 33: 1, 34: 1, 35: 1, 36: 1, 37: 1, 38: 1, 39: 1, and 40: 1.

In some embodiments, the mass ratio of the carrier to the pharmaceutically active ingredient is 15: 1.

In another aspect of the present disclosure, the present disclosure provides a transfection complex. According to an embodiment of the present disclosure, the transfection complex comprises the aforementioned compound or the aforementioned liposome.

In some embodiments, a helper lipid suitable for preparing and forming the transfection complexes disclosed herein may include, but are not limited to, cholesterol, cholesterol derivatives, or any of neutral or cationic lipids known to effect or facilitate the introduction of exogenous bioactive molecules into cells or tissues. In certain embodiments, more than one helper lipid may be used in the formulation of the transfection complex described herein.

In some embodiments, the transfection complex comprises at least one bioactive agent. The transfection complex according to embodiments of the present disclosure are suitable for the delivery of one or more bioactive agents to cells, tissues or organs in vitro or *in vivo.*

In some embodiments, the transfection complex may include one or more bioactive agents to be delivered to cells or to target tissues in vitro or *in vivo.* Suitable bioactive agents may include any molecule that is capable of forming a transfection complex with a transfection agent described herein and that can cause a biological response when delivered to the interior of one or more cells or to tissues *in vivo* or in vitro. Bioactive agents for use in embodiments described herein may be cationic, neutral or anionic agents.

In some embodiments, the bioactive agent comprises at least one selected from a group consisting of a DNA molecule, an RNA molecule, a protein, and a drug. According to an embodiment of the present disclosure, an exemplary bioactive agent suitable for formulating transfection complexes may include but are not limited to a nucleic acid (including, but not limited to, single--stranded or double-stranded linear or cyclic DNA molecules (including a cDNA molecule), a single--stranded or double-stranded RNA molecule, a small interfering RNA (siRNA) molecule, a small hairpin RNA (shRNA) molecule, a micro RNA (miRNA) molecule, an oligonucleotide, an antisense oligonucleotide, and a sense oligonucleotide), a polypeptide, an antibody, an oligopeptide, a therapeutic peptide or protein molecule, a peptide nucleic acid (PNA), a cationic, anionic or neutral organic molecule or drug, and pharmaceutically acceptable salts thereof.

In some embodiments, the transfection complex described herein may optionally include one or more fused peptides or cell-penetrating peptides. The fused peptides or cell-penetrating peptides is any peptide macromolecule capable of promoting the fusion of a lipid-containing complex with a cell membrane (plasma membrane or endosomal membrane). A variety of fused or cell-penetrating peptides are known in the art, and suitable fused or cell-penetrating peptides and conditions for their use in the present disclosure can be identified without undue experimentation within the skill of the practitioner.

In some embodiments, the transfection complex described herein may optionally include one or more transfection-facilitating agents or targeting moieties. The targeting moiety may be a peptides, a modified peptides, an antibody, a modified antibody, a receptor molecule, a modified receptor molecule, a single-stranded or double-stranded nucleic acid molecule, a modified single-stranded or double-stranded nucleic acid molecule, a peptide or nucleic acid aptamer, a modified peptide or nucleic acid aptamer, an organic molecule, a polysaccharide, or any other molecule capable of targeting the transfection complex to a particular tissue or cell type for targeted delivery of the biologically active agent

thereto.

In some embodiments, the transfection complexes provided herein may be stable for up to 1 year and may be contacted with the cell or tissue to be transfected, or administered to the animal or human immediately or shortly after formation, or may optionally be stored for a period of time prior to contact with the cell or tissue or administration to the animal or human. The transfection complex is stable and can be stored at room temperature or at a temperature greater than freezing point up to about room temperature for a period of at least 30 minutes, at least 45 minutes, at least 1 hour, at least 2 hours, at least 3 hours, at least 4 hours, at least 5 hours, at least 10 hours, at least 15 hours, at least 20 hours, at least 24 hours, at least 48 hours, at least 72 hours, at least 5 days, at least 7 days, at least 14 days, at least 28 days, at least 1 month, at least 2 months, at least 3 months, at least 4 months, at least 5 months, at least 6 months, or at least 1 year. It should be understood that the formulations described herein may include one or more stabilizers, preservatives, buffers, and the like, which aid in the long-term stability and storage of the biologically active formulation, as would be readily understood by a practitioner skilled in the biological and pharmaceutical arts, and can be realized without undue experimentation. It should also be understood that the storage period may be any of these time periods, such as 31 minutes to 1 hour, or 1 hour to 24 hours.

The present disclosure will now be described with reference to specific examples, it should be noted that these examples are intended to be illustrative only and not limiting the present disclosure in any way.

Detection and calculation methods

1. NMR spectrometer is used to determine the structure of an ammonium-containing lipid

The nuclear magnetic resonance spectrometer used is the Bruker 400M nuclear magnetic resonance spectrometer. The specific calculation methods can be seen in Boulmedais F., Frisch B., Etienne O., Lavalle Ph., Picart C., Ogier J., Voegel J-C., Schaaf P., Egles C.Polyelectrolyte multilayer films with pegylated polypeptides as a new type of antimicrobial protection for biomaterials, Biomaterials, 2004, 25, 2003-2011.

2. Gel retardation assay is used to demonstrate loading of nucleic acid in the formulation

Anti-apolipoprotein B siRNA (siApoB) was dissolved in DEPC-treated water and freshly assembled with different mass ratios (w/w) of LNP. Thereafter, the mixture was mixed with 6× loading buffer (Takara Bio Technology Beijing Co. Ltd.), and a formulation containing an equal amount of siRNA was added to a 1% (w/w) agarose gel containing 0.01% gel stain (w/w) (TransGen Biotech Beijing). Electrophoresis was performed at a voltage of 90V for 30 minutes in a 1× TAE electrophoresis buffer. The results were recorded with a gel imaging system (Shanghai Tanon Technology Co. Ltd.) at a UV wavelength of 320 nm. The siApoB sequence is shown in Table 1

Table 1

| Name | SEQ ID NO. | Nucleotide Sequence (5'→3') |
|---|---|---|
| siApoB | 1 | GUCAUCACACUGAAUACCAAUdTdT |
| | 2 | AUUGGUAUUCAGUGUGAUGACACdTdT |

3. Determination of particle size and surface potential to preliminarily determine whether lipid formulations are available

The formulation obtained after encapsulating siRNA with LNP was first diluted 10 times before measuring size and surface potential. The instrument used for particle size and surface potential measurement is Zetasizer 3000HS laser particle size analyzer manufactured by Malven, USA. The measurement temperature was 25°C, the angle was 90°, and the incident light wavelength was 677 nm.

4. Determination of siRNA concentration in the formulation

The siRNA concentration was determined using the RiboGreen method. The formulation to be tested was diluted 20 times and taken 100 μL into a 96-well plate to detect the amount of external siRNA fluorescence of the formulation. Another 15 μL of diluted formulation to be tested was taken. 50 μL of 2% Triton-X-100 (w/w) mixture was added to destroy lipid structure so as to completely release internal siRNA. Then, 35 μL of 1×TE buffer was added to the well to make up the volume, so as to detect the total amount of siRNA fluorescence of the formulation. In addition, a standard curve was plotted by using an siRNA standard, 1×TE, and 2% Triton-X-100, which is used to calculate the concentration of siRNA in the sample to be tested. After all the samples to be tested were loaded, 30 minutes of incubation

was performed at room temperature. 100 μL/well RiboGreen working solution was then added in the dark and immediately subjected to fluorescence detection with a microplate reader. The detection conditions are shown in Table 2:

Table 2:

| Parameter | Set |
|---|---|
| Test method | Fluorescent |
| Type | Endpoint/kinetic |
| Optical element type | Monochromator |
| Excitation light | 480/17.0 |
| Emitted light | 520/17.0 |
| Optical element position | Top |
| Gain | 60 |

5. Transmission electron microscopy (TEM) experiment is used to detect the particle morphology of the formulations

The transmission electron microscope used was a JEM-100CX II transmission electron microscope from the Netherlands. The copper mesh was first immersed in the solution of the sample to be tested, and then dyed with a 0.1 wt% phosphotungstic acid solution. After about 3 minutes, the excess liquid was filtered off with filter paper and air-dried at room temperature. The morphology of the particles in the system was observed and captured by transmission electron microscopy.

6. *In vitro* transfection experiment

For the siRNA delivery efficiency of candidate formulations,human hepatocellular carcinoma cell line HepG2 was seeded in a 12-well plate in a DMEM complete medium containing 10% fetal bovine serum at a density of $1\times10^5$ cells/well in 1 mL medium per well and cultured overnight at 37°C.

On the second day, the cell culture medium in the 12-well plate was discarded. 1 mL of fresh DMEM complete medium containing 10% (v/v) fetal bovine serum was added to each well of all the wells to be transfected. The Mock group was not further treated and the corresponding formulation was added to the sample wells to be tested and the sample volume was calculated based on the siRNA concentration in the LNP previously determined using the RiboGreen method such that the final concentration of siApoB was 50 nM. The cells were incubated at 37°C for 4 hours. Then, 1 mL of DMEM complete medium containing 10% fetal bovine serum was added to each well and continuously cultured continued overnight at 37°C.

Thereafter, the relative expression of ApoB mRNA in the samples to be tested was detected using Quantitative Real-Time PCR. The specific steps were as follows: after the transfected cells was cultured for 24 hours, total RNA in the cells was extracted using RNA isolator (Nanjing Vazyme Biotechnology Co. Ltd. Cat. No. R401-01-AA), chloroform, and isopropanol; 1 μg of total RNA was respectively taken and subjected to reverse transcription in accordance with the instructions for use of the reverse transcription kit (TransGen Biotech Beijing Co. Ltd. Cat. No. AT311-03) to obtain cDNA. The expression of target gene ApoB and internal reference gene glyceraldehyde phosphate dehydrogenase (GAPDH) was detected by using quantitative real-time PCR mix (Shanghai Yisheng Biotechnology Co. Ltd. Cat. No. 11201ES08), using cDNA as a template according to the procedure in the instructions for use, wherein the expression of ApoB needs to refer to GAPDH. The PCR primers used to amplify ApoB and GAPDH as an internal reference gene are shown in Table 3.

Table 3:

| Gene name | SEQ ID NO. | Nucleotide Sequence (5'→3') |
|---|---|---|
| Human ApoB | 3 | GCCAGTCCTTCATGTCCCTA |
| | 4 | TGAAGAAAGGAGATGAGCAACAA |
| Human GAPDH | 5 | AGAAGGCTGGGGCTCATTTG |
| | 6 | AGGGGCCATCCACAGTCTTC |

7. Cytotoxicity test

The cytotoxicity of the polymer was determined by the MTT method (tetrazolium salt colorimetric method) as follows:

(1) HepG2 cells were seeded in a 96-well plate at $1.25\times10^4$ cells/well in a medium volume of 100 μL per well and incubated overnight at 37°C in 5% $CO_2$.
(2) After 24 hours, a corresponding amount of siFLA4-13 encapsulated with an insect-resistant luciferase siRNA (siFirefly) was added to each well so that the content of siRNA in the well varied from 1 nM to 200 nM. Control wells (cells, PBS at pH 7.4 with the same volume of fluid containing the formulation, media, MTT, dimethyl sulfoxide) were also set and incubated for 48 hours at 37°C in 5% $CO_2$.
(3) 20 μL of MTT solution (5 mg/mL) was added to each well and incubation continued for 4 hours at 37°C in 5% $CO_2$.
(4) The supernatant culture solution in the well was carefully discarded. 150 μL of dimethyl sulfoxide was added to each well. The plate was shaken on a low-speed shaker for 10 mins, so as to fully dissolve the crystals. The absorbance of each well was measured at 540 nm on a continuous spectrum multifunctional microplate reader (TECAN Infinite 200).
(5) The relative cell viability of each sample well was calculated according to the following formula:

$$\text{Relative viability (\%)} =(OD_{540(sample)}-OD_{650(sample)})/(OD_{540(control)}-OD_{650(control)})*100.$$

The siFirefly sequences are shown in Table 4:

Table 4

| Name | SEQ ID NO. | Nucleotide Sequence (5'→3') |
|---|---|---|
| siFirefly | 7 | CCCUAUUCUCCUUCUUCGCdTdT |
| | 8 | GCGAAGAAGGAGAAUAGGGdTdT |

8. Subcellular localization is used to determine whether the nucleic acid enters the cell

On the day before the test, $2\times10^5$ HepG2 cells will be seeded in a 35 mm culture dish and cultured overnight at 37°C in 5% $CO_2$. The next day, Cy-siFLA4-13 or Lipofectamine 2000 (Invitrogen) encapsulated with LNP attached with Cy5 group at the 3' end of SEQ ID NO. 7 was added to the corresponding culture dish and cultured for another 4 hours to facilitate cell to endocytose the transfection reagent. After the prescribed time, all the medium containing the transfection reagent was discarded and the cells on the culture dish were washed three times with 1×PBS. Thereafter, 1 mL of the working staining solution (Lysotracker diluted with 1×PBS 1: 3000 (v/v), additionally containing Hoechst 33342 0.1 mg/ml) was added to the culture dish and stained for 15 mins at 37°C in the dark. After completion of the staining, the distribution of the fluorescent signal within the cells was observed with a laser confocal microscope (LSM 700, Zeiss).

9. Cellular uptake is used to determine the efficiency of the nucleic acid entry into the cell

HepG2 cells were seeded in a 12-well plate at a concentration of $2\times10^5$ cells/mL in a volume of 1 mL/well the day before the assay. After 24 hours, Cy-siFLA4-13 was added to the wells to a final concentration of 50 nM and incubated for 4 hours at 37°C in 5% $CO_2$. Thereafter, all the culture media containing transfection reagents were discarded, and cells were digested with trypsin. The digestion of the complete culture medium was terminated, and the liquid was collected into a centrifuge tube. After centrifugation at 800 rpm for 5 mins and resuspension with 1×PBS, the cells were washed repeatedly three times. Thereafter, the endocytosis efficiency of the formulation by HepG2 cells was detected by flow cytometry (FACSverse, BD). Data were analyzed using FlowJo 7.6.

10. *in vivo* distribution test is used to determine the metabolism and distribution characteristics of the drug *in vivo*

The C57BL/6 mice of 6-8 weeks were used as the as the experimental subjects, which were divided into two groups. 1×PBS or Cy-siFLA4-13 was administered via the tail vein, respectively, in which the amount of Cy5-siFirefly was 2 mg/kg. The Cy5 signal distribution in mice is detected by the small animal *in vivo* imaging system FX Pro (Kodak) at different time points after administration. In addition, 1-2 animals per group were euthanized and the hearts, livers,

spleens, lungs, kidneys, stomachs, intestines, submandibular glands, and thymus were isolated for organ imaging after live imaging was completed at 0.5, 4, and 24 hours after administration. The result analysis was done by Carestream software and line graphs were generated by Graphpad Prism 8.

In another experiment, the experimental subjects were C57BL/6 mice of 6-8 weeks, and 1×PBS or mLuA4-12 formulation were administered, respectively, by tail vein, and the amount of mRNA was 20 μg/animal. Animals were euthanized at 6 and 9 hours after administration and the hearts, livers, spleens, lungs, kidneys, stomachs, intestines, submandibular glands, and thymus were isolated for organ imaging The result analysis was done by Carestream software.

11. Dose-dependent experiment

Thirty-two C57BL/6 mice of 6-8 weeks were divided into 8 groups and received 1 ×PBS or 0.01 mg/kg to 1 mg/kg of siA4-13, respectively. After 42 hours, animals were fasted for 6 hours and euthanized. Serum and liver tissue samples were collected for analysis of changes in serum triglyceride (TG), total cholesterol (CHO), low-density lipoprotein cholesterol (LDL-c), high-density lipoprotein cholesterol (HDL-c), and changes in mRNA expression of ApoB in the liver tissue. The PCR primers used to amplify mouse ApoB and GAPDH as internal reference genes are shown in Table 5.

Table 5:

| Gene name | SEQ ID NO. | Nucleotide Sequence (5'→3') |
|---|---|---|
| Mouse ApoB | 9 | TTCCAGCCATGGGCAACTTTACCT |
| | 10 | TACTGCAGGGCGTCAGTGACAAAT |
| Mouse GAPDH | 11 | AACTTTGGCATTGTGGAAGGGCTC |
| | 12 | TGGAAGAGTGGGAGTTGCTGTTGA |

12. Effect duration experiment

Ninety-six C57BL/6 mice of 6-8 weeks were divided into three groups and received either 1×PBS or 1 mg/kg or 3 mg/kg of siA4-13, respectively. On days 7, 14, 21, 28, 35, 42, and 49 after administration, 4 animals in each group were fasted for 6 hours and euthanized, and serum and liver tissue samples were collected for analysis of TG, CHO, LDL-c, HDL-c in serum and changes in mRNA expression of ApoB in liver tissue. The effect duration curve was generated by Graphpad Prism 8.

13. Drug Efficacy test

The test was divided into two parts. In the first part, lean receptor knockout db/db mice (C57 BLKS/JGpt-Leprem2Cd/Gpt) were used as experimental subjects, and non-knockout mice (m/m) with the same genetic background were used as controls. The aim of this part of the experiment was to examine the hypolipidemic effect and changes in the expression of target genes of the formulation siANA4-13 encapsulated with the anti-angiogenin-like protein 3 siRNA (siANGPTL3). In the second part, humanized apolipoprotein C3 (APOC3) transgenic mice (hApoC3, Tg(APOC3)3707Bres) were used as experimental subjects, and C57BL/6 mice were used as controls. The aim of this part of the experiment was to determine the hypolipidemic effect of the formulation siANA4-13 encapsulated with siApoC3 and the recovery of blood lipids in animals after drug withdrawal.

In the first part, db/db mice were injected via the tail vein with 1×PBS or 0.5 mg/kg of siFLA4-13 or 0.5 mg/kg of siANA4-13 or 0.25 mg/kg of siANA4-13, respectively. A total of 5 doses were tested once a week. On the third day after each dose, all animals will be fasted for 6 hours and the blood was collected via the orbit for analysis of changes in TG and CHO. On day 2 after the last dose, all animals will be fasted for 6 hours and then executed, and blood and liver tissue samples were collected for further determination of blood lipid changes and mRNA expression of ANGPTL3 in the liver tissue. One animal per group was harvested for oil red O staining for observation of changes in lipid deposition in the liver.

In the second part, hApoC3 mice were injected via the tail vein with 1×PBS or 0.5 mg/kg of siFLA4-13 or 0.5 mg/kg of siApoCA4-13 or 0.25 mg/kg of siApoCA4-13, respectively. The experiment was also planned to be dosed 5 times, each time at intervals of one week, during which the blood lipid changes were detected. After the last dose, all animals still received the lipid test once a week for a total of 4 times. Thereafter, the animals were euthanized and the blood was collected to detect blood lipid. The sequences of siANGPTL3 and siApoC3 involved in the experiment, and the PCR primers used to amplify mouse ANGPTL3 and human ApoC3 are shown in Tables 6 and 7, respectively:

Table 6

| Name | SEQ ID NO. | Nucleotide Sequence (5'→3') |
| --- | --- | --- |
| siANGPTL 3 | 13 | AfsCms AfUmAfUmUfUmGfAfUfCmAfGmUfCmUfUmUfUmUf |
| | 14 | AmsAfsAmAfAmGfAmCfUmGfAmUmCmAfAmAfUmAfUmGfUms UmsGm |
| siApoC3 | 15 | GfsCmsUfUmAfAmAfAmGfGfGfAmCfAmGfUmAfUmUfCmAf |
| | 16 | UmsGfsAmAfUmAfCmUfGmUfCmCmCmUfUmUfUmAfAmGfCmsA msAm |

Table 7

| Gene name | SEQ ID NO. | Nucleotide Sequence (5'→3') |
| --- | --- | --- |
| Mouse GPC3 | 17 | GAGGAGCAGCTAACCAACTTAAT |
| | 18 | TCTGCATGTGCTGTTGACTTAAT |
| Human ApoC3 | 19 | GTGACCGATGGCTTCAGTTC |
| | 20 | ATGGATAGGCAGGTGGACTT |

14. *In vitro* mRNA expression assay

HepG2 cells were seeded in a 12-well plate at a concentration of $2\times10^5$ cells/mL in a volume of 1 mL/well the day before the assay. After 24 hours, mLuA4-12 encapsulated with luciferase mRNA was added to the wells so that the amount of mRNA added to each well was 400 ng and incubated for 6 hours at 37°C in 5% $CO_2$. When the predetermined time is reached, the cells were lysed with a passive lysate and the cell lysate was collected. Thereafter, 10 μL/sample of cell lysate was added to the 96-well plate and 50 μL of luciferase substrate was added to each well. Immediately after completion, the amount of emitted fluorescence of the sample was measured with a microplate reader.

15. Comparative test of gene inhibition between siRNA and CRISPR/Cas system

HepG2-luc cells were seeded 24 hours before transfection in a 6-well plate at a concentration of $2\times10^5$ cells/mL in a volume of 1 mL/well. On the next day, siPLKA4-13, and pCPA4-12 in which LNP encapsulated with siRNA of anti polo-like kinase gene 1 (siPLK1) and plasmids simultaneously expressing Cas protein and PLK1 sgRNA (pCPLK1), respectively, were added to the corresponding wells, so that the amount of siRNA and plasmids is 1600 ng. The Mock group was not further treated. Cells were cultured for 4 hours at 37°C in 5% $CO_2$. Thereafter, 1 mL of fresh medium was added to each well, and further continuously cultured for 20 hours. When the predetermined time is reached, the relative expression of PLK1 mRNA in the test samples was detected by Quantitative Real-Time PCR. The nucleic acid and primer sequences for PLK1 siRNA are shown in Tables 8 and 9, respectively.

Table 8

| Name | SEQ ID NO. | Nucleotide Sequence (5'→3') |
| --- | --- | --- |
| siPLK1 | 21 | UGAAGAAGAUCACCCUCCUUAdTdT |
| | 22 | UAAGGAGGGUGAUCUUCUUCAdTdT |

Table 9

| Gene name | SEQ ID NO. | Nucleotide Sequence (5'→3') |
|---|---|---|
| Human PLK1 | 23 | GCCCCTCACAGTCCTCAATA |
| | 24 | TACCCAAGGCCGTACTTGTC |

Preparation Example 1 Synthesis of amine-containing lipid compound A4

Synthesis of compound A2

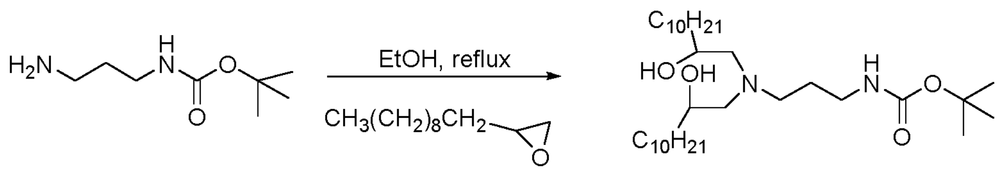


A1 A2

The reaction was carried out in a three-necked flask containing tert-butyl (3-aminopropyl)carbamate (1.01 g, 5.7 mmol), 1, 2-epoxydodecane (2.33 g, 12.6 mmol) and 12.00 mL of ethanol at 50°C under nitrogen reflux. The reaction was monitored by thin layer chromatography (TLC) and stopped after the completion of the reaction. The crude product was concentrated under reduced pressure, separated, and purified by a silica gel column using dichloromethane: methanol=30:1 (v/v) as a developing agent. The product A2 was obtained (1.61 g, 78.1% yield). 1H NMR (400 MHz, CDCl3) δ (ppm) 0.88 (t, J = 13.68 Hz, 6 H), 1.26-1.44 (m, 45 H), 1.67 (m, 2 H), 2.38-2.70 (m, 6 H), 3.18 (m, 2 H), 3.70 (m, 2 H); 13C NMR (100 MHz, CDCl3) δ (ppm) 156.22, 79.39, 69.58, 62.90, 52.70, 38.58, 38.53, 35.13, 34.98, 31.92, 29.62, 29.35, 28.44, 25.65, 22.70, 14.13; high-resolution mass spectrum (EI + mode) scans were performed for compound A2 (MW 543.5023), the scanning result was 543.5108.

Synthesis of compound A3

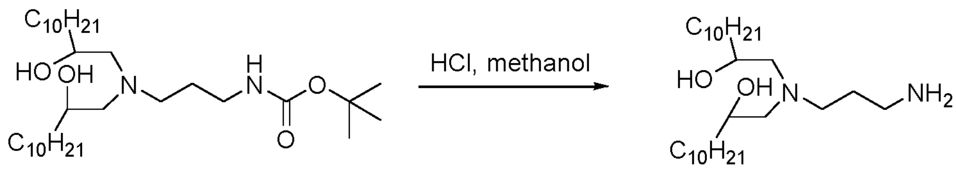


A2 A3

In a three-necked flask containing compound A2 (1.04 g, 1.8 mmol) and 20.00 mL of dichloromethane, hydrochloric acid was slowly added while stirring. The reaction was carried out for 16 hours at room temperature, after the reaction was completed, the reaction was stopped and assayed on a TLC plate. The pH of the reaction mixture was adjusted to 7-8 with 1 M NaOH aqueous solution and extracted three times with water and ethyl acetate. Concentration under reduced pressure gave 398.0 mg of product A3, with a yield of 49.9%. 1H NMR (400 MHz, CDCl3) δ (ppm) 0.88 (t, J = 13.08 Hz, 6 H), 1.26 (m, 32 H), 1.43 (m, 4 H), 1.96 (m, J = 14.5 Hz, 2 H), 2.60 (m, J=20.9 Hz, 4 H), 3.01 (m, 2 H), 3.25 (m, 2 H), 3.80 (m, 2 H); 13C NMR (100 MHz, CDCl3) δ (ppm) 70.41, 63.21, 58.29, 40.79, 36.15, 31.94, 29.91, 29.69, 29.39, 23.90, 22.70, 14.11; high-resolution mass spectrum (EI+ mode) scan were performed for compound A3 (MW 443.4498), the scanning result was 443.4576.

Synthesis of compound A4

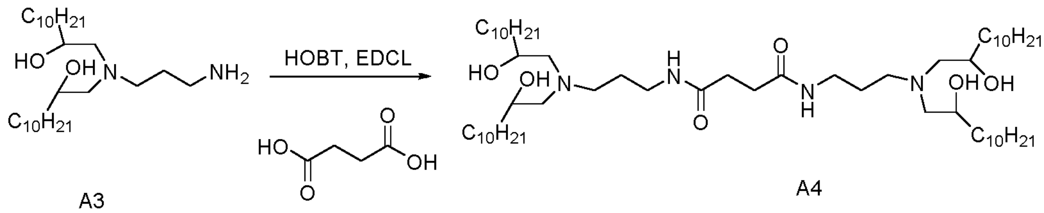


A3

A4

A3 (2.2 g, 4.9 mmol) and succinic acid (263.3 mg, 2.2 mmol) were dissolved in 20.00 mL of dichloromethane and placed in a three-necked flask. The catalyst 1-hydroxybenzotriazole (753.3 mg, 5.6 mmol) and 1-(3-dimethylaminopropyl)-3-ethylcarbodiimide hydrochloride (1.13 g, 5.6 mmol) were then added. Under the protection of nitrogen, the reaction was carried out for 12 hours. The reaction was stopped after detection by thin layer chromatography. The crude product was washed twice with water, twice with saturated brine, and twice with the organic phase. The product A4 was obtained in 0.1 g, with a yield of 3.3%. 1H NMR (400 MHz, CDCl3) δ (ppm) 0.88 (t, J = 13.24 Hz, 12 H), 1.26-1.47 (m, 72 H), 1.93 (m, 4 H), 2.56-3.41.74 (m, 20 H), 3.89 (m, 4 H), 5.03 (m, 4 H), 8.04(m, 2 H); 13C NMR (100 MHz, CDCl3) δ (ppm) 173.26, 67.14, 59.69, 51.69, 35.70, 34.47, 34.21, 33.21, 30.95, 30.52, 29.30, 28.72, 28.42, 21.70, 13.11; High-resolution mass spectrometry (EI+ mode) scan was performed for the compound (MW 967.9051), the scanning result was 967.9137.

Example 1

Lipid A4 obtained in Preparation Example 1 was dissolved in absolute ethanol at a concentration of 20 mg/ml together with DSPC, cholesterol, DMG-PEG (purchased from AVT (Shanghai) Pharmaceutical Technology Co. Ltd.). A4 can be completely dissolved at room temperature and it is a light yellow transparent liquid. DSPC, cholesterol, and DMG-PEG need a water bath at 50°C for several minutes until completely dissolved, all of which are colorless and transparent liquids. After preparation of the above materials, the four raw materials were mixed in a mass ratio of A4: DSPC: cholesterol: DMG PEG=66.8: 10.9: 20.0: 2.2 and all of the mixture was sucked into an insulin syringe for later use. In addition, 10 nM citric acid/sodium citrate buffer solution with three times volume of the organic phase mixture solution, pH = 4.0, were also prepared, which was also sucked into an insulin syringe. Thereafter, the insulin syringes containing the organic phase mixed solution and the buffer solution respectively were placed in the same centrifuge tube and all the liquid was quickly released. The centrifuge tube was gently shaken to prepare lipid nanoparticles A4-1.

Test Example 1

This test example was aimed to detect the ability of the lipid nanoparticles obtained in Example 1 to encapsulate siRNA through a gel retardation test while exploring the optimal mass ratio of lipid nanoparticles to siRNA. The test result is shown in FIG. 1. The results show that when the mass ratio of LNP to siRNA was less than 5: 1, siRNA still had a large amount of leakage, indicating that the low mass ratio is not suitable for delivery of siRNA. When the mass ratio is greater than or equal to 5: 1, siRNA was efficiently encapsulated by LNP, and when the mass ratio was 15: 1, siRNA was almost completely encapsulated. It is indicated that when the mass ratio of LNP to siRNA is 5: 1- 30: 1, it is a suitable range of the mass ratio of LNP to siRNA, and the mass ratio of LNP to siRNA is greater than or equal to 15: 1, it is possible to achieve high-efficiency delivery of siRNA. Therefore, in subsequent preparation examples, this mass ratio was selected to prepare LNPs.

Example 2

The aforementioned four dissolved raw materials were mixed in a mass ratio of A4: DSPC: cholesterol: DMG-PEG = 58.5: 14.3: 23.4: 3.8 and the mixture was sucked into an insulin syringe. Another 3-fold volume of citric acid/sodium citrate buffer solution with three times volume sucked into an insulin syringe, mixed with the organic phase. The mixture was gently shaken to prepare lipid nanoparticles A4-2.

Example 3

The aforementioned four dissolved raw materials were mixed in a mass ratio of A4: DSPC: cholesterol: DMG-PEG = 50.2: 16.4: 25.1: 8.2 and the mixture was sucked into an insulin syringe. Another citric acid/sodium citrate buffer solution with three times volume was sucked into an insulin syringe, mixed with the organic phase, and gently shaken to prepare lipid nanoparticles A4-3.

Example 4

The aforementioned four dissolved raw materials were mixed in a mass ratio of A4: DSPC: cholesterol: DMG-PEG = 42.8: 17.5: 25.7: 14.0 and the mixture was sucked into an insulin syringe. Another citric acid/sodium citrate buffer with three times volume was sucked into an insulin syringe, mixed with the organic phase. The mixture was gently shaken to prepare lipid nanoparticles A4-4.

Example 5

The aforementioned four dissolved raw materials were mixed in a mass ratio of A4: DSPC: cholesterol: DMG-PEG = 61.8: 8.6: 21.0: 8.6 and the mixture was sucked into an insulin syringe. Another citric acid/sodium citrate buffer solution with three times volume was sucked into an insulin syringe, mixed with the organic phase, and gently shaken to prepare lipid nanoparticles A4-5.

Example 6

The aforementioned four dissolved raw materials were mixed in a mass ratio of A4: DSPC: cholesterol: DMG-PEG = 57.4: 12.0: 14.6: 16.0 and the mixture was sucked into an insulin syringe. Another citric acid/sodium citrate buffer solution with three times volume was sucked into an insulin syringe, mixed with the organic phase. The mixture was gently shaken to prepare lipid nanoparticles A4-6.

Example 7

The aforementioned four dissolved raw materials were mixed in a mass ratio of A4: DSPC: cholesterol: DMG-PEG = 55.0: 15.3: 28.1: 1.5 and the mixture was sucked into an insulin syringe. Another citric acid/sodium citrate buffer solution with three times volume was sucked into an insulin syringe, mixed with the organic phase, and gently shaken to prepare lipid nanoparticles A4-7.

Example 8

The aforementioned four dissolved raw materials were mixed in a mass ratio of A4: DSPC: cholesterol: DMG-PEG = 54.7: 19.0: 23.3: 3.1 and the mixture was sucked into an insulin syringe. Another citric acid/sodium citrate buffer solution with three times volume was sucked into an insulin syringe, mixed with the organic phase. The mixture was gently shaken to prepare lipid nanoparticles A4-8.

Example 9

The aforementioned four dissolved raw materials were mixed in a mass ratio of A4: DSPC: cholesterol: DMG-PEG = 57.7: 7.0: 21.3: 14.0 and the mixture was sucked into an insulin syringe. Another citric acid/sodium citrate buffer solution with three times volume was sucked into an insulin syringe, mixed with the organic phase, and gently shaken to prepare lipid nanoparticles A4-9.

Example 10

The aforementioned four dissolved raw materials were mixed in a mass ratio of A4: DSPC: cholesterol: DMG-PEG = 57.2: 10.4: 25.4: 7.0 and the mixture was sucked into an insulin syringe. Another citric acid/sodium citrate buffer solution with three times volume was sucked into an insulin syringe, mixed with the organic phase. The mixture was gently shaken to prepare lipid nanoparticles A4-10.

Example 11

The aforementioned four dissolved raw materials were mixed in a mass ratio of A4: DSPC: cholesterol: DMG-PEG = 66.1: 16.0: 14.7: 3.2 and the mixture was sucked into an insulin syringe. Another citric acid/sodium citrate buffer solution with three times volume sucked into an insulin syringe, mixed with the organic phase, and gently shaken to prepare lipid nanoparticles A4-11.

Example 12

The aforementioned four dissolved raw materials were mixed in a mass ratio of A4: DSPC: cholesterol: DMG-PEG = 61.6: 18.6: 18.2: 1.5 and the mixture was sucked into an insulin syringe. Another citric acid/sodium citrate buffer solution with three times volume sucked into an insulin syringe, mixed with the organic phase. The mixture was gently shaken to prepare lipid nanoparticles A4-12.

Example 13

The aforementioned four dissolved raw materials were mixed in a mass ratio of A4: DSPC: cholesterol: DMG-PEG = 64.8: 6.9: 25.5: 2.8 and the mixture was sucked into an insulin syringe. Another citric acid/sodium citrate buffer solution with three times volume sucked into an insulin syringe, mixed with the organic phase, and gently shaken to prepare lipid nanoparticles A4-13.

Example 14

The aforementioned four dissolved raw materials were mixed in a mass ratio of A4: DSPC: cholesterol: DMG-PEG = 66.2: 10.6: 21.7: 1.4 and the mixture was sucked into an insulin syringe. Another citric acid/sodium citrate buffer solution with three times volume sucked into an insulin syringe, mixed with the organic phase. The mixture was gently shaken to prepare lipid nanoparticles A4-14.

Example 15

The aforementioned four dissolved raw materials were mixed in a mass ratio of A4: DSPC: cholesterol: DMG-PEG = 59.1: 12.7: 15.5: 12.7 and the mixture was sucked into an insulin syringe. Another citric acid/sodium citrate buffer solution with three times volume sucked into an insulin syringe, mixed with the organic phase, and gently shaken to prepare lipid nanoparticles A4-15.

Example 16

The aforementioned four dissolved raw materials were mixed in a mass ratio of A4: DSPC: cholesterol: DMG-PEG = 63.6: 17.1: 21.5: 6.8 and the mixture was sucked into an insulin syringe. Another citric acid/sodium citrate buffer solution with three times volume sucked into an insulin syringe, mixed with the organic phase. The mixture was gently shaken to prepare lipid nanoparticles A4-16.

In the above examples, A4, DSPC, cholesterol and DMG-PEG were set at 4 levels, respectively, as shown in Table 8:

Table 8:

| A4 | DSPC | Cholesterol | DMG-PE G |
|---|---|---|---|
| 40 | 8 | 30 | 0.5 |
| 47 | 12 | 37 | 1 |
| 54 | 16 | 44 | 2.5 |
| 61 | 20 | 51 | 5 |

The proportions of Examples 1-16 were designed using orthogonal design software (orthogonal design assistant ii V3.1), and the molar ratios and mass ratios of 16 LNP with different proportions are shown in Table 9:

Table 9:

| No. | Molar ratio | | | | Mass ratio | | | |
|---|---|---|---|---|---|---|---|---|
| | A4 | DSPC | Cholesterol | DMG-PEG | A4 | DSPC | Cholesterol | DMG-PEG |
| A4-1 | 40 | 8 | 30 | 0.5 | 66.8% | 10.9% | 20.0% | 2.2% |
| A4-2 | 40 | 12 | 40 | 1 | 58.5% | 14.3% | 23.4% | 3.8% |
| A4-3 | 40 | 16 | 50 | 2.5 | 50.2% | 16.4% | 25.1% | 8.2% |

(continued)

| No. | Molar ratio | | | | Mass ratio | | | |
|---|---|---|---|---|---|---|---|---|
| | A4 | DSPC | Cholesterol | DMG-PEG | A4 | DSPC | Cholesterol | DMG-PEG |
| A4-4 | 40 | 20 | 60 | 5 | 42.8% | 17.5% | 25.7% | 14.0% |
| A4-5 | 47 | 8 | 40 | 2.5 | 61.8% | 8.6% | 21.0% | 8.6% |
| A4-6 | 47 | 12 | 30 | 5 | 57.4% | 12.0% | 14.6% | 16.0% |
| A4-7 | 47 | 16 | 60 | 0.5 | 55.0% | 15.3% | 28.1% | 1.5% |
| A4-8 | 47 | 20 | 50 | 1 | 54.7% | 19.0% | 23.3% | 3.1% |
| A4-9 | 54 | 8 | 50 | 5 | 57.7% | 7.0% | 21.3% | 14.0% |
| A4-10 | 54 | 12 | 60 | 2.5 | 57.2% | 10.4% | 25.4% | 7.0% |
| A4-11 | 54 | 16 | 30 | 1 | 66.1% | 16.0% | 14.7% | 3.2% |
| A4-12 | 54 | 20 | 40 | 0.5 | 61.6% | 18.6% | 18.2% | 1.5% |
| A4-13 | 61 | 8 | 60 | 1 | 64.8% | 6.9% | 25.5% | 2.8% |
| A4-14 | 61 | 12 | 50 | 0.5 | 66.2% | 10.6% | 21.7% | 1.4% |
| A4-15 | 61 | 16 | 40 | 5 | 59.1% | 12.7% | 15.5% | 12.7% |
| A4-16 | 61 | 20 | 30 | 2.5 | 63.6% | 17.0% | 12.5% | 6.8% |

Examples 17-32

The concentration of siApoB was adjusted to 1000 ng/μL and mixed with an equal volume of 50% ethanol solution to prepare a 500 ng/μL siApoB solution with an ethanol content of 25%.

The 16 lipid nanoparticles with different ratios obtained in Examples 1-16 were mixed with the siApoB solution at a mass ratio of 15: 1 (i.e. a volume ratio of 1.5: 1), gently shaken and then incubated at 50°C for 20 minutes, so that the siApoB was encapsulated with the lipid nanoparticles as much as possible. Thereafter, 16 the lipid nanoparticle/siApoB complexes were transferred into a dialysis tube with a molecular weight cut-off of 100000 Dalton (spectrum, G235035) and dialyzed in 1×PBS for 3 hours.

Test Example 2

This test example was aimed to detect the particle size and polydispersity index (PDI) of the 16 lipid nanoparticles unencapsulated with siApoB obtained in Examples 1-16. As shown in Table 10, most of the formulations satisfied the precondition as an excellent delivery carrier.

Table 10:

| No. | Size (nm) | PDI |
|---|---|---|
| A4-1 | 106.75±0.21 | 0.173±0.057 |
| A4-2 | 146.90±0.99 | 0.547±0.374 |
| A4-3 | 120.45±0.21 | 0.112±0.002 |
| A4-4 | 171.45±1.63 | 0.179±0.033 |
| A4-5 | 191.70±0.14 | 0.141±0.009 |
| A4-6 | 203.85±3.18 | 0.254±0.013 |
| A4-7 | 201.95±0.49 | 0.263±0.018 |
| A4-8 | 200.70±0.99 | 0.274±0.010 |
| A4-9 | 155.80±0.28 | 0.268±0.006 |
| A4-10 | 187.20±0.71 | 0.248±0.001 |

(continued)

| No. | Size (nm) | PDI |
|---|---|---|
| A4-11 | 129.90±0.71 | 0.142±0.013 |
| A4-12 | 106.34±0.49 | 0.124±0.028 |
| A4-13 | 97.16±0.37 | 0.133±0.008 |
| A4-14 | 92.25±0.32 | 0.130±0.007 |
| A4-15 | 137.10±0.99 | 0.143±0.004 |
| A4-16 | 120.50±0.42 | 0.278±0.011 |

Test Example 3

This test example was aimed to detect the particle size (size), polydispersity index (PDI), surface potential (zeta), encapsulation efficiency (E. E.), and intragranular siRNA concentration (conc) of the 16 formulations encapsulated with siApoB obtained in Examples 17-32. As shown in Table 11, all formulations were highly effective in encapsulating siRNA.

Table 11:

| No. | Size (nm) | PDI | E. E. (%) | Conc. (ng/μL) |
|---|---|---|---|---|
| siA4-1 | 129.90 ± 0.71 | 0.124 ± 0.028 | 91% | 115.8 |
| siA4-2 | 106.35 ± 0.49 | 0.133 ± 0.008 | 92% | 115.4 |
| siA4-3 | 97.16 ± 0.37 | 0.130 ± 0.007 | 91% | 105.0 |
| siA4-4 | 92.25±0.32 | 0.143±0.004 | 89% | 90.5 |
| siA4-5 | 137.10±0.99 | 0.278±0.011 | 90% | 92.9 |
| siA4-6 | 120.50±0.42 | 0.136±0.005 | 86% | 106.5 |
| siA4-7 | 128.30±0.14 | 0.081±0.016 | 90% | 86.1 |
| siA4-8 | 187.30±1.41 | 0.133±0.001 | 87% | 66.1 |
| siA4-9 | 104.10±0.99 | 0.127±0.010 | 92% | 130.6 |
| siA4-10 | 106.35±0.92 | 0.132±0.004 | 91% | 117.3 |
| siA4-11 | 139.90±1.41 | 0.135±0.008 | 91% | 132.6 |
| siA4-12 | 202.55±1.91 | 0.026±0.022 | 89% | 104.0 |
| siA4-13 | 110.00±1.13 | 0.125±0.010 | 94% | 154.5 |
| siA4-14 | 138.30±0.28 | 0.061±0.005 | 94% | 167.4 |
| siA4-15 | 113.55±0.35 | 0.145±0.008 | 91% | 132.4 |
| siA4-16 | 123.65±1.06 | 0.171±0.018 | 92% | 127.1 |

Test Example 4

The 16 formulations obtained in Examples 17-32 were subjected to in vitro transfection experiments. The quantitative real-time PCR results are shown in Table 12. All formulations were able to deliver the encapsulated siRNA into cells and achieve gene inhibition, with siA4-13 having the best performance.

Table 12:

| No. | activity | No. | activity |
|---|---|---|---|
| siA4-1 | 0.413±0.171 | siA4-9 | 0.605±0.167 |

(continued)

| No. | activity | No. | activity |
|---|---|---|---|
| siA4-2 | 0.802±0.059 | siA4-10 | 0.622±0.062 |
| siA4-3 | 0.820±0.033 | siA4-11 | 0.488±0.067 |
| siA4-4 | 1.396±0.521 | siA4-12 | 0.761±0.032 |
| siA4-5 | 0.582±0.028 | siA4-13 | 0.389±0.046 |
| siA4-6 | 0.679±0.267 | siA4-14 | 0.376±0.083 |
| siA4-7 | 0.619±0.099 | siA4-15 | 0.624±0.149 |
| siA4-8 | 0.769±0.303 | siA4-16 | 0.543±0.291 |

Test Example 5

The 16 formulations obtained in Examples 17-32 were validated for activity at the animal level. C57BL/6 was taken as a subject in the test. 68 female mice were divided into 17 groups and injected via the tail vein with 1×PBS or 16 formulations at a dose of 0.5 mg/kg, respectively. At 42 hours after dosing, animals were fasted for 6 hours and euthanized. Liver tissue samples were collected for analysis of changes in ApoB mRNA expression relative to the animals receiving 1×PBS. Apob mRNA expression was detected by quantitative real-time PCR. The analytical results of the activity tests were generated by Graphpad Prism 8 and are shown in Table 13. All formulations achieved gene inhibition at animal level, with siA4-13 having the best performance.

Table 13:

| No. | activity | No. | activity |
|---|---|---|---|
| siA4-1 | 0.263 ± 0.112 | siA4-9 | 0.628 ± 0.200 |
| siA4-2 | 0.500 ± 0.198 | siA4-10 | 0.295 ± 0.086 |
| siA4-3 | 0.540 ± 0.196 | siA4-11 | 0.228 ± 0.068 |
| siA4-4 | 0.850 ± 0.213 | siA4-12 | 0.433 ± 0.101 |
| siA4-5 | 0.364 ± 0.186 | siA4-13 | 0.133 ± 0.057 |
| siA4-6 | 0.690 ± 0.421 | siA4-14 | 0.213 ± 0.094 |
| siA4-7 | 0.531 ± 0.320 | siA4-15 | 0.509 ± 0.133 |
| siA4-8 | 0.840 ± 0.315 | siA4-16 | 0.424 ± 0.154 |

Example 33

Sifirefly was treated with DEPC to adjust the concentration to 1000 ng/μL and mixed with an equal volume of 50% ethanol solution to prepare a 500 ng/μL siFirefly solution with 25% ethanol concentration.

The lipid nanoparticles numbered A4-13 obtained in Example 13 were mixed with the siFirefly solution at a mass ratio of 15: 1 (i.e. a volume ratio of 1.5: 1), gently shaken, and then incubated at 50°C for 20 minutes so that the siFirefly was encapsulated with the lipid nanoparticles as much as possible. Thereafter, the complex was transferred to a dialysis tube (spectrum, G235035) with a molecular weight cut-off of 100000 Daltons and dialyzed in 1×PBS for 3 hours to obtain siFLA4-13.

Test Example 6

This test example was aimed to detect the cytotoxicity of formulation No. siFLA4-13 obtained in Example 33. The test results are shown in Table 14:

Table 14:

| Content | Relative viability | Content | Relative viability |
|---|---|---|---|
| 1 nM | 0.98 ± 0.06 | 50 nM | 0.96 ± 0.02 |
| 5 nM | 0.99 ± 0.02 | 100 nM | 0.97 ± 0.06 |
| 10 nM | 0.98 ± 0.05 | 200 nM | 0.94 ± 0.04 |

Test Example 7

This test example was aimed to test the stability of formulation No. siA4-13 obtained in example 29 at 4°C. Specifically, the particle size of the formulation was measured using a laser granulometer after the formulation was prepared. The formulation was stored at 4°C after the completion of operation. Thereafter, the particle size of the formulation was measured again on days 3, 7, and 21, respectively, and the measurement results are shown in FIG. 2, demonstrating that the formulation can be kept stable at 4°C for at least 3 weeks.

Example 34

Cy5-siFirefly was treated with DEPC to adjust the concentration to 1000 ng/μL and mixed with an equal volume of 50% ethanol solution to prepare a 500 ng/μL siFirefly solution with 25% ethanol concentration.

The lipid nanoparticles No. A4-13 obtained in Example 13 were mixed with the Cy5-siFirefly solution at a mass ratio of 15: 1 (i.e. a volume ratio of 1.5: 1), gently shaken, and then incubated at 50°C for 20 minutes so that the Cy5-siFirefly was encapsulated with the lipid nanoparticles as much as possible. Thereafter, the complex was transferred to a dialysis tube(spectrum, G235035) with a molecular weight cut-off of 100000 Daltons and dialyzed in 1×PBS for 3 hours to obtain Cy-siFLA4-13, the above process must be protected from light throughout.

Test Example 8

This test example was aimed to detect the metabolic profile of Cy-siFLA4-13 obtained in Example 34 in C57BL/6 mice.

Test Example 9

This test example was aimed to test the dose-dependent behavior of formulation No. siA4-13 obtained in Example 29 in C57BL/6 mice. The changes in ApoB mRNA expression and levels of total cholesterol (CHO), triglyceride (TG), low-density lipoprotein cholesterol (LDL-c), and high-density lipoprotein cholesterol (HDL-c) are shown in Table 16 and Table 17, respectively. According to the calculation, the half effective dose of this formulation is about 0.18 mg/kg.

Table 16:

| Dose | mRNA relative expression |
|---|---|
| 1 mg/kg | 0.19 ± 0.08 |
| 0.5 mg/kg | 0.27 ± 0.06 |
| 0.25 mg/kg | 0.38 ± 0.15 |
| 0.1 mg/kg | 0.56 ± 0.23 |
| 0.05 mg/kg | 0.94 ± 0.29 |
| 0.025 mg/kg | 0.92 ± 0.23 |
| 0.01 mg/kg | 0.84 ± 0.24 |

Table 17:

| Dose | TG | CHO | HDL-c | LDL-c |
|---|---|---|---|---|
| PBS | 1.68 ± 0.14 | 2.79 ± 0.19 | 2.30 ± 0.12 | 0.68 ± 0.05 |

(continued)

| Dose | TG | CHO | HDL-c | LDL-c |
|---|---|---|---|---|
| 1 mg/kg | 0.86±0.21 | 1.01 ± 0.26 | 0.89 ± 0.19 | 0.17 ± 0.05 |
| 0.5 mg/kg | 1.06 ± 0.24 | 1.33 ± 0.14 | 1.23 ± 0.11 | 0.20 ± 0.05 |
| 0.25 mg/kg | 1.11 ± 0.21 | 1.73 ± 0.25 | 1.58 ± 0.19 | 0.31 ± 0.09 |
| 0.1 mg/kg | 1.20 ± 0.26 | 2.09 ± 0.53 | 1.81 ± 0.46 | 0.42 ± 0.10 |
| 0.05 mg/kg | 1.17 ± 0.28 | 2.06 ± 0.63 | 1.69 ± 0.56 | 0.58 ± 0.14 |
| 0.025 mg/kg | 1.21 ± 0.16 | 2.62 ± 0.20 | 2.19 ± 0.11 | 0.75 ± 0.10 |
| 0.01 mg/kg | 1.47 ± 0.19 | 2.57 ± 0.16 | 2.09 ± 0.14 | 0.64 ± 0.08 |

Test Example 10

This test example was aimed to detect changes in ApoB mRNA expression and changes in CHO, TG, LDL-c, and HDL-c levels at different time points after C57BL/6 mice received a single injection of the siA4-13 formulation obtained in Example 29. The results are shown in FIG. 4. After 4 weeks of treatment, ApoB mRNA, TG, and LDL-c levels were still lower than those in the control group, which supports dosing once every 4 weeks.

Example 35

The formulation obtained in Example 33 was concentrated with an ultrafiltration tube having a pore size of 100Kd, and the concentration of siRNA in the formulation was adjusted to 300 ng/μL (corresponding to a dosing concentration of 3 mg/kg) and 100 ng/μL (corresponding to a dosing concentration of 1 mg/kg), respectively, after determining the concentration of siRNA by the RiboGreen method.

Test Example 11

This test example was aimed to detect the change of major serum biochemical indicators after receiving the formulation obtained in Example 34 in C57BL/6 mice, and thus the safety of the formulation was determined. The results are shown in FIG. 5. The test experiment showed that the toxicity of the formulation can meet the requirements.

Example 36

The concentration of siANGPTL3 was adjusted to 1000 ng/μL with DEPC-treated water and mixed with an equal volume of 50% ethanol solution to prepare a 500 ng/μL siANGPTL3 solution with 25% ethanol concentration.

The lipid nanoparticles numbered A4-13 obtained in Example 13 were mixed with the siANGPTL3 solution at a mass ratio of 15: 1 (i.e. a volume ratio of 1.5: 1), gently shaken and then incubated at 50°C for 20 minutes so that the siANGPTL3 was encapsulated with the lipid nanoparticles as much as possible. Thereafter, the complex was transferred to a dialysis tube (spectrum, G235035) with a molecular weight cut-off of 100000 Daltons and dialyzed in 1×PBS for 3 hours to yield siANA4-13.

Test Example 12

This test example was aimed to detect the hypolipidemic effect and ANGPTL3 mRNA expression inhibition ability of siANA4-13 obtained in Example 35 in db/db mice. The changes in TG and CHO in the treated mice are shown in FIG. 6. This experiment demonstrates that the blood lipids in model animals can be significantly reduced to the level of normal animals after receiving the treatment.

Target gene expression comparison between the mice treated with 0.5 mg/kg and 0.25 mg/kg siANGPT3 and the mice treated with 0.5 mg/kg siNC is shown in Table 18. The decrease in blood lipids in FIG. 6 is caused by inhibition of the target gene.

Table 18:

| Group | MRNA expression change |
|---|---|
| 0.5mg/kg siANG | 0.03 ± 0.02 |
| 0.25mg/kg siANG | 0.15 ± 0.06 |

Lipid deposition in the livers of animals in different treatment groups is shown in FIG. 7, which shows that the formulation has the ability to reverse lipid deposition in the liver.

Example 37

The concentration of siApoC3 was adjusted to 1000 ng/μL with DEPC-treated water and mixed with an equal volume of 50% ethanol solution to prepare a 500 ng/μL siANGPTL3 solution with 25% ethanol concentration.

The lipid nanoparticles No. A4-13 obtained in Example 13 were mixed with the siApoC3 solution at a mass ratio of 15: 1 (i.e. a volume ratio of 1.5: 1), gently shaken, and then incubated at 50°C for 20 minutes so that the siApoC3 was encapsulated with the lipid nanoparticles as much as possible. Thereafter, the complex was transferred to a dialysis tube (spectrum, G235035) with a molecular weight cut-off of 100000 Daltons and dialyzed in 1×PBS for 3 hours to yield siApoCA4-13.

Test Example 13

This test example was aimed to detect the hypolipidemic effects and changes in blood lipid of hApoC3 mice receiving siApoCA4-13 treatment obtained in Example 34 and within 4 weeks of withdrawal. The changes of TG and CHO in the treated mice are shown in FIG. 8. This test shows that the blood lipids of the model animals were significantly decreased after treatment. After the withdrawal, it took a period of time for the blood lipid to return to the pre-treatment level.

Preparation Example 2 Synthesis of amine-containing lipid compound A10

Synthesis of compound A7

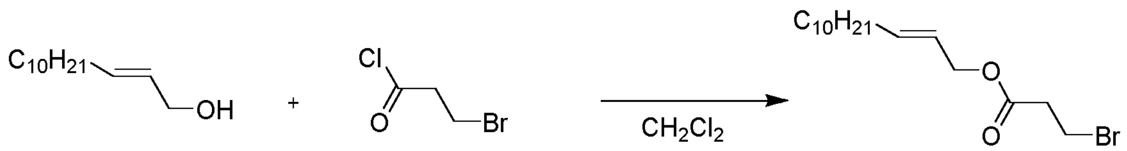


A5 A6 A7

20 mL of anhydrous dichloromethane was added under the protection of Ar atmosphere, and 2 g of trans-2-tridecen-1-ol and 1.71 g of 3-bromopropionyl chloride were added under stirring. The reaction was stirred at room temperature for 2h. After the reaction was completed as determined by TLC, the reaction solution was concentrated under reduced pressure to remove dichloromethane and purified by a silica gel column to give a 2.7 g of pale yellow oil, with a yield of 89%. 1H NMR(400MHz, CDCl3) δ: 5.75-5.86(1, m, CH = CH), 5.52-5.65(1, m, CH = CH), 4.56-4.61(2H, d $OCH_2$), 3.56-3.62(2H, t $CH_2$), 2.89-2.97(2H, t $CH_2$), 2.16-2.97(2H, m $CH_2$), 1.22-1.45(16H, m), 0.90(3H, t, $CH_3$)ppm.

Synthesis of compound A8

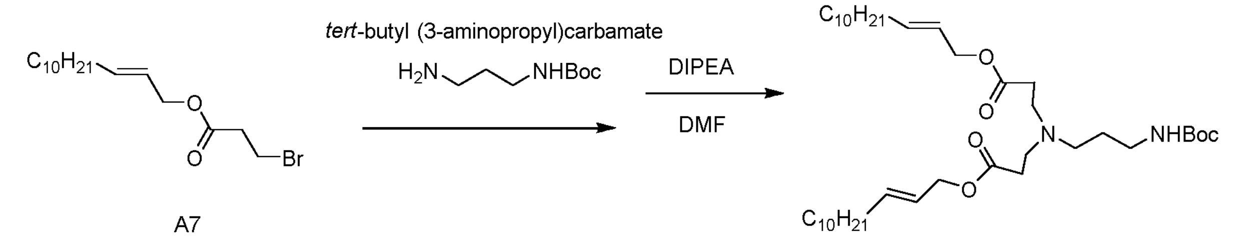


A7 A8

Under the protection of Ar gas, 10 ml of anhydrous DMF was added into the reaction flask, and 280 mg of N-Boc-1,3-diaminopropane hydrochloride, and 721.4 mg of DIPEA were added under stirring. After stirring at room temperature for 2 h, 1 g of compound A7 was added dropwise and stirred at room temperature overnight. After the completion of the reaction, the reaction solution was concentrated under increased pressure to remove DMF and chromatographed on silica gel to give 163 mg of pale yellow oily compound A8, with a yield of 18%.

1H NMR(400MHz, CDCl3) δ: 5.76-5.83(2H, m, CH = CH), 5.54-5.62(2, m, CH=CH), 4.53-4.57(4H, d $OCH_2$), 2.71-2.78(4H, t $CH_2$), 2.43-2.52(6H, m $CH_2$), 2.04-2.18(4H, m $CH_2$), 1.60-1.68(2H, m), 1.43-1.50(12H, m), 1.22-1.36(32H, m), 0.84-0.90(6H, m, $CH_3$) ppm.

Synthesis of compound A9

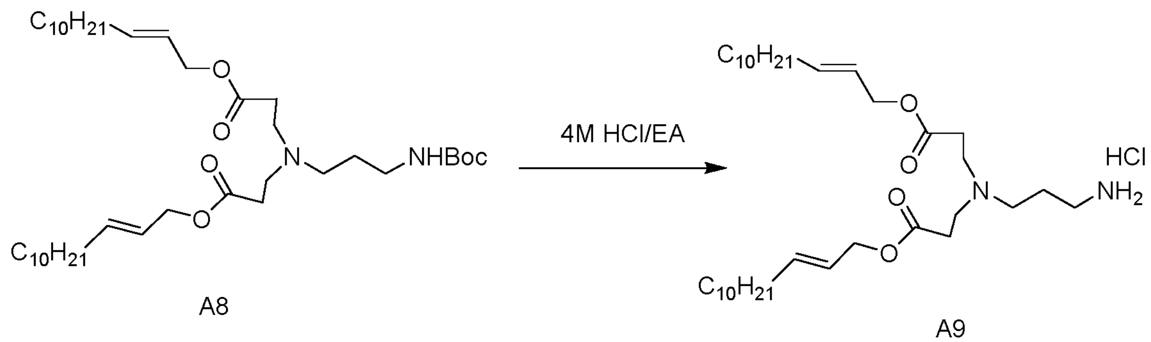


A8 A9

163 mg of compound A8 was added to 5 ml of 4M HCl ethyl acetate solution at room temperature and the reaction was stirred at room temperature. The reaction was detected by TLC after 1h. There was no starting material, indicating the reaction was complete. The solvent was removed by concentration under reduced pressure and concentrated with a vacuum pump for 5 mins and used directly in the next reaction.

Synthesis of compound A10

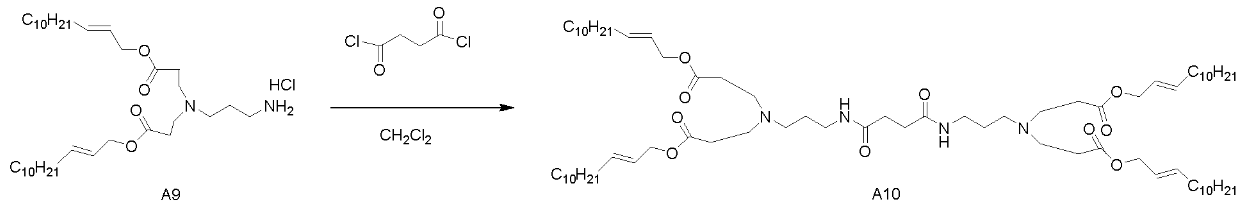


A9 A10

Under the Ar atmosphere, the product of the above step was dissolved in 2 ml of anhydrous dichloromethane. 45 mg of triethylamine was added, and 14 mg of succinyl chloride was added dropwise after stirring at room temperature for 30 mins. The reaction was stirred at room temperature for 1 h and detected by TLC. The reaction solution was diluted with 10 ml of dichloromethane and washed with 1M hydrochloric acid. The organic phase was collected, concentrated under reduced pressure, and passed through a silica gel column. The mobile phase was DCM: MeOH = 60: plus 1. 5‰ ammonia water (ammonia-free product was put on the column) to give a 56 mg of pale yellow oily product A10, the two-step yield was 37%.

1H NMR(400MHz, CDCl3)δ: 6.85 (2H, s, NH) ,5.77-5.83(4H, m, CH = CH), 5.52-5.61(4, m, CH = CH), 4.51-4.55(8H, d $OCH_2$), 3.20-3.26(4H, m), 2.71-2.77(8H, t $CH_2$), 2.56 < 4H, s), 2.45-2.51(12H, m $CH_2$), 2.07-2.15(8H, m $CH_2$), 1.61-1.68(4H m,),1.21-1.33(64H, m), 0.85-0.91(12H, m, $CH_3$)ppm.

Mass calc. 1239.88, found M + 1 = 1239.9.

Example 38

The lipid A10 obtained in Preparation Example was dissolved in absolute ethanol at a concentration of 20 mg/ml together with DSPC, cholesterol, DMG-PEG (purchased from AVT (Shanghai) Pharmaceutical Technology Co. Ltd.). A10 can be completely dissolved at room temperature and it is a light yellow transparent liquid. DSPC, cholesterol, and DMG-PEG need a water bath at 50°C for several minutes until completely dissolved, all of which are colorless and transparent liquids. After preparation of the above materials, the four raw materials were mixed in a mass ratio of A10: DSPC: cholesterol: DMG PEG=72.2:8.1:14.9:4.9 were mixed and all of the mixture was sucked into an insulin syringe for later use. In addition, 10 nM citric acid/sodium citrate buffer solution with three times volume of the organic phase mixture solution, pH = 4.0, were also prepared, which was also sucked into an insulin syringe. Thereafter, the insulin syringes containing the organic phase mixed solution and the buffer solution respectively were placed in the same centrifuge tube and all the liquid was quickly released. The centrifuge tube was gently shaken to prepare LNP A10-1.

Example 39

The aforementioned four dissolved raw materials were mixed in a mass ratio of A10: DSPC: cholesterol: DMG-PEG = 61.8: 12.1: 19.1: 7.0 and the mixture was sucked into an insulin syringe. Another citric acid/sodium citrate buffer solution with three times volume was sucked into an insulin syringe, mixed with the organic phase, and gently shaken to prepare LNP A10-2.

Example 40

The aforementioned four dissolved raw materials were mixed in a mass ratio of A10: DSPC: cholesterol: DMG-PEG = 52.1: 14.6: 21.5: 11.5 and the mixture was sucked into an insulin syringe. Another citric acid/sodium citrate buffer solution with three times volume was sucked into an insulin syringe, mixed with the organic phase, and gently shaken to prepare LNP A10-3.

Example 41

The aforementioned four dissolved raw materials were mixed i n a mass ratio of A10: DSPC: cholesterol: DMG-PEG = 65.9: 5.9: 16.3: 11.9 and the mixture was sucked into an insulin syringe. Another citric acid/sodium citrate buffer solution with three times volume was sucked into an insulin syringe, mixed with the organic phase, and gently shaken to prepare LNP A10-4.

Example 42

The aforementioned four dissolved raw materials were mixed in a mass ratio of A10: DSPC: cholesterol: DMG-PEG = 64.9: 10.2: 21.4: 3.5 and the mixture was sucked into an insulin syringe. Another citric acid/sodium citrate buffer solution with three times volume was sucked into an insulin syringe, mixed with the organic phase, and gently shaken to prepare LNP A10-5.

Example 43

The aforementioned four dissolved raw materials were mixed in a mass ratio of A10: DSPC: cholesterol: DMG-PEG = 69.3: 15.6: 11.4: 3.7 and the mixture was sucked into an insulin syringe. Another citric acid/sodium citrate buffer solution with three times volume was sucked into an insulin syringe, mixed with the organic phase, and gently shaken to prepare LNP A10-6.

Example 44

The aforementioned four dissolved raw materials were mixed in a mass ratio of A10: DSPC: cholesterol: DMG-PEG = 70.2: 5.3: 19.3: 5.3 and the mixture was sucked into an insulin syringe. Another citric acid/sodium citrate buffer

solution with three times volume was sucked into an insulin syringe, mixed with the organic phase, and gently shaken to prepare LNPA10-7.

Example 45

The aforementioned four dissolved raw materials were mixed in a mass ratio of A10: DSPC: cholesterol: DMG-PEG = 70.5: 9.2: 9.7: 10.6 and the mixture was sucked into an insulin syringe. Another citric acid/sodium citrate buffer solution with three times volume was sucked into an insulin syringe, mixed with the organic phase, and gently shaken to prepare LNPA10-8.

Example 46

The aforementioned four dissolved raw materials were mixed in a mass ratio of A10: DSPC: cholesterol: DMG-PEG = 69.5: 13.0: 14.3: 3.1 and the mixture was sucked into an insulin syringe. Another citric acid/sodium citrate buffer solution with three times volume was sucked into an insulin syringe, mixed with the organic phase, and gently shaken to prepare LNP A10-9.

In the above examples, A10, DSPC, cholesterol, and DMG-PEG were set at 3 levels, respectively, as shown in Table 19:

Table 19

| A10 | DSPC | Choleste rol | DMG-PEG |
|---|---|---|---|
| 40 | 8 | 30 | 1.5 |
| 50 | 14 | 45 | 2.5 |
| 60 | 20 | 60 | 5 |

The proportions of Examples 38-46 were designed using orthogonal design software (orthogonal design assistant ii V3.1), and the molar ratios and mass ratios of 9 LNP with different proportions are shown in Table 20:

Table 20

| No. | Molar ratio | | | | Mass ratio | | | |
|---|---|---|---|---|---|---|---|---|
| | A10 | DSPC | Cholesterol | DMG-PEG | A10 | DSPC | Cholesterol | DMG-PEG |
| A10-1 | 40 | 8 | 30 | 1.5 | 72.2% | 8.1% | 14.9% | 4.9% |
| A10-2 | 50 | 14 | 45 | 2.5 | 61.8% | 12.1% | 19.1% | 7.0% |
| A10-3 | 60 | 20 | 60 | 5 | 52.1% | 14.6% | 21.5% | 11.7% |
| A10-4 | 50 | 8 | 45 | 5 | 65.9% | 5.9% | 16.3% | 11.9% |
| A10-5 | 50 | 14 | 60 | 1.5 | 64.9% | 10.2% | 21.4% | 3.5% |
| A10-6 | 50 | 20 | 30 | 1.5 | 69.3% | 15.6% | 11.4% | 3.7% |
| A10-7 | 60 | 8 | 60 | 2.5 | 70.2% | 5.3% | 19.3% | 5.3% |
| A10-8 | 60 | 14 | 30 | 5 | 70.5% | 9.2% | 9.7% | 10.6% |
| A10-9 | 60 | 20 | 45 | 1.5 | 69.5% | 13.0% | 14.3% | 3.1% |

Examples 47-55

The 9 LNPs with different ratios obtained in Examples 38-46 were mixed with the siApoB solution at a mass ratio of 15: 1 (i.e. a volume ratio of 1.5: 1), gently shaken, and then incubated at 50°C for 20 minutes, so that the siApoB was encapsulated with the LNP as much as possible. Thereafter, 9 LNP/siApoB complexes were transferred into a dialysis tube (spectrum, G235035) with a molecular weight cut-off of 100000 Dalton and dialyzed in 1×PBS for 3 hours.

Test Example 14

This test example was aimed to detect the particle size, polydispersity index (PDI), and surface potential (zeta) of 9 LNP unencapsulated with siApoB obtained in Examples 38-46. The results are shown in Table 21. All formulations had the premise of being excellent delivery carriers.

Table 21

| No. | Size (nm) | PDI | zeta (mV) |
|---|---|---|---|
| A10-1 | 86.54 | 0.190 ± 0.004 | 33.65 ± 5.30 |
| A10-2 | 62.87 ± 0.02 | 0.138 ± 0.016 | 35.35 ± 0.35 |
| A10-3 | 62.58 ± 0.03 | 0.123 ± 0.027 | 34.10 ± 0.28 |
| A10-4 | 72.58 ± 0.01 | 0.233 ± 0.013 | 30.25 ± 3.89 |
| A10-5 | 72.32 ± 0.01 | 0.172 ± 0.013 | 32.90 ± 1.56 |
| A10-6 | 77.2 | 0.201 ± 0.005 | 44.70 ± 0.42 |
| A10-7 | 64.41 ± 0.01 | 0.100 ± 0.010 | 46.65 ± 1.06 |
| A10-8 | 75.53 ± 0.01 | 0.206 ± 0.006 | 36.00 ± 2.83 |
| A10-9 | 79.68 | 0.218 ± 0.004 | 39.78 ± 2.21 |

Test Example 15

This test example was aimed to detect the particle size (size), polydispersity index (PDI), surface potential (zeta), encapsulation efficiency (E. E.) and intragranular siRNA concentration (conc) of the 9 formulations encapsulated with siApoB obtained in Examples 47-55. The results are shown in Table 22. All of the formulations had the premise of being excellent delivery carriers.

Table 22

| No. | Size (nm) | PDI | zeta (mV) | E. E. (%) | Conc. (ng/μL) |
|---|---|---|---|---|---|
| siA10-1 | 180.65 ± 3.32 | 0.266 ± 0.032 | -6.05 ± 1.76 | 90.0 ± 1.1 | 151.1 |
| siA10-2 | 136.40 ± 1.41 | 0.181 ± 0.040 | -3.83 ± 0.64 | 88.7 ± 0.1 | 142.2 ± 0.9 |
| siA10-3 | 126.15 ± 1.20 | 0.172 ± 0.006 | -3.06 ± 0.44 | 86.3 ± 0.1 | 134.3 ± 0.4 |
| siA10-4 | 149.75 ± 2.19 | 0.163 ± 0.027 | -4.13 ± 0.21 | 88.1 ± 0.2 | 127.7 ± 4.0 |
| siA10-5 | 148.10 ± 0.85 | 0.201 ± 0.017 | -3.88 ± 0.13 | 93.1 ± 0.4 | 131.6 ± 0.2 |
| siA10-6 | 153.05 ± 0.49 | 0.240 ± 0.011 | -0.42 ± 0.84 | 90.8 ± 0.3 | 149.3 ± 2.3 |
| siA10-7 | 135.25 ± 1.20 | 0.119 ± 0.013 | -5.22 ± 0.42 | 87.2 ± 0.5 | 120.8 ± 2.7 |
| siA10-8 | 149.05 ± 0.92 | 0.188 ± 0.014 | 0.09 ± 0.19 | 81.3 ± 0.8 | 152.1 ± 0.5 |
| siA10-9 | 164.65 ± 2.62 | 0.190 ± 0.009 | -3.89 ± 0.92 | 89.2 ± 0.2 | 110.4 ± 0.8 |

Test Example 16

The 9 Formulations obtained in Examples 47-55 were subjected to *in vitro* transfection experiments and real-time fluorescent quantitation PCR. The results are shown in Table 23. All formulations can achieve gene inhibition.

Table 23

| No. | MRNA relative expression |
|---|---|
| siA10-1 | 0.82 ± 0.17 |
| siA10-2 | 0.89 ± 0.24 |

(continued)

| No. | MRNA relative expression |
|---|---|
| siA10-3 | 0.59 ± 0.18 |
| siA10-4 | 0.70 ± 0.32 |
| siA10-5 | 0.84 ± 0.20 |
| siA10-6 | 0.85 ± 0.04 |
| siA10-7 | 0.67 ± 0.04 |
| siA10-8 | 0.60 ± 0.10 |
| siA10-9 | 0.67 ± 0.06 |

Preparation Example 3 Synthesis of amine-containing lipid compound B8

Synthesis of compound B3

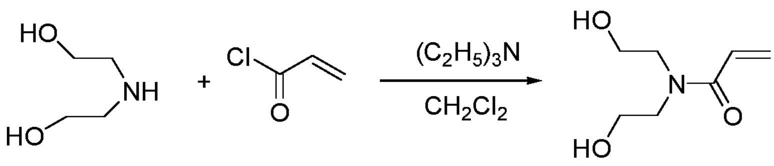


B1 B2 B3

1.0 g of compound B1 (1.0 eq) was dissolved in 15 ml of anhydrous dichloromethane. 0.97 g (1.01 eq) of triethylamine was added under ice bath, and then 0.87 g (1.01 eq) of compound B2 was dropped into the system for a reaction protected by argon. After 2 hours, it was monitored by TLC that the starting material had reacted completely. The obtained crude compound B3 was directly used for the next step without treatment.

Synthesis of compound B4

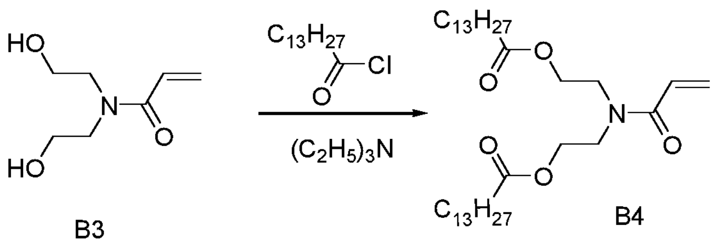


B3 B4

1.92 g (2.1 eq) of triethylamine was injected into the reaction system of the previous step, then 4.92 g of myristoyl chloride was injected into the reaction system, and the reaction was continued under the protection of argon and under ice bath. After 2 hours, it was monitored by TLC that the starting material had reacted completely. 1M dilute hydrochloric acid was added to wash for three times and $H_2O$ for one time. The organic phase was collected, dried over anhydrous sodium sulfate, concentrated under reduced pressure to remove the solvent, and purified on a silica gel column (DCM/DCM: MeOH = 500: 1) to give compound B4, 3.0 g, as a white solid. 1H NMR(400MHz, CDCl3)δ: 6.58-5.63(1H, m, CH = CH), 6.32-5.42(1H, d, CH = CH), 5.68-5.73(1, d, CH = CH), 4.18-4.30(4H, dt $NCH_2$), 3.64-3.74(4H, t $CH_2$), 2.30-2.38(4H, t $CH_2$), 1.58-1.68(4H, m),1.29-1.29(42H, m), 0.85-0.90(6H, m, $CH_3$)ppm.

Synthesis of compound B5

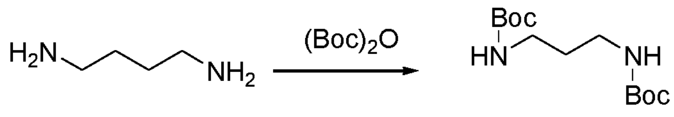


B5

1.0 g (1.0 eq) of butane diamine was dissolved in 30 ml of anhydrous dichloromethane, and 5.2 g (2.1eq) of Boc anhydride was dropped into the system under ice bath. When it was monitored by TLC that the starting material had reacted completely, 1M dilute hydrochloric acid and saturated NaHCO3 were added successively to wash. The organic phase was collected, dried over anhydrous sodium sulfate, and concentrated under reduced pressure to remove the solvent to obtain 2.2 g of compound B5, as a white solid powder.

Synthesis of compound B6

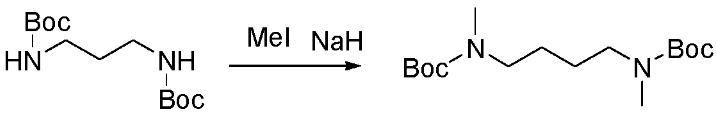


B5 B6

1.0 g (1.0 eq) of compound B5 was dissolved in 20 ml of anhydrous dichloromethane. 0.55 g (3.0 eq) of NaH with a mass fraction of 60% was slowly added into the system under ice bath. After stirring for 15 mins, 1.0 g (2.0 eq) of $CH_3I$ was slowly dropped into the system using a constant pressure dropping funnel. After 1 hour of reaction at low temperature, the reaction was moved to room temperature and allowed to react overnight. The next day, it was monitored that the starting material had reacted completely. 1M dilute hydrochloric acid was added to quench the reaction. The reaction solution was concentrated under reduced pressure to remove DMF, then dissolved with DCM, extracted, and washed with DCM/H2O. The organic phase was collected, concentrated under reduced pressure to remove the solvent, and purified on a silica gel column (PE; EA = 10: 1) to give 3.0 g of compound B6, as a white solid. 1H NMR(400MHz, CDCl3)δ: 3,24(4H, m), 2.83(6H, s, $CH_3$), 1.46-1.51(4H, m), 1.38-1.48(18H, m)ppm.

Synthesis of compound B7

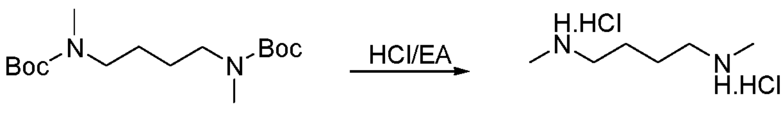


B6 B7

2.5 g (1.0 eq) of compound B6 was dissolved in 30 ml of HCl in EA solution (4M). The reaction was stirred at room temperature for 3 h. The solvent was removed by concentration under reduced pressure to give 0.8 g of compound B7, as a white solid powder.

1H NMR(400MHz, CDCl3)δ: 2.97(4H, s), 2.57(6H, s, $CH_3$), 1.56-1.61(4H, m) ppm

Synthesis of compound B8

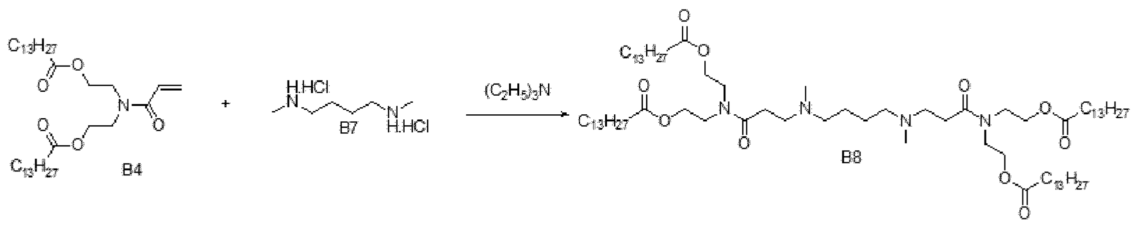


130 mg (1.0 eq) of compound B7 was dissolved in 15 ml of isopropanol, and 142.6 mg (2.05 eq) of triethylamine was added for the reaction under stirring at room temperature for 30 mins. 817.1 mg (2.05 eq) of compound B4 was added for a reaction under stirring at 90°C for 24h. It was showed by TLC that a small amount of starting material remained, and the reaction was stopped. The reaction solution was concentrated under reduced pressure to remove the solvent. DCM was added for dissolution. Then 1M diluted hydrochloric acid was added to wash for three times and H2O for one time. The organic phase was collected, dried over anhydrous sodium sulfate, concentrated under reduced pressure to remove the solvent, and purified by a silica gel column to give 136 mg of compound B8, with a yield of 16%. 1H NMR(400MHz, CDCl3)δ: 4.19-2.24(8H, m), 3.58-3.68(8H, m), 2.67-2.76(4H, m), 2.54-2.62(4H, m), 2.25-2.34(8H, m), 2.24-2.25(6H, m), 1.61-1.70(8H, m), 1.43-1.50(4H, m), 1.23-1.32(80H, m), 0.76-0.89(12H, t, $CH_3$)ppm. MASS calc. = 1275.99, found M +1 = 1276.0.

Example 56

Lipid B8 obtained in Preparation Example 2 was dissolved in absolute ethanol at a concentration of 20 mg/ml together with DSPC, cholesterol, DMG-PEG (purchased from AVT (Shanghai) Pharmaceutical Technology Co. Ltd.). B8 needs to be incubated at 30°C for 5 minutes to be completely dissolved, as a colorless and transparent liquid. DSPC, cholesterol, and DMG-PEG need to be dissolved under water bath at 50°C for several minutes until completely dissolved, all of which are colorless and transparent liquids. After preparation of the above materials, the four raw materials in a mass ratio of B8: DSPC: cholesterol: DMG-PEG=70.1:8.7:15.9:5.2 were mixed and all the mixture was sucked into an insulin syringe for later use. In addition, 10 nM citric acid/sodium citrate buffer with three times volume of the organic phase mixed solution, pH= 4.0, was also prepared, and also sucked into an insulin syringe. Thereafter, the insulin syringes containing the organic phase mixed solution and the buffer solution respectively were placed in the same centrifuge tube and all the liquid was quickly released. The centrifuge tube was gently shaken to prepare LNP B8-1.

Example 57

The aforementioned four dissolved raw materials were mixed in a mass ratio of B8: DSPC: cholesterol: DMG-PEG = 59.5: 12.9: 20.3: 7.4 and the mixture was sucked into an insulin syringe. Another citric acid/sodium citrate buffer solution with three times volume was sucked into an insulin syringe, mixed with the organic phase, and gently shaken to prepare LNP B8-2.

Example 58

The aforementioned four dissolved raw materials were mixed in a mass ratio of B8: DSPC: cholesterol: DMG-PEG = 49.7: 15.4: 22.6: 12.4 and the mixture was sucked into an insulin syringe. Another citric acid/sodium citrate buffer solution with three times volume was sucked into an insulin syringe, mixed with the organic phase, and gently shaken to prepare LNP B8-3.

Example 59

The aforementioned four dissolved raw materials were mixed in a mass ratio of B8: DSPC: cholesterol: DMG-PEG = 63.7: 6.3: 17.4: 12.7 and the mixture was sucked into an insulin syringe. Another citric acid/sodium citrate buffer solution with three times volume was sucked into an insulin syringe, mixed with the organic phase, and gently shaken to prepare LNP B8-4.

Example 60

The aforementioned four dissolved raw materials were mixed in a mass ratio of B8: DSPC: cholesterol: DMG-PEG = 62.6: 10.9: 22.8: 3.7 and the mixture was sucked into an insulin syringe. Another citric acid/sodium citrate buffer

solution with three times volume was sucked into an insulin syringe, mixed with the organic phase, and gently shaken to prepare LNP B8-5.

Example 61

The aforementioned four dissolved raw materials were mixed in a mass ratio of B8: DSPC: cholesterol: DMG-PEG = 67.1: 16.6: 12.2: 4.0 and the mixture was sucked into an insulin syringe. Another citric acid/sodium citrate buffer solution with three times volume was sucked into an insulin syringe, mixed with the organic phase, and gently shaken to prepare LNP B8-6.

Example 62

The aforementioned four dissolved raw materials were mixed in a mass ratio of B8: DSPC: cholesterol: DMG-PEG = 68.1: 5.6: 20.6: 5.6 and the mixture was sucked into an insulin syringe. Another citric acid/sodium citrate buffer solution with three times volume was sucked into an insulin syringe, mixed with the organic phase, and gently shaken to prepare LNP B8-7.

Example 63

The aforementioned four dissolved raw materials were mixed in a mass ratio of B8: DSPC: cholesterol: DMG-PEG = 68.4: 9.9: 10.4: 11.3 and the mixture was sucked into an insulin syringe. Another citric acid/sodium citrate buffer solution with three times volume was sucked into an insulin syringe, mixed with the organic phase, and gently shaken to prepare LNP B8-8.

Example 64

The aforementioned four dissolved raw materials were mixed in a mass ratio of B8: DSPC: cholesterol: DMG-PEG = 67.4: 13.9: 15.3: 3.4 and the mixture was sucked into an insulin syringe. Another citric acid/sodium citrate buffer solution with three times volume was sucked into an insulin syringe, mixed with the organic phase, and gently shaken to prepare LNP B8-9.

In the above examples, B8, DSPC, cholesterol, and DMG-PEG were set at 3 levels, respectively, as shown in Table 24:

Table 24:

| B8 | DSPC | Choleste rol | DMG-PEG |
|---|---|---|---|
| 40 | 8 | 30 | 1.5 |
| 50 | 14 | 45 | 2.5 |
| 60 | 20 | 60 | 5 |

The proportions of Examples 56-64 were designed using orthogonal design software (orthogonal design assistant ii V3.1), and the molar ratios and mass ratios of 9 LNP with different proportions are shown in Table 25:

Table 25:

| No. | Molar ratio | | | | Mass ratio | | | |
|---|---|---|---|---|---|---|---|---|
| | B8 | DSPC | Cholesterol | DMG-PEG | B8 | DSPC | Cholesterol | DMG-PEG |
| B8-1 | 40 | 8 | 30 | 1.5 | 70.1% | 8.7% | 15.9% | 5.2% |
| B8-2 | 50 | 14 | 45 | 2.5 | 59.5% | 12.9% | 20.3% | 7.4% |
| B8-3 | 60 | 20 | 60 | 5 | 49.7% | 15.4% | 22.6% | 12.4% |
| B8-4 | 50 | 8 | 45 | 5 | 63.7% | 6.3% | 17.4% | 12.7% |
| B8-5 | 50 | 14 | 60 | 1.5 | 62.6% | 10.9% | 22.8% | 3.7% |
| B8-6 | 50 | 20 | 30 | 1.5 | 67.1% | 16.6% | 12.2% | 4.0% |

(continued)

| No. | Molar ratio | | | | Mass ratio | | | |
|---|---|---|---|---|---|---|---|---|
| | B8 | DSPC | Cholesterol | DMG-PEG | B8 | DSPC | Cholesterol | DMG-PEG |
| B8-7 | 60 | 8 | 60 | 2.5 | 68.1% | 5.6% | 20.6% | 5.6% |
| B8-8 | 60 | 14 | 30 | 5 | 68.4% | 9.9% | 10.4% | 11.3% |
| B8-9 | 60 | 20 | 45 | 1.5 | 67.4% | 13.9% | 15.3% | 3.4% |

Examples 65-73

The 9 LNPs with different ratios obtained in Examples 56-64 were mixed with the siApoB solution at a mass ratio of 15: 1 (i.e. a volume ratio of 1.5: 1), gently shaken, and then incubated at 50°C for 20 minutes, so that the siApoB was encapsulated with the LNP as much as possible. Thereafter, 9 LNP/siApoB complexes were transferred into a dialysis tube(spectrum, G235035) with a molecular weight cut-off of 100000 Dalton and dialyzed in 1×PBS for 3 hours.

Test Example 17

This test example was aimed to detect the particle size (size), polydispersity index (PDI), and surface potential (zeta) of 9 LNP unencapsulated with siApoB obtained in Examples 56-64. The results are shown in Table 26. All the formulations had good physicochemical properties and had the potential to be excellent delivery carriers.

Table 26:

| No. | Size (nm) | PDI | zeta (mV) |
|---|---|---|---|
| B8-1 | 155.99 ± 0.05 | 0.303 ± 0.049 | 41.50 ± 2.12 |
| B8-2 | 92.95 ± 0.01 | 0.234 ± 0.013 | 37.90 ± 2.69 |
| B8-3 | 68.80 ± 0.04 | 0.147 ± 0.040 | 10.12 ± 2.52 |
| B8-4 | 49.21 ± 0.02 | 0.094 ± 0.019 | 45.95 ± 4.03 |
| B8-5 | 87.75 ± 0.02 | 0.243 ± 0.024 | 28.05 ± 3.18 |
| B8-6 | 115.70 ± 0.01 | 0.221 ± 0.010 | 43.90 ± 2.26 |
| B8-7 | 77.60 ± 0.02 | 0.236 ± 0.020 | 44.45 ± 4.45 |
| B8-8 | 70.26 ± 0.04 | 0.174 ± 0045 | 29.20 ± 4.10 |
| B8-9 | 220.45 ± 0.07 | 0.511 ± 0.065 | 34.50 ± 5.94 |

Test Example 18

This test example was aimed to detect the size (size), polydispersity index (PDI), surface potential (zeta), encapsulation efficiency (E.E.), and intragranular siRNA concentration (conc) of 9 formulations encapsulated with siApoB obtained in Examples 65-73. The results are shown in Table 27. All of the formulations had good physicochemical properties and had the potential to be excellent delivery carriers.

Table 27:

| No. | Size (nm) | PDI | zeta (mV) | E. E. (%) | Conc. (ng/μL) |
|---|---|---|---|---|---|
| siB8-1 | 184.05 ± 0.78 | 0.172 ± 0.021 | -9.16 ± 0.85 | 84.9 ± 0.8 | 191.2 ± 2.1 |
| siB8-2 | 131.75 ± 0.64 | 0.236 ± 0.019 | 1.15 ± 0.86 | 90.4 ± 1.1 | 189.2 ± 0.6 |
| siB8-3 | 142.40 ± 0.14 | 0.160 ± 0.002 | -3.08 ± 0.04 | 93.2 ± 0.8 | 200 |
| siB8-4 | 99.19 ± 0.38 | 0.191 ± 0.001 | 2.13 ± 0.23 | 91.1 ± 0.3 | 200 |
| siB8-5 | 213.30 ± 1.98 | 0.332 ± 0.046 | 2.33 ± 0.15 | 91.3 ± 0.3 | 185.1 ± 3.7 |

(continued)

| No. | Size (nm) | PDI | zeta (mV) | E. E. (%) | Conc. (ng/μL) |
|---|---|---|---|---|---|
| siB8-6 | 212.50 ± 2.83 | 0.325 ± 0.011 | 6.93 ± 0.25 | 89.2 ± 0.8 | 200 |
| siB8-7 | 186.90 ± 0.42 | 0.213 | 2.28 ± 0.31 | 92.6 ± 1.1 | 193.8 ± 0.5 |
| siB8-8 | 119.70 ± 0.57 | 0.179 ± 0.041 | 4.53 ± 0.93 | 85.8 ± 1.4 | 200 |
| siB8-9 | 210.75 ± 7.85 | 0.309 ± 0.033 | 1.91 ± 0.44 | 87.2 ± 1.0 | 174.4 ± 10.6 |

Test Example 19

The 9 formulations from Examples 65-73 were subjected to in vitro transfection experiments and real-time fluorescent quantitation PCR. The results are shown in Table 28. All formulations can achieve gene inhibition.

Table 28

| No. | MRNA relative expression |
|---|---|
| siB8-1 | 0.74 ± 0.01 |
| siB8-2 | 0.96 ± 0.07 |
| siB8-3 | 0.70 ± 0.05 |
| siB8-4 | 0.80 ± 0.12 |
| siB8-5 | 0.84 ± 0.14 |
| siB8-6 | 0.49 ± 0.04 |
| siB8-7 | 0.63 ± 0.02 |
| siB8-8 | 0.66 ± 0.05 |
| siB8-9 | 0.84 ± 0.02 |

Preparation Example 4 Synthesis of amine-containing lipid compound C3

Synthesis of compound C2

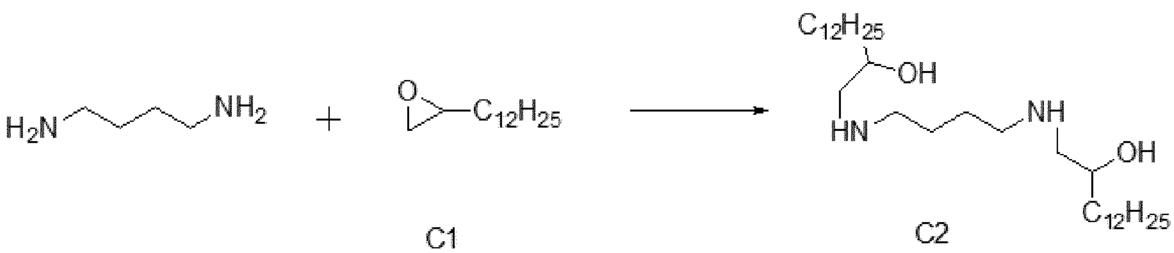

C1 C2

200 mg (2.27 mmol) of 1,4-butanediamine was dissolved in 10 ml of isopropanol and added with 1.1 g (4.76 mmol) of compound C1. The system was colorless and transparent, heated to 70°C and stirred. After 3 h, a white solid precipitated. After heating and stirring for 5 h, it was detected by TLC that the starting material C1 was completely reacted, the reaction was stopped. The isopropanol was removed under reduced pressure. 20 ml of n-hexane was added and stirred at room temperature for 1h. The reaction solution was subjected to a suction filtration to obtain compound C2 (540 mg, 47% yield). 1H NMR(400MHz, CDCl3)δ: 3.61(2H, s), 2.58-2.65(6H, m), 2.44-2.52(2H, m), 1.47-1.59(8H, m), 1.23-1.34(40H, m), 0.73-0.88(6H, t, $CH_3$)ppm.

Synthesis of compound C3

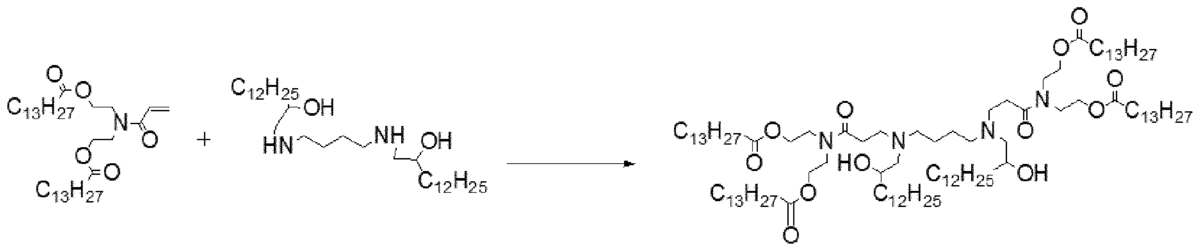

B4 C2 C3

130 mg (0.224 mmol) of compound B4 was dissolved in 5 ml of isopropanol and 57.49 mg (0.112 mmol) of compound C2 was added for a reaction at 90°C. After 10 h, it was detected by TLC that both starting materials B4 and C2 were completely reacted, and the reaction was stopped. The product was purified by the silica gel column directly without post-treatment to give the final product C3 (54 mg, 29% yield). 1H NMR(400MHz, CDCl3)$\delta$: 4.25C8H, m), 3.56-3.67(10H, m), 2.24-2.95(24H, m), 1.44-1.62(12H, m), 1.17-1.39(124H, m), 0.76-0.91(18H, t, $CH_3$)ppm. MASS calc. = 1672.68, found M + 1 = 1673.6.

Example 74

Lipid C3 obtained in Preparation Example 3 was dissolved in absolute ethanol at a concentration of 20 mg/ml together with DSPC, cholesterol, and DMG-PEG (purchased from AVT (Shanghai) Pharmaceutical Technology Co. Ltd.). C3 needs to be sonicated at 50°C for 5 minutes to be fully dissolved as a colorless and transparent liquid. DSPC, cholesterol, and DMG-PEG need to be dissolved under water bath at 50°C for several minutes until completely dissolved, all of which are colorless and transparent liquids. After preparation of the above materials, the four raw materials were mixed in a mass ratio of C3: DSPC: cholesterol: DMG-PEG=75.5:7.1:13.1:4.3, and all the mixture was sucked into an insulin syringe for later use. In addition, 10 nM citric acid/sodium citrate buffer with three times volume of the organic phase mixed solution, pH = 4.0, were also prepared, which was also sucked into an insulin syringe. Thereafter, the insulin syringes containing the organic phase mixed solution and the buffer solution respectively were placed in the same centrifuge tube and all the liquid was quickly released. The centrifuge tube was gently shaken to prepare LNP C3-1.

Example 75

The aforementioned four dissolved raw materials were mixed in a mass ratio of C3: DSPC: cholesterol: DMG-PEG = 65.8: 10.9: 17.1: 6.2 and the mixture was sucked into an insulin syringe. Another citric acid/sodium citrate buffer solution with three times volume was sucked into an insulin syringe, mixed with the organic phase, and gently shaken to prepare LNP C3-2.

Example 76

The aforementioned four dissolved raw materials were mixed in a mass ratio of B8: DSPC: cholesterol: DMG-PEG = 56.4: 13.3: 19.6: 10.7 and the mixture was sucked into an insulin syringe. Another citric acid/sodium citrate buffer solution with three times volume was sucked into an insulin syringe, mixed with the organic phase, and gently shaken to prepare LNP C3-3.

Example 77

The aforementioned four dissolved raw materials were mixed in a mass ratio of C3: DSPC: cholesterol: DMG-PEG = 69.7: 5.3: 14.5: 10.6 and the mixture was sucked into an insulin syringe. Another citric acid/sodium citrate buffer solution with three times volume was sucked into an insulin syringe, mixed with the organic phase, and gently shaken to prepare LNP C3-4.

Example 78

The aforementioned four dissolved raw materials were mixed in a mass ratio of C3: DSPC: cholesterol: DMG-PEG = 68.7: 9.1: 19.1: 3.1 and the mixture was sucked into an insulin syringe. Another citric acid/sodium citrate buffer solution with three times volume was sucked into an insulin syringe, mixed with the organic phase, and gently shaken to prepare LNP C3-5.

Example 79

The aforementioned four dissolved raw materials were mixed in a mass ratio of C3: DSPC: cholesterol: DMG-PEG = 72.8: 13.8: 10.1: 3.3 and the mixture was sucked into an insulin syringe. Another citric acid/sodium citrate buffer solution with three times volume was sucked into an insulin syringe, mixed with the organic phase, and gently shaken to prepare LNP C3-6.

Example 80

The aforementioned four dissolved raw materials were mixed in a mass ratio of C3: DSPC: cholesterol: DMG-PEG = 73.7: 4.6: 17.0: 4.7 and the mixture was sucked into an insulin syringe. Another citric acid/sodium citrate buffer solution with three times volume was sucked into an insulin syringe, mixed with the organic phase, and gently shaken to prepare LNP C3-7.

Example 81

The aforementioned four dissolved raw materials were mixed in a mass ratio of C3: DSPC: cholesterol: DMG-PEG = 74.0: 8.2: 8.5: 9.3 and the mixture was sucked into an insulin syringe. Another citric acid/sodium citrate buffer solution with three times volume was sucked into an insulin syringe, mixed with the organic phase, and gently shaken to prepare LNP C3-8.

Example 82

The aforementioned four dissolved raw materials were mixed in a mass ratio of C3: DSPC: cholesterol: DMG-PEG = 73.1: 11.5: 12.7: 2.8 and the mixture was sucked into an insulin syringe. Another citric acid/sodium citrate buffer solution with three times volume was sucked into an insulin syringe, mixed with the organic phase, and gently shaken to prepare LNP C3-9.

In the above examples, C3, DSPC, cholesterol and DMG-PEG were set at 3 levels, respectively, as shown in Table 29:

Table 29

| C3 | DSPC | Choleste rol | DMG-PEG |
|---|---|---|---|
| 40 | 8 | 30 | 1.5 |
| 50 | 14 | 45 | 2.5 |
| 60 | 20 | 60 | 5 |

The proportions of Examples 74-82 were designed using orthogonal design software (orthogonal design assistant ii V3.1), and the molar ratios and mass ratios of 9 LNP with different proportions are shown in Table 30:

Table 30:

| No. | Molar ratio | | | | Mass ratio | | | |
|---|---|---|---|---|---|---|---|---|
| | C3 | DSPC | Cholesterol | DMG-PEG | C3 | DSPC | Cholesterol | DMG-PEG |
| C3-1 | 40 | 8 | 30 | 1.5 | 75.5% | 7.1% | 13.1% | 4.3% |
| C3-2 | 50 | 14 | 45 | 2.5 | 65.8% | 10.9% | 17.1% | 6.2% |
| C3-3 | 60 | 20 | 60 | 5 | 56.4% | 13.3% | 19.6% | 10.7% |
| C3-4 | 50 | 8 | 45 | 5 | 69.7% | 5.3% | 14.5% | 10.6% |
| C3-5 | 50 | 14 | 60 | 1.5 | 68.7% | 9.1% | 19.1% | 3.1% |
| C3-6 | 50 | 20 | 30 | 1.5 | 72.8% | 13.8% | 10.1% | 3.3% |
| C3-7 | 60 | 8 | 60 | 2.5 | 73.7% | 4.6% | 17.0% | 4.7% |
| C3-8 | 60 | 14 | 30 | 5 | 74.0% | 8.2% | 8.5% | 9.3% |

(continued)

| No. | Molar ratio | | | | Mass ratio | | | |
|---|---|---|---|---|---|---|---|---|
| | C3 | DSPC | Cholesterol | DMG-PEG | C3 | DSPC | Cholesterol | DMG-PEG |
| C3-9 | 60 | 20 | 45 | 1.5 | 73.1% | 11.5% | 12.7% | 2.8% |

Examples 83-91

The 9 LNPs with different ratios obtained in Examples 74-82 were mixed with the siApoB solution at a mass ratio of 15: 1 (i.e. a volume ratio of 1.5: 1), gently shaken and then incubated at 50°C for 20 minutes, so that the siApoB was encapsulated with the LNP as much as possible. Thereafter, 9 LNP/siApoB complexes were transferred into a dialysis tube (spectrum, G235035)with a molecular weight cut-off of 100000 Dalton and dialyzed in 1× PBS for 3 hours.

Test Example 20

This test example was aimed to detect the particle size (size), polydispersity index (PDI), and surface potential (zeta) of 9 LNP unencapsulated with siApoB obtained in Examples 74-82. The results are shown in Table 31. All the formulations had good physicochemical properties and had the potential to be excellent delivery carriers.

Table 31:

| No. | Size (nm) | PDI | zeta (mV) |
|---|---|---|---|
| C3-1 | 76.93 | 0.256 ± 0.005 | 38.65 ± 4.17 |
| C3-2 | 91.38 ± 0.02 | 0380 ± 0.017 | 51.06 ± 2.76 |
| C3-3 | 53.25 ± 0.01 | 0.247 ± 0.009 | 30.65 ± 3.61 |
| C3-4 | 69.21 ± 0.01 | 0.216 ± 0.013 | 38.90 ± 0.14 |
| C3-5 | 57.71 ± 0.03 | 0.194 ± 0.027 | 36.20 ± 3.25 |
| C3-6 | 101.66 ± 0.02 | 0.247 ± 0.018 | 45.65 ± 1.20 |
| C3-7 | 83.64 ± 0.04 | 0.142 ± 0.037 | 56.65 ± 3.75 |
| C3-8 | 66.05 | 0.216 ± 0.002 | 29.45 ± 1.20 |
| C3-9 | 72.79 ± 0.01 | 0.270 ± 0.013 | 35.25 ± 3.61 |

Test Example 21

This test example was aimed to detect the particle size (size), polydispersity index (PDI), surface potential (zeta), encapsulation efficiency (E.E.), and intragranular siRNA concentration (conc) of 9 formulations encapsulated with siApoB obtained in Examples 83-91. The results are shown in Table 32. All of the formulations had good physicochemical properties and had the potential to be excellent delivery carriers.

Table 32:

| No. | Size (nm) | PDI | zeta (mV) | E.E. (%) | Conc. (ng/μL) |
|---|---|---|---|---|---|
| siC3-1 | 197.10 ± 0.14 | 0.353 ± 0.026 | -6.56 ± 1.00 | 87.4 ± 0.2 | 200 |
| siC3-2 | 176.15 ± 3.04 | 0.221 ± 0.018 | -3.82 ± 0.01 | 85.3 | 174.2 ± 2.0 |
| siC3-3 | 112.85 ± 0.78 | 0.212 ± 0.009 | 1.59 ± 0.60 | 83.1 | 200 |
| siC3-4 | 152.30 ± 0.57 | 0.249 | -2.51 ± 0.16 | 89.3 ± 0.1 | 200 |
| siC3-5 | 166.80 ± 0.42 | 0.149 ± 0.029 | -1.95 ± 1.47 | 81.4 ± 2.6 | 124.8 ± 0.8 |
| siC3-6 | 302.65 ± 5.44 | 0.406 ± 0.016 | -1.53 ± 0.88 | 78.9 ± 8.1 | 38.6 ± 3.9 |
| siC3-7 | 179.75 ± 1.20 | 0.196 ± 0.021 | -2.10 ± 0.40 | 91.4 ± 2.7 | 81.0 ± 1.1 |

(continued)

| No. | Size (nm) | PDI | zeta (mV) | E.E. (%) | Conc. (ng/μL) |
|---|---|---|---|---|---|
| siC3-8 | 142.25 ± 2.33 | 0.187 ± 0.022 | -9.57 ± 0.41 | 90.1 ± 0.1 | 200 |
| siC3-9 | 178.15 ± 2.62 | 0.246 ± 0.008 | -0.55 ± 0.12 | 78.4 ± 5.0 | 46.8 ± 1.1 |

Test Example 22

The 9 formulations obtained from Examples 83-91 were subjected to in vitro transfection experiments and real-time fluorescent quantitation PCR. The results are shown in Table 33. All formulations can achieve gene inhibition.

Table 33:

| No. | MRNA relative expression |
|---|---|
| siC3-1 | 0.89 ± 0.04 |
| siC3-2 | 1.10 ± 0.30 |
| siC3-3 | 0.91 ± 0.03 |
| siC3-4 | 0.40 ± 0.12 |
| siC3-5 | 0.68 ± 0.10 |
| siC3-6 | 0.78 ± 0.09 |
| siC3-7 | 0.62 ± 0.16 |
| siC3-8 | 0.65 ± 0.13 |
| siC3-9 | 0.57 ± 0.08 |

Example 92

Lipid A4 obtained in Preparation Example 1 was dissolved in absolute ethanol at a concentration of 20 mg/ml together with DOPE, cholesterol, DMG-PEG (purchased from AVT (Shanghai) Pharmaceutical Technology Co. Ltd.). A4 was completely soluble at room temperature as a light yellow transparent liquid. DOPE, cholesterol, and DMG-PEG need to be dissolved under water bath at 50°C for several minutes until completely dissolved, all of which are colorless and transparent liquids. After the preparation of the above materials, the four raw materials were mixed in a mass ratio of A4: DOPE: cholesterol: DMG PEG=45.0:23.1:30:2.0 and all the mixture was sucked into an insulin syringe for later use. In addition, 10 nM citric acid/sodium citrate buffer with three times volume of the organic phase mixed solution, pH = 4.0, were also prepared, and also sucked into an insulin syringe. Thereafter, the insulin syringes containing the organic phase mixed solution and the buffer solution respectively were placed in the same centrifuge tube and all the liquid was quickly released. The centrifuge tube was gently shaken to prepare lipid nanoparticles mA4-1.

Example 93

The aforementioned four dissolved raw materials were mixed in a mass ratio of A4: DOPE: cholesterol: DMG-PEG = 36.9: 28.4: 31.5: 3.2 and the mixture was sucked into an insulin syringe. Another citric acid/sodium citrate buffer solution with three times volume was sucked into an insulin syringe, mixed with the organic phase, and gently shaken to prepare LNP mA4-2.

Example 94

The aforementioned four dissolved raw materials were mixed in a mass ratio of A4: DOPE: cholesterol: DMG-PEG = 31.3: 32.1: 32.5: 4.1 and the mixture was sucked into an insulin syringe. Another citric acid/sodium citrate buffer solution with three times volume was sucked into an insulin syringe, mixed with the organic phase, and gently shaken to prepare LNP mA4-3.

Example 95

The aforementioned four dissolved raw materials were mixed in a mass ratio of A4: DOPE: cholesterol: DMG-PEG = 27.3: 35.1: 32.8: 4.8 and the mixture was sucked into an insulin syringe. Another citric acid/sodium citrate buffer solution with three times volume was sucked into an insulin syringe, mixed with the organic phase, and gently shaken to prepare LNP mA4-4.

Example 96

The aforementioned four dissolved raw materials were mixed in a mass ratio of A4: DOPE: cholesterol: DMG-PEG = 47.1: 18.1: 30.1: 4.6 and the mixture was sucked into an insulin syringe. Another citric acid/sodium citrate buffer solution with three times volume was sucked into an insulin syringe, mixed with the organic phase, and gently shaken to prepare LNP mA4-5.

Example 97

The aforementioned four dissolved raw materials were mixed in a mass ratio of A4: DOPE: cholesterol: DMG-PEG = 45.3: 26.1: 22.6: 5.9 and the mixture was sucked into an insulin syringe. Another citric acid/sodium citrate buffer solution with three times volume was sucked into an insulin syringe, mixed with the organic phase, and gently shaken to prepare LNP mA4-6.

Example 98

The aforementioned four dissolved raw materials were mixed in a mass ratio of A4: DOPE: cholesterol: DMG-PEG = 37.1: 28.5: 33.3: 1.2 and the mixture was sucked into an insulin syringe. Another citric acid/sodium citrate buffer solution with three times volume was sucked into an insulin syringe, mixed with the organic phase, and gently shaken to prepare LNP mA4-7.

Example 99

The aforementioned four dissolved raw materials were mixed in a mass ratio of A4: DOPE: cholesterol: DMG-PEG = 35.6: 34.3: 27.8: 2.3 and the mixture was sucked into an insulin syringe. Another citric acid/sodium citrate buffer solution with three times volume was sucked into an insulin syringe, mixed with the organic phase, and gently shaken to prepare LNP mA4-8.

Example 100

The aforementioned four dissolved raw materials were mixed in a mass ratio of A4: DOPE: cholesterol: DMG-PEG = 49.1: 15.1: 30.6: 5.2 and the mixture was sucked into an insulin syringe. Another citric acid/sodium citrate buffer solution with three times volume was sucked into an insulin syringe, mixed with the organic phase, and gently shaken to prepare LNP mA4-9.

Example 101

The aforementioned four dissolved raw materials were mixed in a mass ratio of A4: DOPE: cholesterol: DMG-PEG = 44.2: 20.4: 31.8: 3.5 and the mixture was sucked into an insulin syringe. Another citric acid/sodium citrate buffer solution with three times volume was sucked into an insulin syringe, mixed with the organic phase, and gently shaken to prepare LNP mA4-10.

Example 102

The aforementioned four dissolved raw materials were mixed in a mass ratio of A4: DOPE: cholesterol: DMG-PEG = 48.4: 29.8: 19.3: 2.5 and the mixture was sucked into an insulin syringe. Another citric acid/sodium citrate buffer solution with three times volume was sucked into an insulin syringe, mixed with the organic phase, and gently shaken to prepare LNP mA4-11.

Example 103

The aforementioned four dissolved raw materials were mixed in a mass ratio of A4: DOPE: cholesterol: DMG-PEG = 43.3: 33.3: 22.2: 1.1 and the mixture was sucked into an insulin syringe. Another citric acid/sodium citrate buffer solution with three times volume was sucked into an insulin syringe, mixed with the organic phase, and gently shaken to prepare LNP mA4-12.

Example 104

The aforementioned four dissolved raw materials were mixed in a mass ratio of A4: DOPE: cholesterol: DMG-PEG = 52.6: 13.5: 31.6: 2.3 and the mixture was sucked into an insulin syringe. Another citric acid/sodium citrate buffer solution with three times volume was sucked into an insulin syringe, mixed with the organic phase, and gently shaken to prepare LNP mA4-13.

Example 105

The aforementioned four dissolved raw materials were mixed in a mass ratio of A4: DOPE: cholesterol: DMG-PEG = 51.9: 20.0: 27.0: 1.1 and the mixture was sucked into an insulin syringe. Another citric acid/sodium citrate buffer solution with three times volume was sucked into an insulin syringe, mixed with the organic phase, and gently shaken to prepare LNP mA4-14.

Example 106

The aforementioned four dissolved raw materials were mixed in a mass ratio of A4: DOPE: cholesterol: DMG-PEG = 49.3: 25.3: 21.0: 4.3 and the mixture was sucked into an insulin syringe. Another citric acid/sodium citrate buffer solution with three times volume was sucked into an insulin syringe, mixed with the organic phase, and gently shaken to prepare LNP mA4-15.

Example 107

The aforementioned four dissolved raw materials were mixed in a mass ratio of A4: DOPE: cholesterol: DMG-PEG = 49.0: 31.4: 16.3: 3.2 and the mixture was sucked into an insulin syringe. Another citric acid/sodium citrate buffer solution with three times volume was sucked into an insulin syringe, mixed with the organic phase, and gently shaken to prepare LNP mA4-16.

In the above examples, A4, DOPE, cholesterol, and DMG-PEG were set at 4 levels, respectively, as shown in Table 34:

Table 34:

| A4 | DOPE | Choleste rol | DMG-PEG |
|---|---|---|---|
| 15 | 10 | 25 | 0.25 |
| 20 | 15 | 32 | 0.5 |
| 25 | 20 | 39 | 0.75 |
| 30 | 25 | 45 | 1 |

The proportions of Examples 92-107 were designed using orthogonal design software (orthogonal design assistant ii V3.1), and the molar ratios and mass ratios of 16 LNP with different proportions are shown in Table 35:

Table 35:

| No. | Molar ratio | | | | Mass ratio | | | |
|---|---|---|---|---|---|---|---|---|
| | A4 | DOPE | Cholesterol | DMG-PEG | A4 | DOPE | Cholesterol | DMG-PEG |
| mA4-1 | 15 | 10 | 25 | 0.25 | 45.0% | 23.1% | 30.0% | 2.0% |
| mA4-2 | 15 | 15 | 32 | 0.5 | 36.9% | 28.4% | 31.5% | 3.2% |
| mA4-3 | 15 | 20 | 39 | 0.75 | 31.3% | 32.1% | 32.5% | 4.1% |

(continued)

| No. | Molar ratio | | | | Mass ratio | | | |
|---|---|---|---|---|---|---|---|---|
| | A4 | DOPE | Cholesterol | DMG-PEG | A4 | DOPE | Cholesterol | DMG-PEG |
| mA4-4 | 15 | 25 | 45 | 1 | 27.3% | 35.1% | 32.8% | 4.8% |
| mA4-5 | 20 | 10 | 32 | 0.75 | 47.1% | 18.1% | 30.1% | 4.6% |
| mA4-6 | 20 | 15 | 25 | 1 | 45.3% | 26.1% | 22.6% | 5.9% |
| mA4-7 | 20 | 20 | 45 | 0.25 | 37.0% | 28.5% | 33.3% | 1.2% |
| mA4-8 | 20 | 25 | 39 | 0.5 | 35.6% | 34.3% | 27.8% | 2.3% |
| mA4-9 | 25 | 10 | 39 | 1 | 44.2% | 20.4% | 31.8% | 3.5% |
| mA4-10 | 25 | 15 | 45 | 0.75 | 48.4% | 29.8% | 19.3% | 2.5% |
| mA4-11 | 25 | 20 | 25 | 0.5 | 43.3% | 33.3% | 22.2% | 1.1% |
| mA4-12 | 25 | 25 | 32 | 0.25 | 52.6% | 13.5% | 31.6% | 2.3% |
| mA4-13 | 30 | 10 | 45 | 0.5 | 51.9% | 20.0% | 27.0% | 1.1% |
| mA4-14 | 30 | 15 | 39 | 0.25 | 49.3% | 25.3% | 21.0% | 4.3% |
| mA4-15 | 30 | 20 | 32 | 1 | 49.0% | 31.4% | 16.3% | 3.2% |
| mA4-16 | 30 | 25 | 25 | 0.75 | 49.1% | 15.1% | 30.6% | 5.2% |

Examples 108-123

The concentration of luciferase mRNA (mLuc) was adjusted to 1000 ng/$\mu$L and mixed with an equal volume of 50% ethanol solution to prepare a 500 ng/$\mu$L mLuc solution with an ethanol content of 25%.

The 16 lipid nanoparticles with different ratios obtained in Examples 92-107 were mixed with the mLuc solution at a mass ratio of 15: 1 (i.e. a volume ratio of 1.5: 1), gently shaken and then incubated at 50°C for 20 minutes, so that the mLuc was encapsulated with the lipid nanoparticles as much as possible. Thereafter, 16 LNP/mLuc complexes were transferred into a dialysis tube (spectrum, G235035) with a molecular weight cut-off of 100000 Dalton and dialyzed in 1$\times$PBS for 3 hours.

Test Example 23

This test example was aimed to detect the in vitro mRNA expression efficiency of the 16 formulations obtained in Examples 108-123, and the test results are shown in FIG. 9. The results showed that 16 formulations could mediate the high expression of mRNA in cells, with mLuA4-12 performing the best.

Test Example 24

This test example was aimed to detect the metabolic characteristics of mLuA4-12 obtained in Example 119 in C57BL/6 mice. The expression amount of luciferase in each major organ 6 hours after tail vein injection of mLuA4-12 is shown in FIG. 10, indicating that mA4-12 can mediate the high expression of mRNA *in vivo.*

Example 124

The concentration of the plasmid pCPLK1 expressing both PLK1 sgRNA and Cas9 protein was adjusted to 1000 ng/$\mu$L and mixed with an equal volume of 50% ethanol solution to prepare a 500 ng/$\mu$L pCPLK1 solution with an ethanol content of 25%. The mA4-12 obtained in Example 92 was mixed with the pCPLK1 solution at a mass ratio of 15: 1 (i.e. a volume ratio of 1.5: 1), gently shaken and then incubated at 50°C for 20 minutes so that the pCPLK1 was encapsulated with lipid nanoparticles as much as possible to obtain pCPA4-12. Thereafter, sgPLKA1-12 complex was transferred into a dialysis tube(spectrum, G235035) with a molecular weight cut-off of 100000 Dalton and dialyzed in 1$\times$PBS for 3 hours.

Example 125

The concentration of PLK1 siRNA was adjusted to 1000 ng/μL and mixed with an equal volume of 50% ethanol to prepare a 500 ng/μL siPLK1 solution with an ethanol content of 25%. A4-13 was mixed with the siPLK1 solution at a mass ratio of 15: 1 (i.e. a volume ratio of 1.5: 1), gently shaken and then incubated at 50°C for 20 minutes so that the siPLK1 was encapsulated with lipid nanoparticles as much as possible to obtain siPLKA4-13. Thereafter, siPLKA4-13 complex was transferred into a dialysis tube (spectrum, G235035) with a molecular weight cut-off of 100000 Dalton and dialyzed in 1 ×PBS for 3 hours.

Example 126

The sgRNA targeting PLK1 was dissolved in DEPC-treated water and the concentration was adjusted to 1000 ng/μ L, which was then mixed with an equal volume of 50% ethanol solution to prepare a 500 ng/μL sgPLK1 solution with an ethanol content of 25%. The Cas9 protein was dissolved in DEPC-treated water and the concentration was determined using the BCA method. The solution was mixed with an equal amount of sgPLK1. Thereafter, A4-13 was mixed with the above solution at a mass ratio of 30:1, gently shaken and incubated at 50°C for 20 minutes to ensure that sgPLK1 and Cas9 protein were encapsulated by lipid nanoparticles as much as possible to obtain CasPLKA4-13. After that, the CasPLKA4-13 complex was transferred to a dialysis tube (spectrum, G235035) with a molecular weight cutoff of 100000 Dalton and dialyzed in 1×PBS for 3 hours.

The HepG2 cells were used as the experimental object, cells were transfected with CasPLKA4-13 24 hours after plating, and siPLKA4-13 was used as a positive control. After 24 hours, cells were harvested. The relative expression of PLK1 was detected by QPCR.

Example 127

Doxorubicin hydrochloride was dissolved in PBS to be fully dissolved, and the concentration was determined. A4-13 was mixed with the above solution at a mass ratio of 15:1, gently shaken, and incubated at room temperature for 20 minutes to form the DOXA4-13 complex. After that, the DOXA4-13 complex was transferred to a dialysis tube (spectrum, G235035) with a molecular weight cutoff of 100,000 Dalton and dialyzed in 1×PBS for 3 hours.

The HepG2 cells were used as the experimental object, cells were transfected with DOXA4-13 24 hours after plating, and free DOX was used as a positive control. After 24 hours, cells were harvested. The relative viability of cells treated with different samples was detected by the MTT assay.

Test Example 25

This test example was aimed to detect the editing efficiency of the target gene of pCPA4-12 obtained in Example 124 at the cellular level and compare the gene inhibition efficiency with that of siPLKA4-13 obtained in Example 125. The results are shown in Table 34. The results showed that the expression of target gene PLK1 in cells treated with pCPA4-12 was significantly higher than that in cells treated with siPLKA4-13, but there was still a very significant difference compared with PBS control group, indicating that mA4-12 has a good prospect of gene editing.

Table 35

| Group | MRNA relative expression |
|---|---|
| PBS | 1.00 ± 0.02 |
| siPLKA4-13 | 0.14 ± 0.04 |
| pCPA4-12 | 0.66 ± 0.13 |

Comparative example. 1

This comparative example was aimed to compare the stability of the lipid A4 obtained in Preparation Example 1 with the control lipid E. The chemical structure of lipid E is shown in FIG. 11.

Lipid A4 and lipid E were dissolved in a solvent containing 90% absolute ethanol and 10% citrate (W/W) at a concentration of 0.05M and a pH of 4.0, respectively. 100 μL of sample was immediately pipetted as a pre-incubation sample and immediately stored at -80°C to prevent sample degradation. This day was day 0 of the experiment. Thereafter, A4 and E test samples were placed at 40°C for continuous incubation, respectively. 100 μL samples were pipetted on

the 1st, 3rd, 5th and 7th day of the experiment and stored at -80°C. After all samples were obtained, the samples were tested for stability using HPLC under the following conditions:

Table 34:

| Parameter | Set |
|---|---|
| Column type | Waters XBridge C8: 186006050 |
| Flow rate (LPM) | 1 ml/min |
| Column temperature | 50°C |
| High-pressure limit | 10000 psi |
| Sample temperature | 10°C |
| Moving phase A | 0.05% TFA-Water |
| Moving phase A | 0.05%TFA-33%ACN-67%IPA |
| Test time | 16 mins |

Table 35:

| Time (min) | Flow Rate (mL/min) | Moving phase A (%) | Moving phase B (%) | curve |
|---|---|---|---|---|
| 0 | 1 | 50 | 50 | Start |
| 1 | 1 | 50 | 50 | 6 |
| 10 | 1 | 5 | 95 | 6 |
| 14 | 1 | 5 | 95 | 6 |
| 16 | 1 | 50 | 50 | 1 |

Comparative example. 2

Lipofectime 2000 (Lipo) is a commercially available transfection reagent that mediates efficient nucleic acid delivery at the cellular level, whereas it rarely achieves efficient delivery at the animal level. This experiment was aimed to compare the efficacy of A4-13 in C57 mice (administered at a dose of 0.5 mg/kg), the relative expression of Lipo/siApoB is 0.85 and the relative expression of A4-13/siApoB is 0.27. The results are shown in FIG. 12. Experimental results demonstrated that Lipo delivery is inefficient *in vivo,* whereas A4-13 can achieve efficient siRNA delivery.

Comparative example. 3

This test example was aimed to detect the cellular uptake efficiency of Cy-siFLA4-13 obtained in Example 34. The results of the assay, as shown in Table 35, indicated that the delivery efficiency of this formulation at the cellular level was comparable to that of commercial transfection reagents. Considering that Lipo cannot achieve efficient nucleic acid delivery at the animal level, A4-13 has obvious advantages in the application potential of nucleic acid delivery.

Table 35:

| Group | Mean fluorescence signal intensity | Positive rate (%) |
|---|---|---|
| Mock | 4.3 ± 2.3 | 0.4±0.1 |
| Lipo | 5370.5 ± 313.2 | 91.9 ± 3.9 |
| Cy-siFLA4-13 | 4502.5 ± 477.3 | 90.4 ± 4.2 |

Comparative example. 4

This comparative example was aimed to compare the cell-mediated endocytosis efficiency of the complex obtained by Lipo after encapsulating mRNA with that of mLuA4-12 obtained in Example 119. The test results are shown

in Table 36, indicating that both Lipo and mLuA4-12 can successfully endocytose luciferase mRNA in almost all cells and that Lipo can achieve a higher average fluorescence signal intensity, i.e. more luciferase mRNA can be endocytosed by a single cell. However, considering that Lipo cannot achieve efficient nucleic acid delivery at the animal level,, mA4-12 has a broader range of applications.

Table 36:

| Group | Mean fluorescence signal intensity | Positive rate (%) |
|---|---|---|
| Mock | 5.3 ± 1.9 | 0.8 ± 0.2 |
| Lipo | 6262 | 98.85 ± 0.07 |
| mLuA4-12 | 4381 | 98.25 ± 0.07 |

Comparative example. 5

This comparative example was aimed to compare the cell-mediated delivery efficiency of the siE obtained by liposome E which is obtained by using lipid E as the core lipid, and further incubation with siApoB with siA4-13 obtained in Example 29. The results are shown in Table 37, indicating that siA4-13 can achieve efficient target gene suppression at the cellular level, while siE can hardly achieve target gene suppression at the cellular level.

Table 37

| Group | MRNA relative expression |
|---|---|
| siE | 0.891 ± 0.072 |
| siA-13 | 0.402 ± 0.066 |

In the description of this specification, references to descriptions of the terms "one embodiment", "some embodiments", "example", "specific examples", or "some examples", etc. mean that a particular feature, structure, material, or characteristic described in connection with the embodiment or example is included in at least one embodiment or example of the present disclosure. In this description, schematic representations of the terms above do not necessarily refer to the same embodiment or example. Furthermore, the particular features, structures, materials, or characteristics described may be combined in any suitable manner in any one or more embodiments or examples. Further, without contradicting each other, the different embodiments or examples and the features of the different embodiments or examples described in this description can be integrated and combined by a person skilled in the art.

While the examples of the present disclosure have been shown and described, it will be understood that the above-described embodiments are exemplary and should not be construed as limiting the present disclosure and changes, modifications, substitutions, and alterations may be made by those skilled in the art without departing from the scope of the present disclosure.

## Claims

1. A compound represented by formula (I) or a stereoisomer, tautomer, solvate, or pharmaceutically acceptable salt of the compound represented by formula (I),

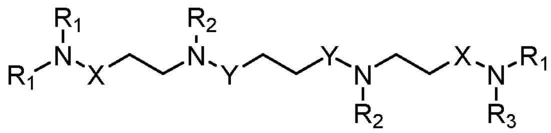

(I)

wherein

X and Y are each independently selected from $-CH_2-$ or -CO-, respectively; X and Y are not $-CH_2$ - or -CO- at the same time, wherein each X is the same or different and each Y is the same or different;

$R_2$ is selected from H, optionally substituted $C_1$-$C_{20}$ alkyl, optionally substituted $C_1$-$C_{20}$ alkenyl, or optionally substituted $C_1$-$C_{20}$ alkynyl; $R_3$ is selected from H, optionally substituted $C_1$-$C_{20}$ alkyl, optionally substituted $C_1$-$C_{20}$ alkenyl or optionally substituted $C_1$-$C_{20}$ alkynyl, wherein the substituent groups are each independently selected from H, F, Cl, Br, CN, and $NO_2$;
$R_1$ is selected from

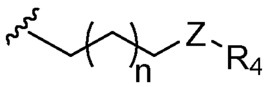 or 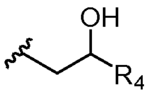,

wherein n is an integer selected from 0-10, and Z is selected from

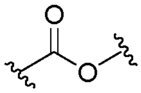 or 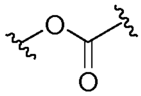;

$R_4$ is selected from optionally substituted $C_4$-$C_{20}$ alkyl, optionally substituted $C_4$-$C_{20}$ alkenyl or optionally substituted $C_4$-$C_{20}$ alkynyl, wherein the substituent groups are each independently selected from H, F, Cl, Br, CN, and $NO_2$.

**2.** The compound of claim 1, wherein, $R_2$ is selected from H, $CH_3$-, or Ri; $R_3$ is selected from H or $R_1$;

$R_1$ is selected from

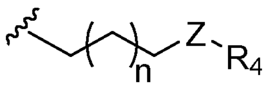 or 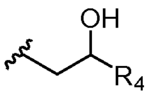,

wherein n is an integer selected from 1-5, and Z is selected from

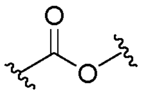 or 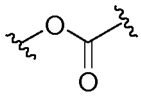;

$R_4$ is selected from $C_4$-$C_{20}$ alkyl, $C_4$-$C_{20}$ alkenyl or $C_4$-$C_{20}$ alkynyl, wherein the $C_4$-$C_{20}$ alkenyl contains 1-3 double bonds and the $C_4$-$C_{20}$ alkynyl contains 1-3 triple bonds.

**3.** The compound of claim 2, wherein, $R_4$ is selected from $C_5$-$C_{15}$ alkyl, $C_5$-$C_{15}$ alkenyl, wherein the $C_5$-$C_{15}$ alkenyl contains 1-3 double bonds.

**4.** The compound of claim 3, wherein, $R_1$, $R_2$, and $R_3$ are the same; and
$R_4$ is selected from $C_{10}$-$C_{15}$ alkyl, $C_{10}$-$C_{15}$ alkenyl, wherein the $C_{10}$-$C_{15}$ alkenyl contains 1-3 double bonds.

**5.** The compound of claim 4, having one of the following structures:

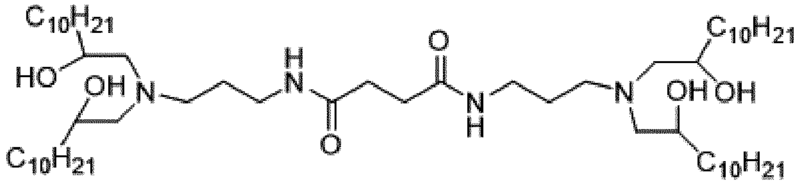

;

(A4)

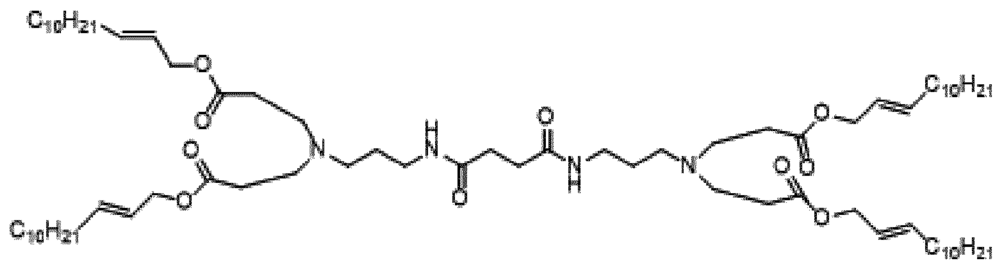

;

(A10)

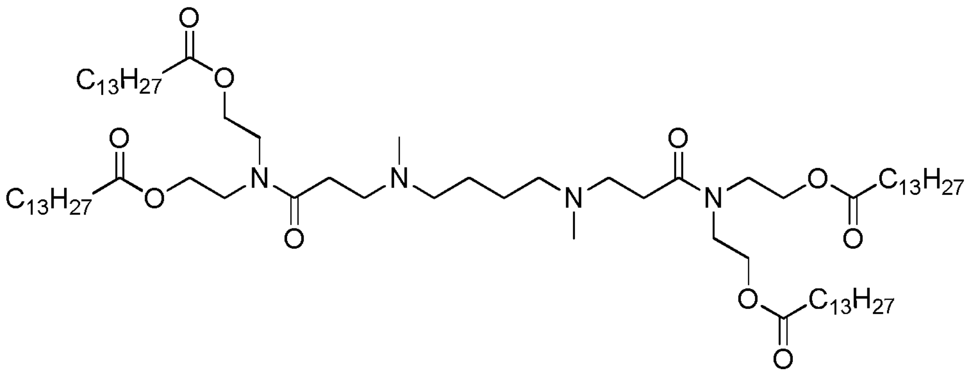

;

(B8)

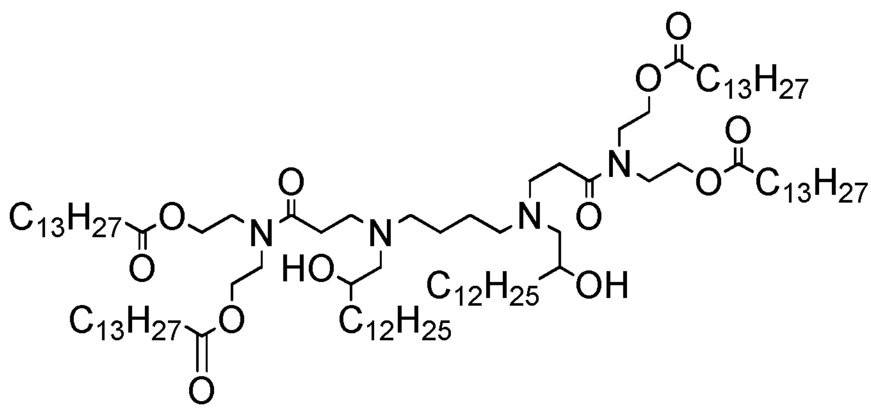

(C3).

**6.** Use of the compound of any one of claims 1-5 in the preparation of a liposome.

7. A liposome made of the compound of any one of claims 1-4.

8. The liposome of claim 7, further comprises at least one selected from the group consisting of a hydrophobic lipid, an amphiphilic lipid, a buffering agent, and an organic solvent, wherein the amphiphilic lipid comprises at least one selected from the group consisting of a neutral lipid and a PEG-lipid, and the hydrophobic lipid is selected from sterols;

   optionally, the neutral lipid comprises at least one selected from the group consisting of: distearoyl phosphatidyl choline, dipalmitoyl phosphatidyl choline, phosphatidyl choline, dioleoyl phosphatidyl ethanolamine, dilauroyl phosphatidyl choline, diethyl pyrocarbonate, dimyristoyl phosphatidyl choline, and egg phosphatidylcholine;
   optionally, the PEG-lipid comprises at least one selected from the group consisting of dipalmitoyl phosphatidyl ethanolamine-PEG, distearoyl phosphatidyl ethanolamine-PEG, dimyristyl glycerol, and dimethacrylate;
   optionally, the sterol is cholesterol;
   optionally, the buffering agent is an acidic buffering agent;
   optionally, the buffering agent comprises at least one selected from citric acid, sodium citrate, acetic acid, sodium acetate, disodium hydrogen phosphate, sodium dihydrogen phosphate, trihydroxymethylaminomethane-hydrochloric acid, potassium dihydrogen phosphate-sodium hydroxide, boric acid-borax, glycine-hydrochloric acid, phthalic acid-hydrochloric acid, potassium hydrogen phthalate, and sodium dihydrogen phosphate-citric acid;
   optionally, the organic solvent comprises at least one selected from methanol, ethanol, isopropanol, benzene, toluene, xylene, pentane, hexane, octane, cyclohexane, cyclohexanone, toluene cyclohexanone, chlorobenzene, dichlorobenzene, dichloromethane, ethyl ether, propylene oxide, acetone, methyl butanone, methyl isobutyl ketone, acetonitrile, pyridine, phenol, styrene, perchloroethylene, trichloroethylene, vinyl ethylene glycol ether, and triethanolamine.

9. The liposome of claim 8, wherein an amount of the compound is 20-80% mol/mol based on the liposome;

   optionally, a molar ratio of the compound, the neutral lipid, the sterol, and the PEG-lipid is (20-80): (5-50): (10-60): (0.01-20);
   optionally, a volume of the buffering agent is 50-90% v/v based on the liposome; preferably, the volume of the buffering agent is 75% v/v based on the liposomes.

10. A method for preparing the liposome of any one of claims 7-9, comprising:
   mixing a predetermined amount of the compound of any one of claims 1-5 with an organic solvent so as to obtain the liposomes.

11. The method of claim 10, further comprising:
   mixing a predetermined amount of the hydrophobic lipid, the amphiphilic lipid and/or the buffering agent with solution obtained in claim 10, so as to obtain the liposome.

12. A drug carrier comprising the compound of any one of claims 1 and 2 or the liposome of any one of claims 3-9.

13. The use of the compound of any one of claims 1-5, the liposome of any one of claims 7-9, or the drug carrier of claim 12 in the preparation of a drug.

14. A pharmaceutical composition comprising a carrier and a pharmaceutically active ingredient, wherein the carrier comprises the drug carrier of claim 12.

15. The pharmaceutical composition of claim 14, wherein the pharmaceutically active ingredient comprises at least one selected from the group consisting of
   a DNA molecule, an RNA molecule, a protein, a polypeptide and a small molecule drug.

16. The pharmaceutical composition of claim 14, wherein the pharmaceutically active ingredient is negatively charged.

17. The pharmaceutical composition of claim 15, wherein the pharmaceutically active ingredient contains nucleic acid.

18. The pharmaceutical composition of claim 14, wherein the mass ratio of the carrier to the pharmaceutically active ingredient is (5-40): 1;

   preferably, the mass ratio of the carrier to the pharmaceutically active ingredient is (8-20): 1; and

preferably, the mass ratio of the carrier to the pharmaceutically active ingredient is 15: 1.

**19.** A transfection complex, comprising the compound of any one of claims 1-5 or the liposome of claims 7-9.

**20.** The transfection complex of claim 19, wherein the transfection complex comprises at least one bioactive agent.

**21.** The transfection complex of claim 20, wherein, the bioactive agent comprises at least one selected from the group consisting of:
a DNA molecule, an RNA molecule, a protein, and a drug.

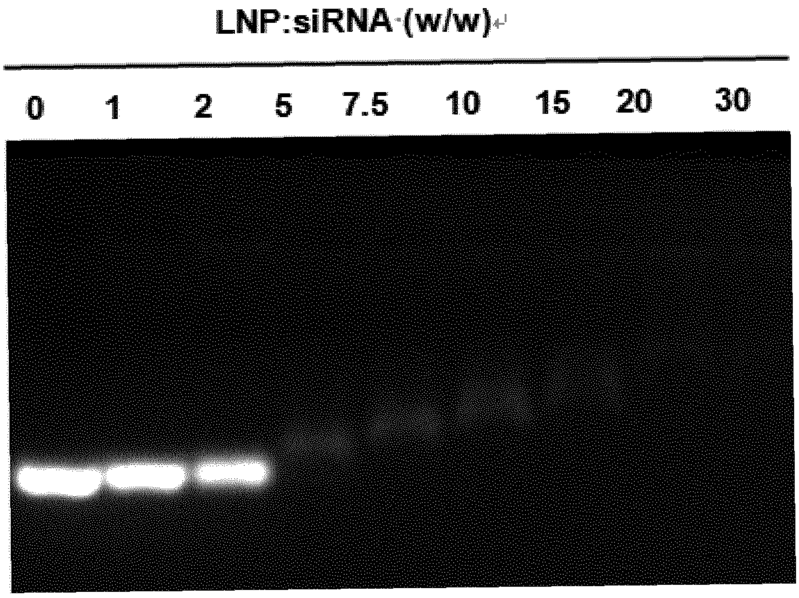
LNP:siRNA (w/w)
0
1
2
5
7.5
10
15
20
30

Fig. 1

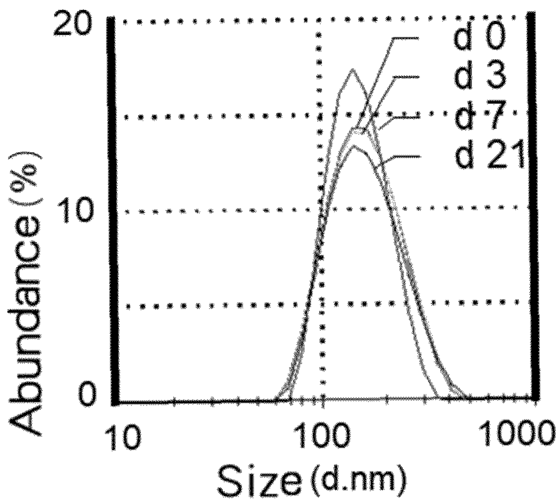
20
10
0
Abundance (%)
10
100
1000
Size (d.nm)
d 0
d 3
d 7
d 21

Fig. 2

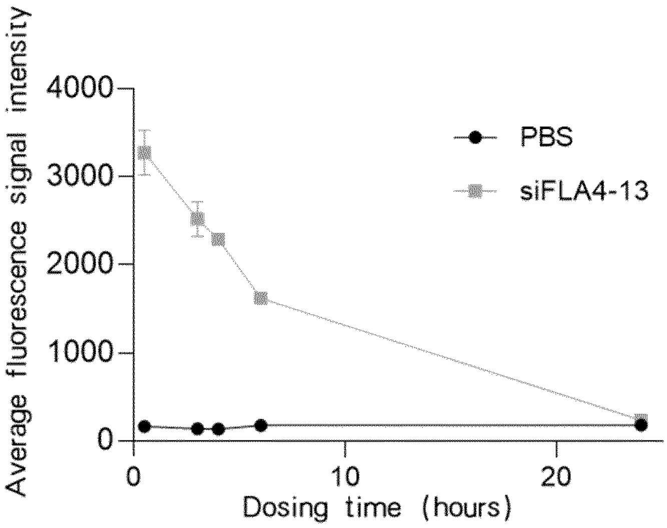
Average fluorescence signal intensity
4000
3000
2000
1000
0
0
10
20
Dosing time (hours)
PBS
siFLA4-13

Fig. 3

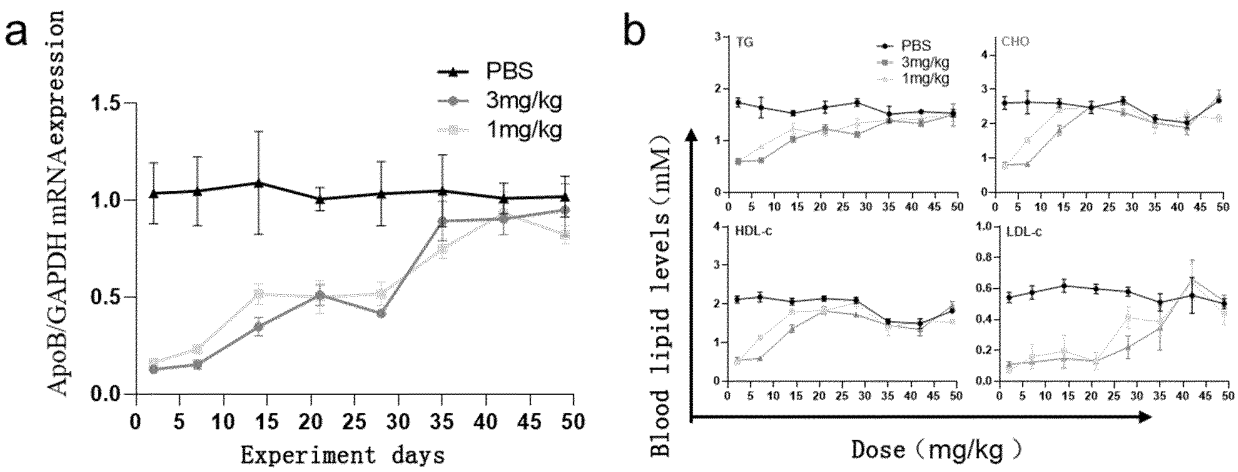
a
ApoB/GAPDH mRNAexpression
PBS
3mg/kg
1mg/kg
Experiment days
b
TG
CHO
HDL-c
LDL-c
Blood lipid levels (mM)
Dose (mg/kg)

Fig. 4

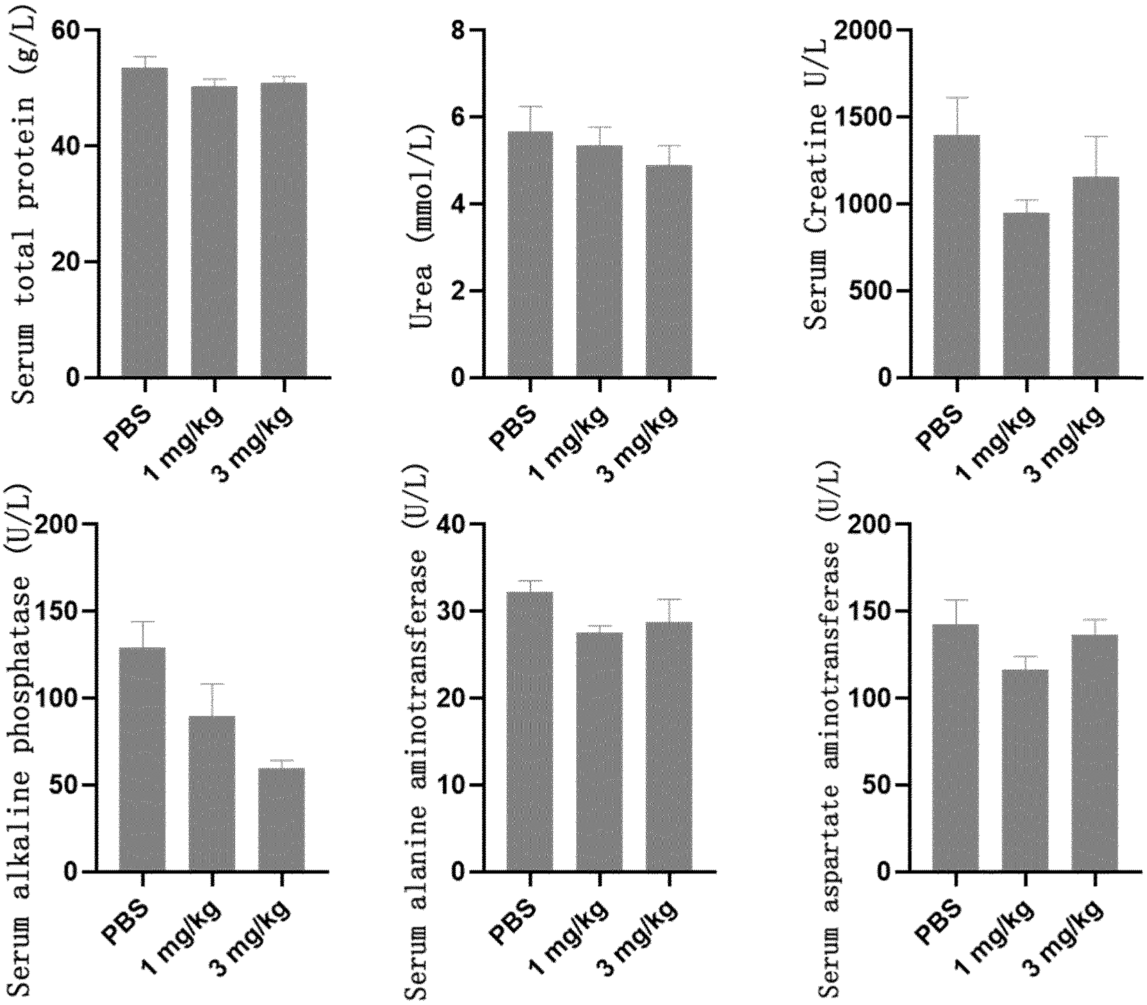
Serum total protein (g/L)
PBS
1 mg/kg
3 mg/kg
Urea (mmol/L)
Serum Creatine U/L
Serum alkaline phosphatase (U/L)
Serum alanine aminotransferase (U/L)
Serum aspartate aminotransferase (U/L)

Fig. 5

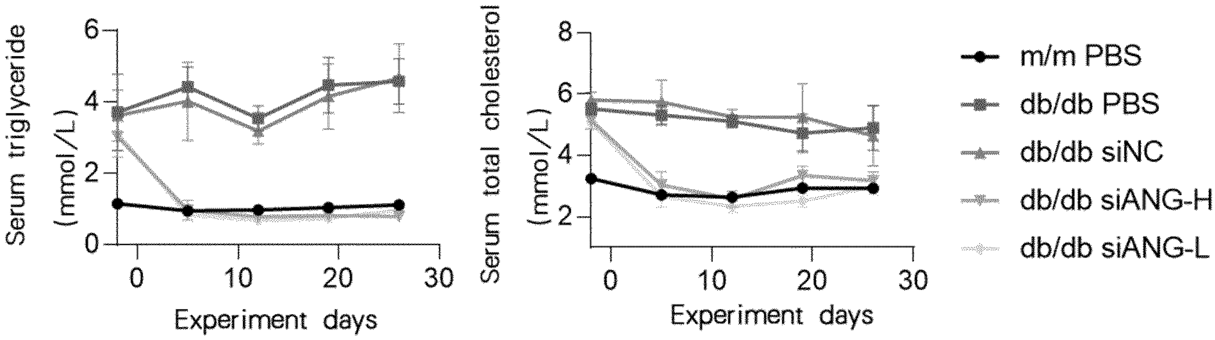
Serum triglyceride (mmol/L)
Serum total cholesterol (mmol/L)
Experiment days
m/m PBS
db/db PBS
db/db siNC
db/db siANG-H
db/db siANG-L

Fig. 6

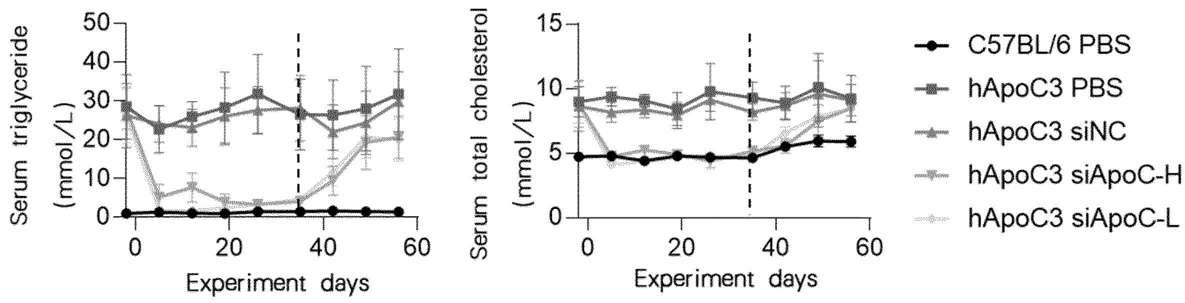


Fig. 7

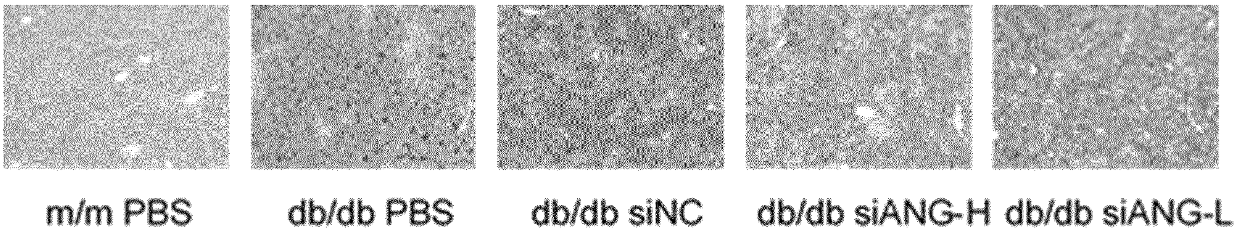


Fig. 8

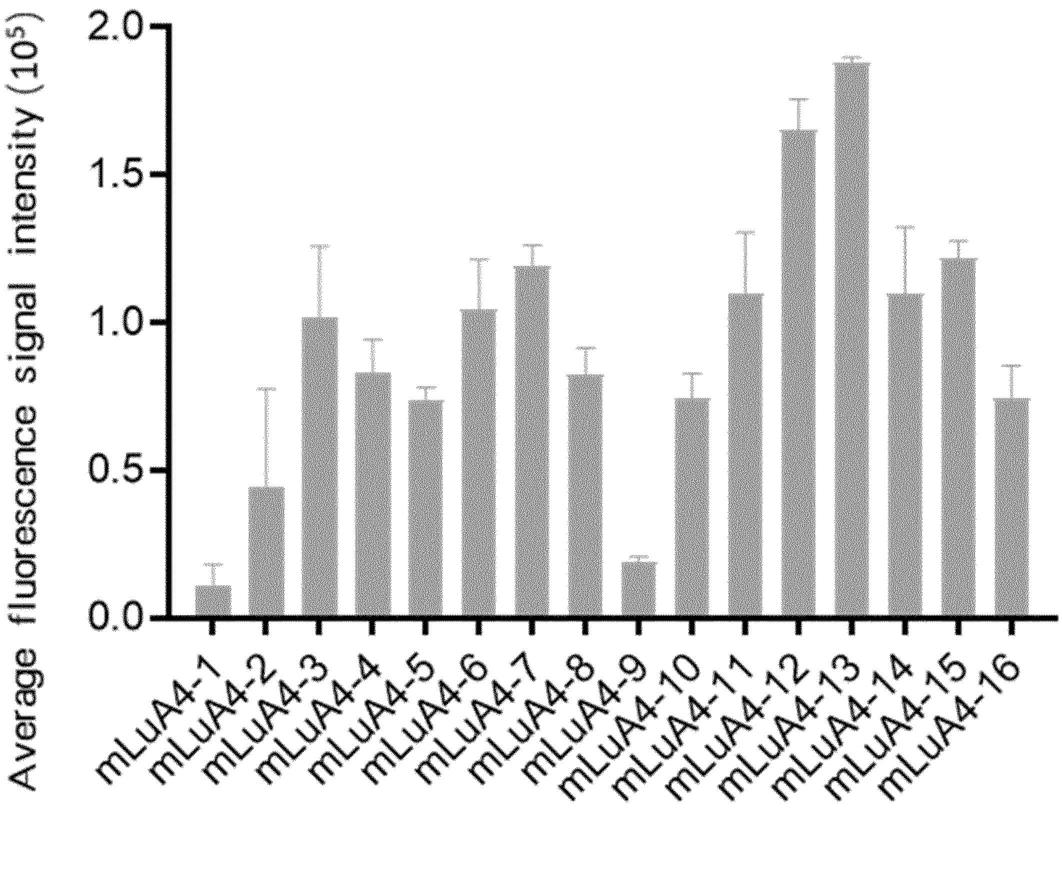


Fig. 9

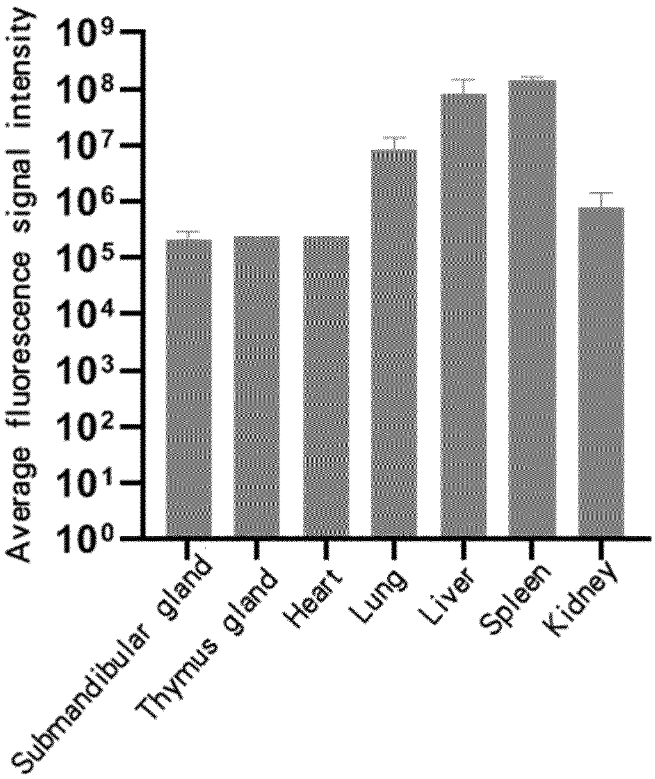
Average fluorescence signal intensity
10⁹
10⁸
10⁷
10⁶
10⁵
10⁴
10³
10²
10¹
10⁰
Submandibular gland
Thymus gland
Heart
Lung
Liver
Spleen
Kidney

Fig. 10

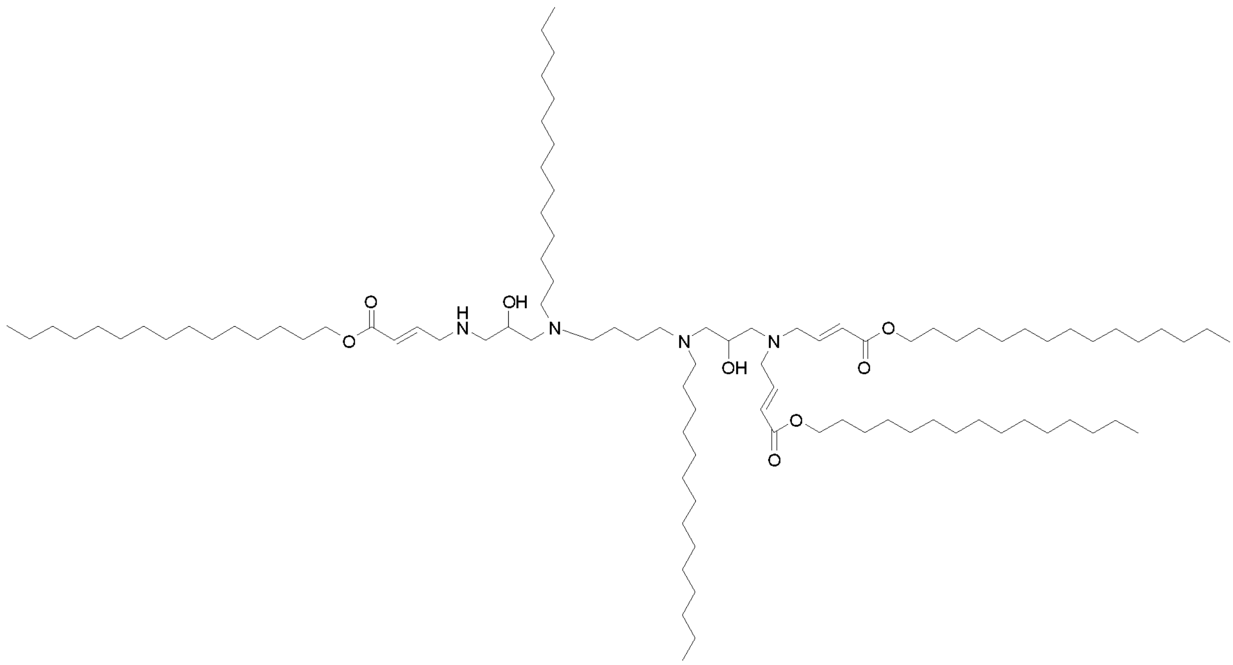

Fig. 11

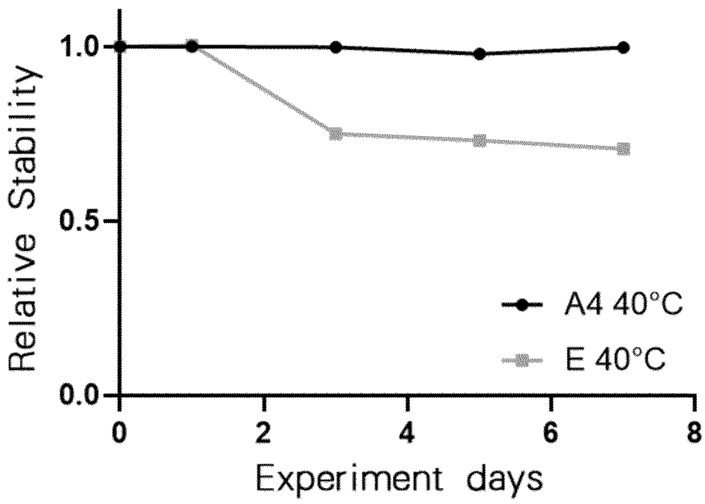
Relative Stability
1.0
0.5
0.0
0
2
4
6
8
Experiment days
A4 40°C
E 40°C

Fig. 12

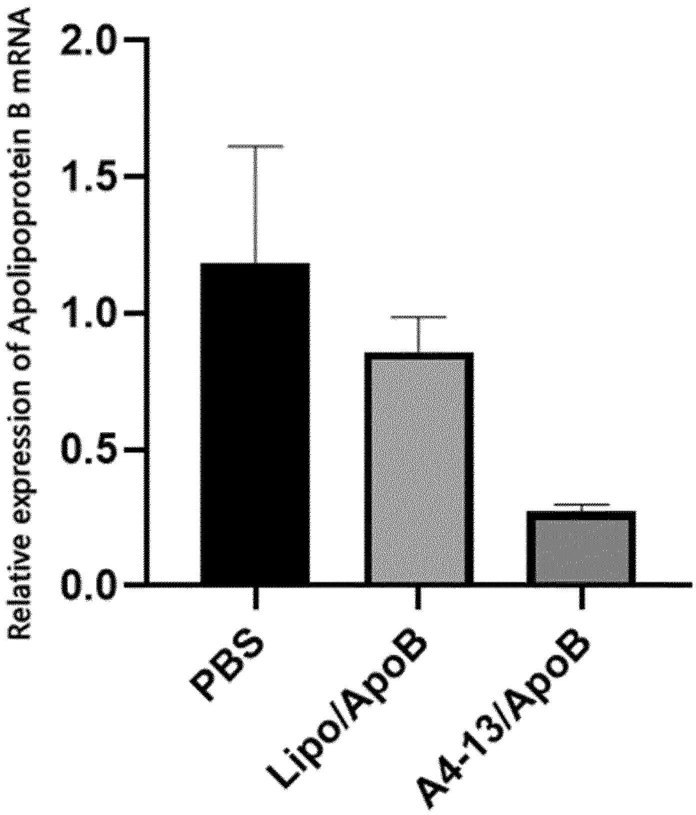
Relative expression of Apolipoprotein B mRNA
2.0
1.5
1.0
0.5
0.0
PBS
Lipo/ApoB
A4-13/ApoB

Fig. 13

**INTERNATIONAL SEARCH REPORT**

International application No.
**PCT/CN2021/094937**

**A. CLASSIFICATION OF SUBJECT MATTER**

C07C 229/16(2006.01)i; C07C 229/24(2006.01)i; C07C 229/26(2006.01)i; A61K 48/00(2006.01)i; C12N 15/00(2006.01)i

According to International Patent Classification (IPC) or to both national classification and IPC

**B. FIELDS SEARCHED**

Minimum documentation searched (classification system followed by classification symbols)

C07C; A61K; C12N

Documentation searched other than minimum documentation to the extent that such documents are included in the fields searched

Electronic data base consulted during the international search (name of data base and, where practicable, search terms used)

CNTXT; CNABS; DWPI; SIPOABS; CNKI; Web of Science; STNext: 脂质体, 阳离子脂质, 载体, 转染复合物, 北京理工大学, 黄渊余, liposome, carrier, vehicle, cationic lipids, gene transfection, 基于式I化合物的结构检索, Structural Search Based on Formula I Compounds

**C. DOCUMENTS CONSIDERED TO BE RELEVANT**

| Category* | Citation of document, with indication, where appropriate, of the relevant passages | Relevant to claim No. |
|---|---|---|
| Y | PAECHAROENCHAI, O. et al. . "Nonionic Surfactant Vesicles Composed of Novel Spermine-Derivative Cationic Lipids as an Effective Gene Carrier In Vitro." *AAPS Pharm. Sci. Tech.,* Vol. 15, No. 3, 30 June 2014 (2014-06-30), pages 722-730. | 1-21 |
| Y | CN 103380113 A (LIFE TECHNOLOGIES CORP.) 30 October 2013 (2013-10-30) claims 1, 13, 23, and 35-46, and description, paragraphs 94 and 205 | 1-21 |
| Y | CN 108265052 A (SUZHOU RIBO LIFE SCIENCE CO., LTD.) 10 July 2018 (2018-07-10) claims 7-10 | 1-21 |
| Y | WO 2019110067 A1 (AARHUS UNIVERSITET) 13 June 2019 (2019-06-13) claims 1, 7-9, and 14, description, page 23, embodiment 1, and figure 3A | 1-21 |
| Y | KIM, C. et al. . "Supramolecular Assembly of Amide Dendrons." *J. Am. Chem. Soc.,* Vol. 123, No. 23, 15 May 2001 (2001-05-15), pages 5586-5587. | 1-21 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2022** | **17 February 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/094937**

**C. DOCUMENTS CONSIDERED TO BE RELEVANT**

| Category* | Citation of document, with indication, where appropriate, of the relevant passages | Relevant to claim No. |
|---|---|---|
| Y | IWAMOTO, S. et al. . "Gemini Peptide Lipids with Ditopic Ion-Recognition Site. Preparation and Functions as an Inducer for Assembling of Liposomal Membranes." *Tetrahedron.*, Vol. 60, 15 September 2004 (2004-09-15), pages 9841-9847. | 1-21 |
| A | WU, Junyan et al. "Cationic Gemini Surfactants Containing Both Amide and Ester Groups: Synthesis, Surface Properties and Antibacterial Activity." *Journal of Molecular Liquids.*, Vol. vol. 299, 02 December 2019 (2019-12-02), pages 112248(1-7). | 1-21 |
| A | CN 101346468 A (MASSACHUSETTS INSTITUTE OF TECHNOLOGY) 14 January 2009 (2009-01-14) claims 1-131 | 1-21 |
| A | CN 101616677 A (ALNYLAM PHARMACEUTICALS INC.) 30 December 2009 (2009-12-30) claims 1-349 | 1-21 |
| A | CN 111386105 A (NITTO DENKO CORP.) 07 July 2020 (2020-07-07) claims 1-52 | 1-21 |

Form PCT/ISA/210 (second sheet) (January 2015)

**INTERNATIONAL SEARCH REPORT**
**Information on patent family members**

International application No.
**PCT/CN2021/094937**

| Patent document cited in search report | Publication date (day/month/year) | Patent family member(s) | Publication date (day/month/year) |
|---|---|---|---|
| CN 103380113 A | 30 October 2013 | US 2020061197 A1 | 27 February 2020 |
| | | JP 2021059555 A | 15 April 2021 |
| | | JP 2014508102 A | 03 April 2014 |
| | | US 2018200374 A1 | 19 July 2018 |
| | | US 10406237 B2 | 10 September 2019 |
| | | JP 2017031186 A | 09 February 2017 |
| | | DK 2640700 T3 | 14 January 2019 |
| | | US 2015174260 A1 | 25 June 2015 |
| | | US 9901642 B2 | 27 February 2018 |
| | | CN 108358812 A | 03 August 2018 |
| | | CN 113214102 A | 06 August 2021 |
| | | EP 2640700 A1 | 25 September 2013 |
| | | EP 2640700 B1 | 31 October 2018 |
| | | JP 2018197255 A | 13 December 2018 |
| | | JP 6807900 B2 | 06 January 2021 |
| | | WO 2012068176 A1 | 24 May 2012 |
| | | ES 2702428 T3 | 28 February 2019 |
| | | JP 2018080187 A | 24 May 2018 |
| | | JP 6383480 B2 | 29 August 2018 |
| | | EP 3470395 A1 | 17 April 2019 |
| | | US 2012136073 A1 | 31 May 2012 |
| CN 108265052 A | 10 July 2018 | None | |
| WO 2019110067 A1 | 13 June 2019 | None | |
| CN 101346468 A | 14 January 2009 | WO 2006138380 A2 | 28 December 2006 |
| | | WO 2006138380 A3 | 24 July 2008 |
| | | JP 2009143960 A | 02 July 2009 |
| | | CN 101346468 B | 30 March 2016 |
| | | CA 2611944 A1 | 28 December 2006 |
| | | JP 2014169288 A | 18 September 2014 |
| | | US 2016009657 A1 | 14 January 2016 |
| | | JP 2016027057 A | 18 February 2016 |
| | | EP 2476756 A1 | 18 July 2012 |
| | | JP 2009501699 A | 22 January 2009 |
| | | JP 5777846 B2 | 09 September 2015 |
| | | US 2011009641 A1 | 13 January 2011 |
| | | US 9006487 B2 | 14 April 2015 |
| | | AU 2006259415 A1 | 28 December 2006 |
| | | AU 2006259415 B2 | 30 August 2012 |
| | | EP 1912679 A2 | 23 April 2008 |
| | | EP 1912679 A4 | 29 July 2009 |
| CN 101616677 A | 30 December 2009 | US 2009023673 A1 | 22 January 2009 |
| | | US 8034376 B2 | 11 October 2011 |
| | | MX 2019003005 A | 05 August 2019 |
| | | CN 110066224 A | 30 July 2019 |
| | | ES 2611924 T3 | 11 May 2017 |
| | | CA 2665225 A1 | 10 April 2008 |
| | | CA 2665225 C | 30 June 2015 |
| | | US 2012046478 A1 | 23 February 2012 |
| | | US 8642076 B2 | 04 February 2014 |
| | | DK 2068886 T3 | 18 November 2013 |

Form PCT/ISA/210 (patent family annex) (January 2015)

**INTERNATIONAL SEARCH REPORT**
**Information on patent family members**

International application No.
**PCT/CN2021/094937**

| Patent document cited in search report | Publication date (day/month/year) | Patent family member(s) | Publication date (day/month/year) |
|---|---|---|---|
| | | EP 2695608 A2 | 12 February 2014 |
| | | EP 2695608 A3 | 21 May 2014 |
| | | EP 2695608 B1 | 23 November 2016 |
| | | MX 363224 B | 15 March 2019 |
| | | US 2014121393 A1 | 01 May 2014 |
| | | IN 255DEN2015 A | 12 June 2015 |
| | | JP 2015227370 A | 17 December 2015 |
| | | JP 2010505873 A | 25 February 2010 |
| | | JP 5933163 B2 | 08 June 2016 |
| | | US 2018065918 A1 | 08 March 2018 |
| | | JP 2021011503 A | 04 February 2021 |
| | | ES 2430206 T3 | 19 November 2013 |
| | | AU 2007303205 A1 | 10 April 2008 |
| | | JP 2017036330 A | 16 February 2017 |
| | | CN 105030654 A | 11 November 2015 |
| | | CA 2927045 A1 | 10 April 2008 |
| | | KR 20090088362 A | 19 August 2009 |
| | | KR 101129509 B1 | 13 April 2012 |
| | | EP 2068886 A2 | 17 June 2009 |
| | | EP 2068886 A4 | 22 December 2010 |
| | | EP 2068886 B1 | 18 September 2013 |
| | | MX 2009003548 A | 15 April 2009 |
| | | EP 3192788 A1 | 19 July 2017 |
| | | WO 2008042973 A2 | 10 April 2008 |
| | | WO 2008042973 A3 | 28 August 2008 |
| | | US 2020361854 A1 | 19 November 2020 |
| | | CA 2848238 A1 | 10 April 2008 |
| | | CA 2848238 C | 19 July 2016 |
| | | JP 2019048887 A | 28 March 2019 |
| | | CN 101616677 B | 22 July 2015 |
| | | JP 2013213036 A | 17 October 2013 |
| | | JP 5833597 B2 | 16 December 2015 |
| CN 111386105 A | 07 July 2020 | AU 2018359904 A1 | 18 June 2020 |
| | | KR 20200084338 A | 10 July 2020 |
| | | WO 2019090359 A1 | 09 May 2019 |
| | | WO 2019090359 A9 | 26 March 2020 |
| | | JP 2021502346 A | 28 January 2021 |
| | | RU 2020117606 A | 08 December 2021 |
| | | BR 112020008451 A2 | 01 December 2020 |
| | | CA 3081758 A1 | 09 May 2019 |
| | | EP 3706729 A1 | 16 September 2020 |
| | | EP 3706729 A4 | 01 December 2021 |
| | | US 2019133948 A1 | 09 May 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Handbook of Chemicals. 1994
- **THOMAS SORRELL.** Organic Chemistry. University Science Books, 1999
- **MICHAEL B. SMITH ; JERRY MARCH.** March's Advanced Organic Chemistry. John Wiley & Sons, 2007
- Dictionary of Chemical Terms. McGraw-Hill, 1984
- Book Company. McGraw-Hill
- **ELIEL, E. ; WILEN, S.** Stereochemistry of Organic Compounds. John Wiley & Sons, Inc, 1994
- **JERRY MARCH.** Advanced Organic Chemistry. wiley Interscience
- **L. W. DEADY.** *Syn. Comm.,* 1977, vol. 7, 509-514
- **S. M. BERGE et al.** describe pharmaceutically acceptable salts in detail. *J. Pharmaceutical Sciences,* 1977, vol. 66, 1-19
- **JACQUES et al.** Enantiomers, Racemates and Resolutions. Wiley Interscience, 1981
- **ROBERT E. GAWLEY ; JEFFREY AUBÉ.** Principles of Asymmetric Synthesis. Elsevier, 2012
- **ELIEL, E.L.** Stereochemistry of Carbon Compounds. McGraw-Hill, 1962
- **WILEN, S.H.** Tables of Resolving Agents and Optical Resolutions. Univ. of Notre Dame Press, 1972, 268
- Remington's Pharmaceutical Sciences. Mack Printing Company, 1990, 1289-1329
- Remington's Pharmaceutical Sciences. Mack Publishing Company, 1985
- **STAHL ; WERMUTH.** Handbook of Pharmaceutical Salts: Properties, Selection, and Use. Wiley-VCH, 2002
- Polyelectrolyte multilayer films with pegylated polypeptides as a new type of antimicrobial protection for biomaterials. **BOULMEDAIS F ; FRISCH B. ; ETIENNE O. ; LAVALLE PH. ; PICART C. ; OGIER J. ; VOEGEL J-C. ; SCHAAF P. ; EGLES C.** Biomaterials. 2004, vol. 25, 2003-2011